# EUROPEAN PATENT SPECIFICATION

(11) **EP 4 275 208 B1**
(45) Date of publication and mention of the grant of the patent: **24.06.2026**
(21) Application number: 22702821.4
(22) Date of filing: 07.01.2022
(51) Int. Cl.: G16B 30/10, G16B 40/30, G16B 20/10

(54) **SYSTEMS AND METHODS FOR DEDUCING COPY NUMBER VARIATION FOR CLINICAL DIAGNOSTICS USING JOINT LOW-COVERAGE WHOLE GENOME SEQUENCING AND WHOLE EXOME SEQUENCING**
SYSTEME UND METHODEN ZUR ABLEITUNG DER KOPIENZAHLVARIATION FÜR KLINISCHE DIAGNOSTIK MIT HILFE VON GEMEINSAMER GESAMTGENOMSEQUENZIERUNG MIT NIEDRIGER ABDECKUNG UND GESAMTEXOMSEQUENZIERUNG
SYSTÈMES ET PROCÉDÉS POUR DÉDUIRE LA VARIABILITÉ DU NOMBRE DE COPIES POUR LE DIAGNOSTIC CLINIQUE À L'AIDE DU SÉQUENÇAGE DU GÉNOME ENTIER À FAIBLE COUVERTURE ET DU SÉQUENÇAGE DE L'EXOME ENTIER INFERENCE

(30) Priority: 07.01.2021 US 202163134913 P
(43) Date of publication of application: 15.11.2023
(73) Proprietor: Tempus AI, Inc., Chicago, IL 60654 (US)
(72) Inventor: DE LA VEGA, Francisco, M., Chicago, IL 60654 (US)
(74) Representative: Grund, Martin
(86) International application number: PCT/US2022/011724
(87) International publication number: WO 2022/150663

(56) References cited:
- US-A1- 2020 087 710
- SOVERINI SIMONA ET AL: "Genome-Wide Molecular Portrait of Aggressive Systemic Mastocytosis and Mast Cell Leukemia Depicted By Whole Exome Sequencing and Copy Number Variation Analysis", BLOOD, AMERICAN SOCIETY OF HEMATOLOGY, US, vol. 126, no. 23, 3 December 2015 (2015-12-03), pages 4085, XP086649475, ISSN: 0006-4971, DOI: 10.1182/BLOOD.V126.23.4085.4085
- HEHIR-KWA JAYNE Y ET AL: "Exome sequencing and whole genome sequencing for the detection of copy number variation", EXPERT REVIEWS IN MOLECULAR DIAGNOSTICS, vol. 15, no. 8, 3 August 2015 (2015-08-03), GB, pages 1023 - 1032, XP055910889, ISSN: 1473-7159, Retrieved from the Internet <URL:http://dx.doi.org/10.1586/14737159.2015.1053467> [retrieved on 20220408], DOI: 10.1586/14737159.2015.1053467

## Description

### TECHNICAL FIELD

The present disclosure relates generally to use of low-coverage whole genome sequencing and panel-targeted sequencing to jointly identify copy number variations in a genome.

### BACKGROUND

Genomic deletions or insertions affecting the coding regions of genes, known as copy number variants (CNVs) are often deleterious. These events can range in size from very large (*e.g.,* completely overlapping and/or disrupting one or more genes) or very small (*e.g.,* a single exon), and can occur in both the germline and in abnormal cells (*e.g.,* cancer cells) as the product of somatic mutation processes. The pathogenicity of such variants depends on the type of event (*e.g.,* deletions are generally more likely to be deleterious while whole gene duplications can result in a gain of function) or on the region of the coding sequence of a gene that is affected by such variants (*e.g.,* changes in the last exon of a gene are less likely to be deleterious). CNV variants, by virtue of their damaging effect, can impact the inherited risk to diseases such as cancer or provide a growth advantage in tumors, and hence can affect clinical outcomes and/or provide opportunities for targeted therapies.

However, detecting small CNVs from targeted short-read sequencing data can be challenging. Most conventional methods for detecting CNV events from next-generation sequencing (NGS) rely on detecting changes in the mean or median depth of coverage that such events are expected to cause (*e.g.,* deletions would result in a reduction in depth of coverage, and vice versa for duplications). US 2020/087710 A1, for instance, discloses methods and systems for non-invasive classification of a mosaic copy number variation for a test sample. However, it is particularly difficult to differentiate actual changes in sequencing depth from several types of technical artifacts that change the depth profile irrespective of gene dosage changes, including, but not limited to, a) sequencing biases due to GC content, b) read mapping biases due to repeats, c) segmental duplications, e) paralogous regions, and/or f) systematic capture biases prevalent in targeted sequencing chemistries.

### SUMMARY

Given the above background, what is needed in the art are improved methods and systems for identifying CNVs. Particularly, methods and systems for identifying short CNVs from sequencing data that can also be used to identify disease risk, such as panel-targeted sequencing, are desired. The present disclosure solves this and other needs in the art by providing improvements to methods, systems, and software for determining a CNV status of a subject.

The invention is defined by the appended claims.

Accordingly, one aspect of the present disclosure provides a method for determining a copy number variation status of a subject, comprising:
on a computer system having one or more processors, and memory storing one or more programs for execution by the one or more processors:
   A) obtaining, in electronic form, a first plurality of at least 100,000 nucleic acid sequences for a first plurality of DNA molecules from a first biological sample of the subject generated by whole genome sequencing at an average sequencing depth of from 0.5X to 5X across at least 90% of a reference genome for the species of the subject;
   B) obtaining, in electronic form, a second plurality of at least 10,000 nucleic acid sequences for a second plurality of DNA molecules from a second biological sample of the subject generated by panel-targeted sequencing;
   C) obtaining a first mapped dataset by a process comprising mapping the first plurality of nucleic acid sequences to positions within a reference genome for the species of the subject;
   D) obtaining a second mapped dataset by a process comprising mapping the second plurality of nucleic acid sequences to positions within a reference construct for a plurality of genomic regions targeted by the panel-targeted sequencing; and
   E) applying a model to (i) all or a portion of the first mapped dataset, or a first plurality of dimensionality reduction components thereof, and (ii) all or a portion of the second mapped dataset, or a second plurality of dimensionality reduction components thereof, wherein:
the model comprises a first component model and a second component model, wherein,
   the first component model provides a first respective copy number state for a respective genomic region of the one or more respective genomic regions upon input to the first component model of all or a portion of the first mapped dataset, or the first plurality of dimensionality reduction components thereof, and
   the second component model provides a second respective copy number state for the respective genomic region of the one or more respective genomic regions upon input to the second component model of all or a portion of the second mapped dataset, or the second plurality of dimensionality reduction components thereof; and
when both (i) the first respective copy number state and (ii) the second respective copy number state indicates the presence of a copy number variation at the respective genomic region, the copy number variation at the respective genomic region is accepted; and
when either (i) the first respective copy number state or (ii) the second respective copy number state does not indicate the presence of a copy number variation at the respective genomic region, the copy number variation at the respective genomic region is rejected,
thereby identifying one or more copy number variations, as output of the model, that indicate the copy number variation status of the subject.

Another aspect of the present disclosure provides a computer system for determining a copy number variation status, the computer system comprising:
one or more processors; and
memory addressable by the one or more processors, the memory storing at least one program for execution by the one or more processors, the at least one program comprising instructions for:
   A) obtaining, in electronic form, a first plurality of at least 100,000 nucleic acid sequences for a first plurality of DNA molecules from a first biological sample of the subject generated by whole genome sequencing at an average sequencing depth of from 0.5X to 5X across at least 90% of a reference genome for the species of the subject;
   B) obtaining, in electronic form, a second plurality of at least 10,000 nucleic acid sequences for a second plurality of DNA molecules from a second biological sample of the subject generated by panel-targeted sequencing;
   C) obtaining a first mapped dataset by a process comprising mapping the first plurality of nucleic acid sequences to positions within a reference genome for the species of the subject;
   D) obtaining a second mapped dataset by a process comprising mapping the second plurality of nucleic acid sequences to positions within a reference construct for a plurality of genomic regions targeted by the panel-targeted sequencing; and
   E) applying a model to (i) all or a portion of the first mapped dataset, or a first plurality of dimensionality reduction components thereof, and (ii) all or a portion of the second mapped dataset, or a second plurality of dimensionality reduction components thereof, wherein:
the model comprises a first component model and a second component model, wherein,
   the first component model provides a first respective copy number state for a respective genomic region of the one or more respective genomic regions upon input to the first component model of all or a portion of the first mapped dataset, or the first plurality of dimensionality reduction components thereof, and
   the second component model provides a second respective copy number state for the respective genomic region of the one or more respective genomic regions upon input to the second component model of all or a portion of the second mapped dataset, or the second plurality of dimensionality reduction components thereof; and
when both (i) the first respective copy number state and (ii) the second respective copy number state indicates the presence of a copy number variation at the respective genomic region, the copy number variation at the respective genomic region is accepted; and
when either (i) the first respective copy number state or (ii) the second respective copy number state does not indicate the presence of a copy number variation at the respective genomic region, the copy number variation at the respective genomic region is rejected,
   thereby identifying one or more copy number variations, as output of the model that indicate the copy number variation status of the subject.

Another aspect of the present disclosure provides a non-transitory computer readable storage medium, wherein the non-transitory computer readable storage medium stores instructions, which when executed by a computer system, cause the computer system to perform a method for determining a copy number variation status, the method comprising:
A) obtaining, in electronic form, a first plurality of at least 100,000 nucleic acid sequences for a first plurality of DNA molecules from a first biological sample of the subject generated by whole genome sequencing at an average sequencing depth of from 0.5X to 5X across at least 90% of a reference genome for the species of the subject;
B) obtaining, in electronic form, a second plurality of at least 10,000 nucleic acid sequences for a second plurality of DNA molecules from a second biological sample of the subject generated by panel-targeted sequencing;
C) obtaining a first mapped dataset by a process comprising mapping the first plurality of nucleic acid sequences to positions within a reference genome for the species of the subject;
D) obtaining a second mapped dataset by a process comprising mapping the second plurality of nucleic acid sequences to positions within a reference construct for a plurality of genomic regions targeted by the panel-targeted sequencing; and
E) applying a model to (i) all or a portion of the first mapped dataset, or a first plurality of dimensionality reduction components thereof, and (ii) all or a portion of the second mapped dataset, or a second plurality of dimensionality reduction components thereof, wherein:
   the model comprises a first component model and a second component model, wherein,
      the first component model provides a first respective copy number state for a respective genomic region of the one or more respective genomic regions upon input to the first component model of all or a portion of the first mapped dataset, or the first plurality of dimensionality reduction components thereof, and
      the second component model provides a second respective copy number state for the respective genomic region of the one or more respective genomic regions upon input to the second component model of all or a portion of the second mapped dataset, or the second plurality of dimensionality reduction components thereof; and
   when both (i) the first respective copy number state and (ii) the second respective copy number state indicates the presence of a copy number variation at the respective genomic region, the copy number variation at the respective genomic region is accepted; and
   when either (i) the first respective copy number state or (ii) the second respective copy number state does not indicate the presence of a copy number variation at the respective genomic region, the copy number variation at the respective genomic region is rejected,
thereby identifying one or more copy number variations, as output of the model that indicate the copy number variation status of the subject.

### BRIEF DESCRIPTION OF THE DRAWINGS

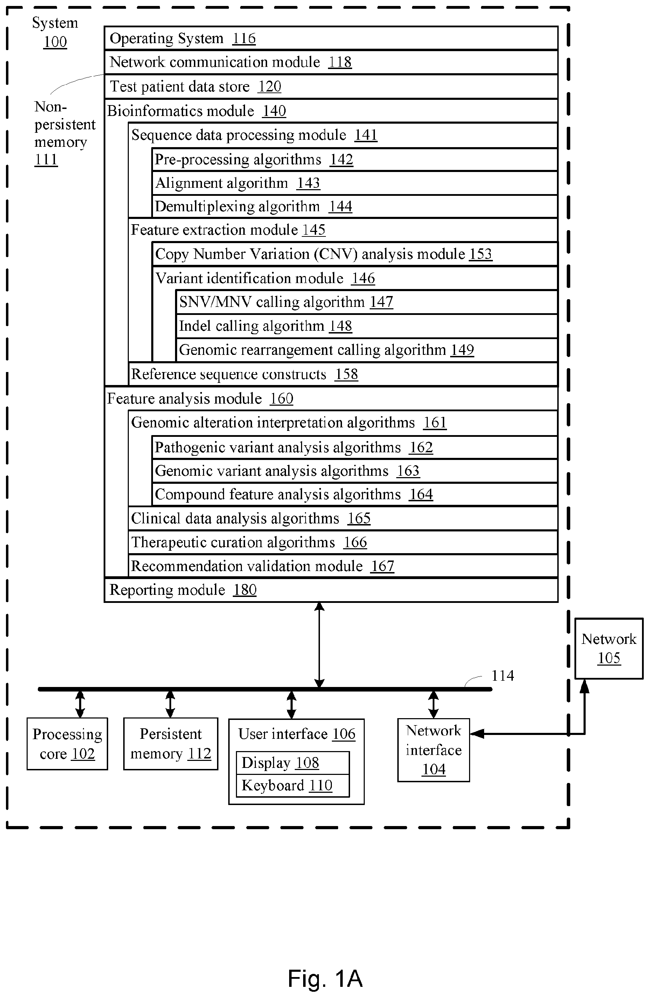

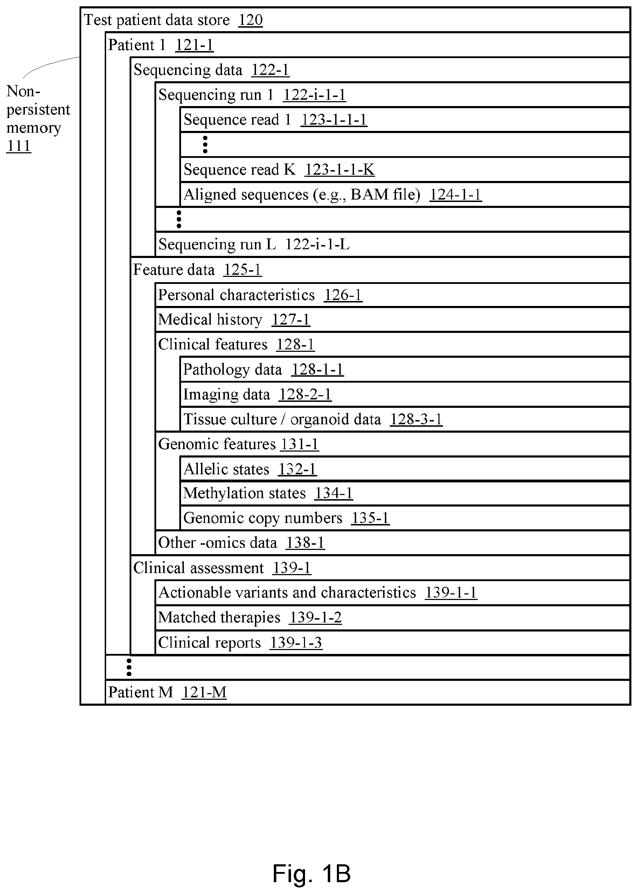

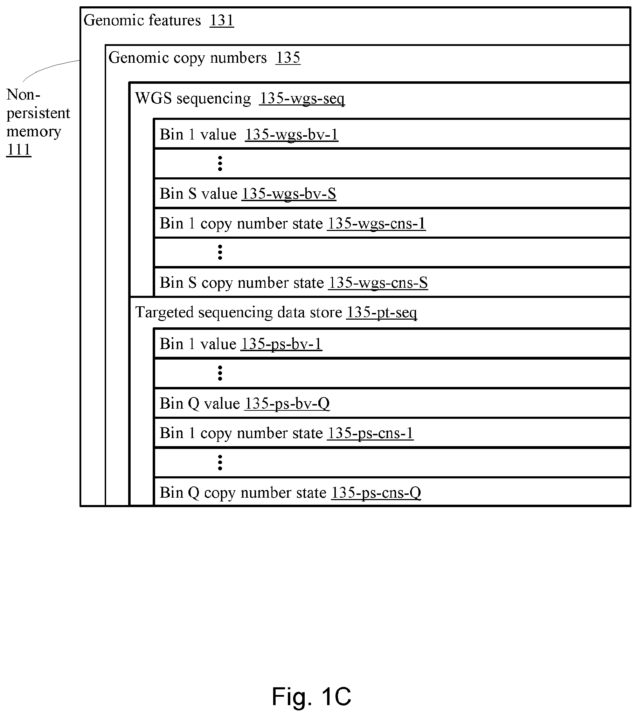

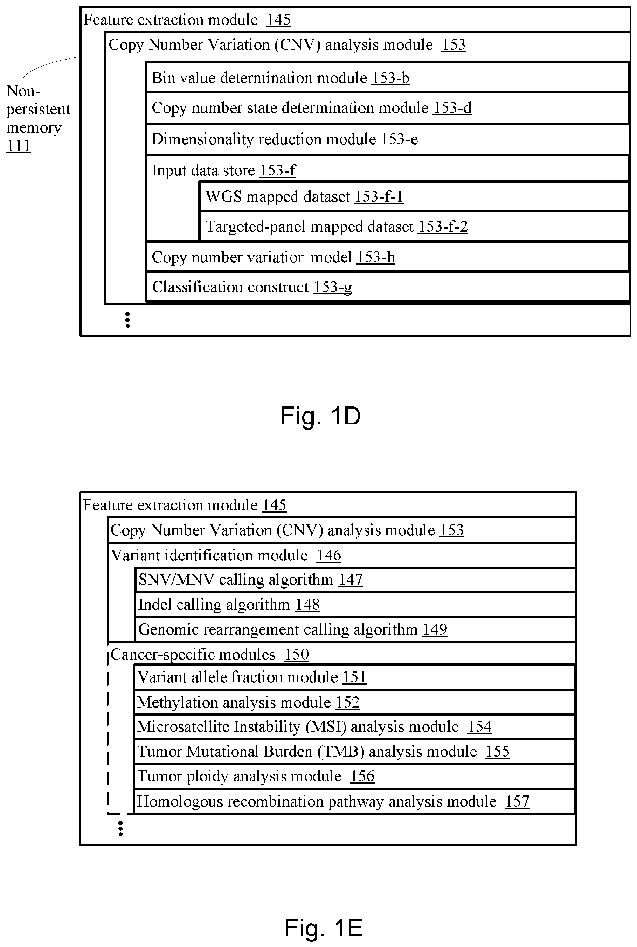

**Figures 1A****,** **1B****,** **1C****,** **1D, and 1E** collectively illustrate a block diagram of an example computing device for determining a copy number variation status of a subject.

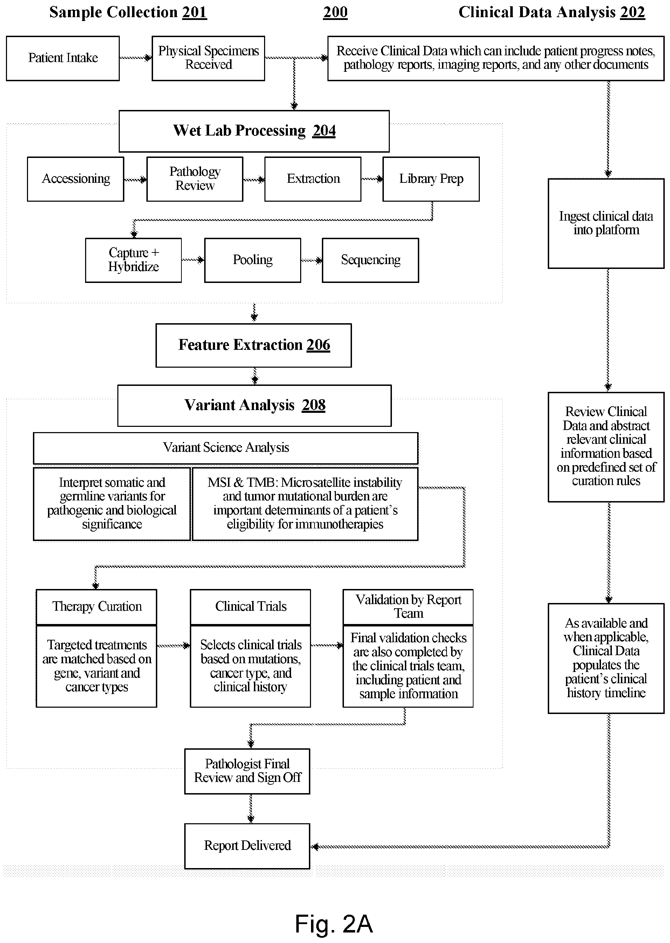

**Figure 2A** illustrates an example workflow for generating a clinical report based on information generated from analysis of one or more patient specimens.

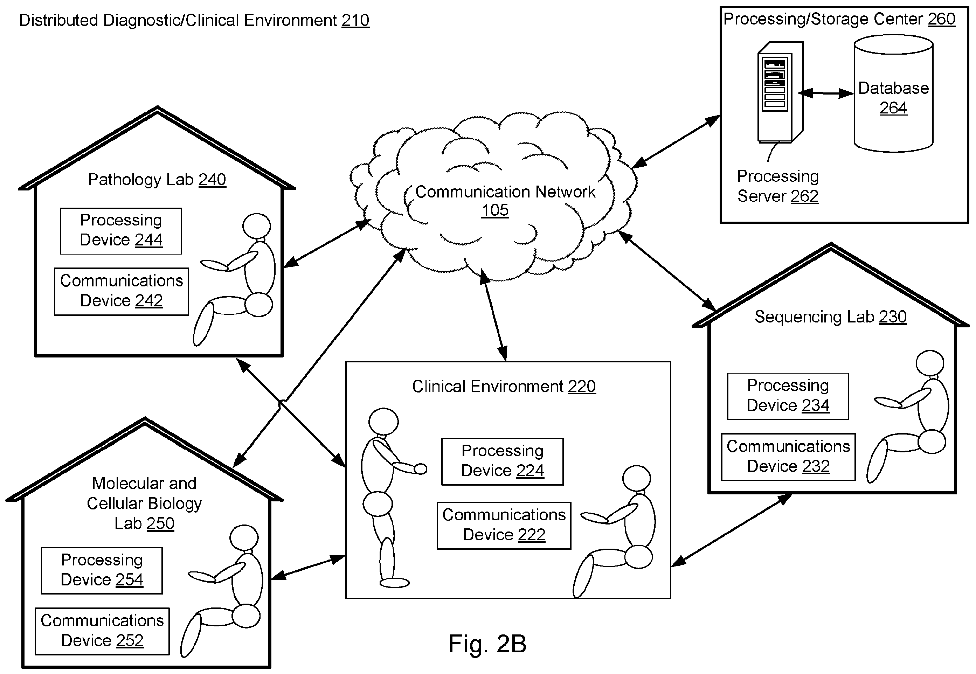

**Figure 2B** illustrates an example of a distributed diagnostic environment for collecting and evaluating patient data for the purpose of precision oncology.

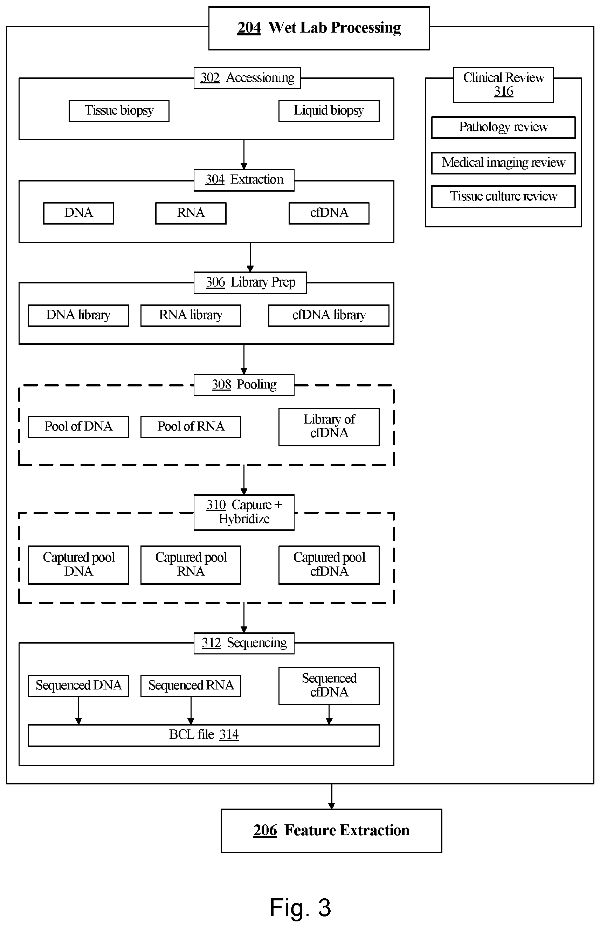

**Figure 3** provides an example flow chart of processes and features for sample collection and analysis for use in precision medicine.

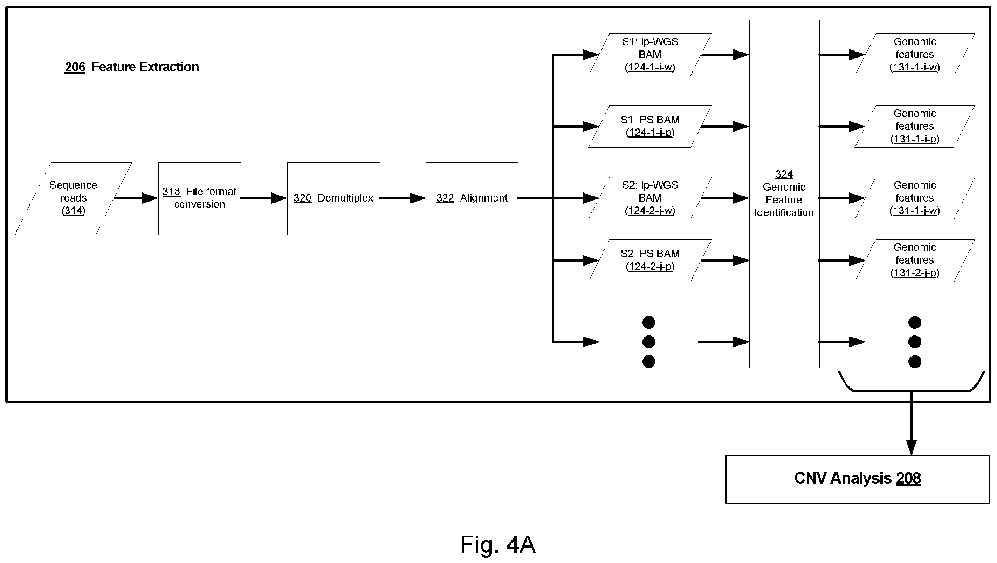

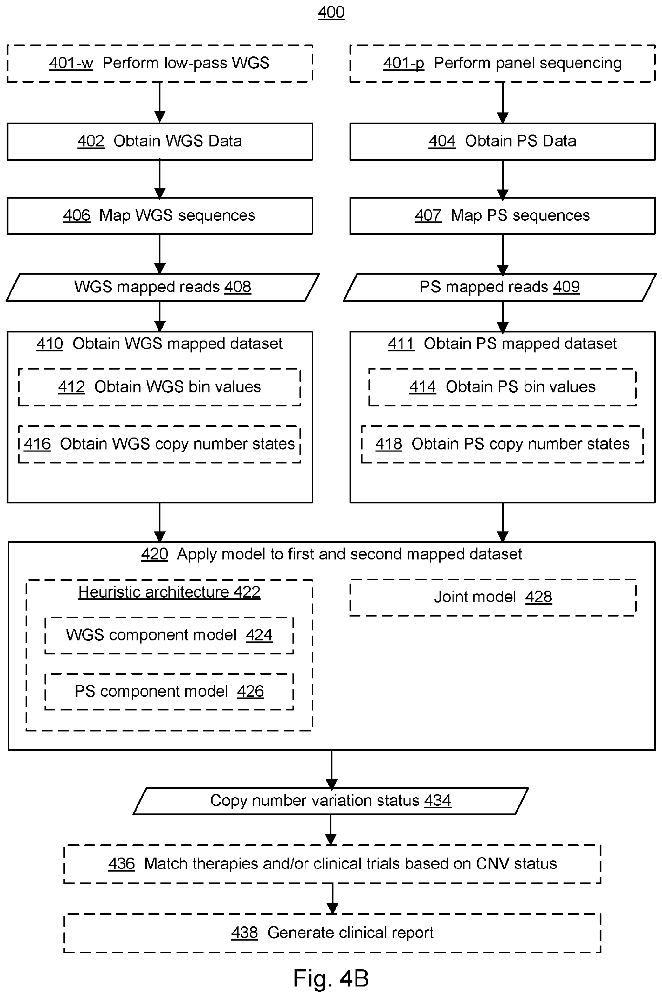

**Figures 4A** **and** **4B** collectively illustrate an example bioinformatics pipeline for determining a copy number variation status of a subject. Figure 4A provides an overview flow chart of processes and features in a bioinformatics pipeline. Figure 4B illustrates an example flow chart of processes and features for determining a copy number variation status of a subject, in which dashed boxes represent optional portions of the method.

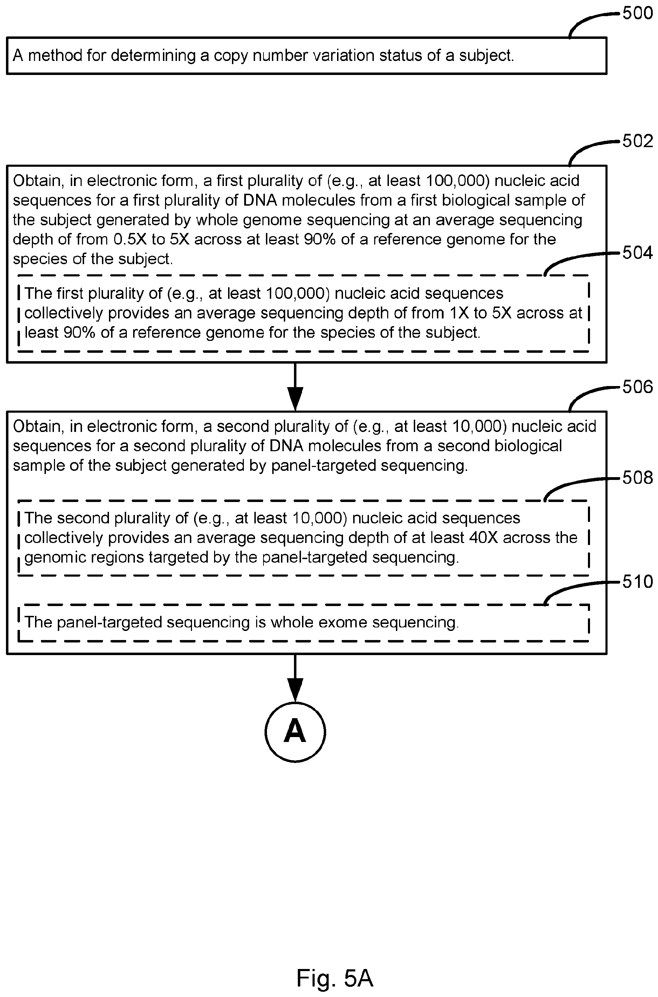

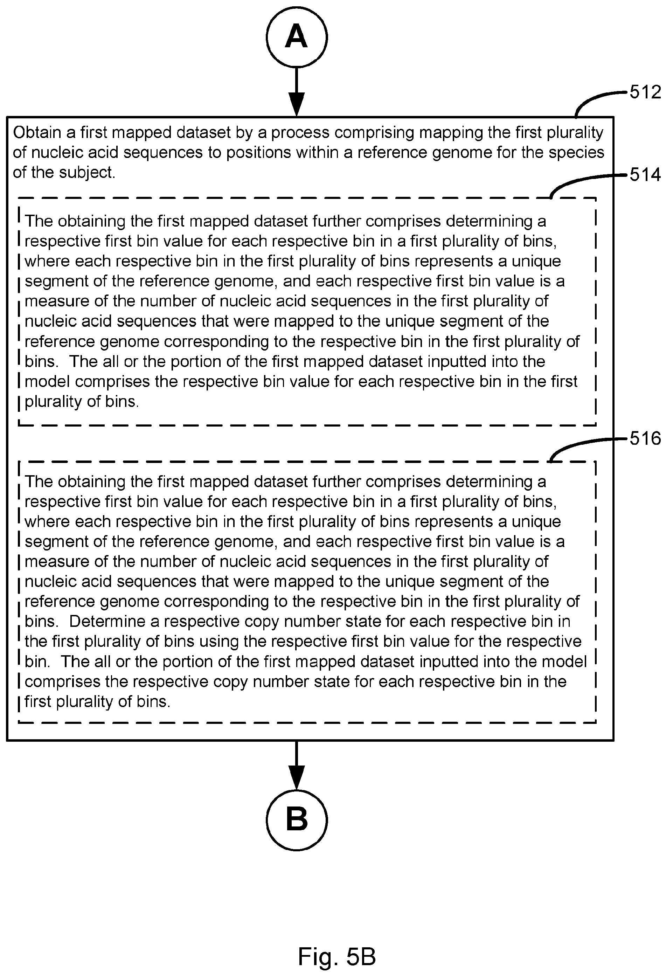

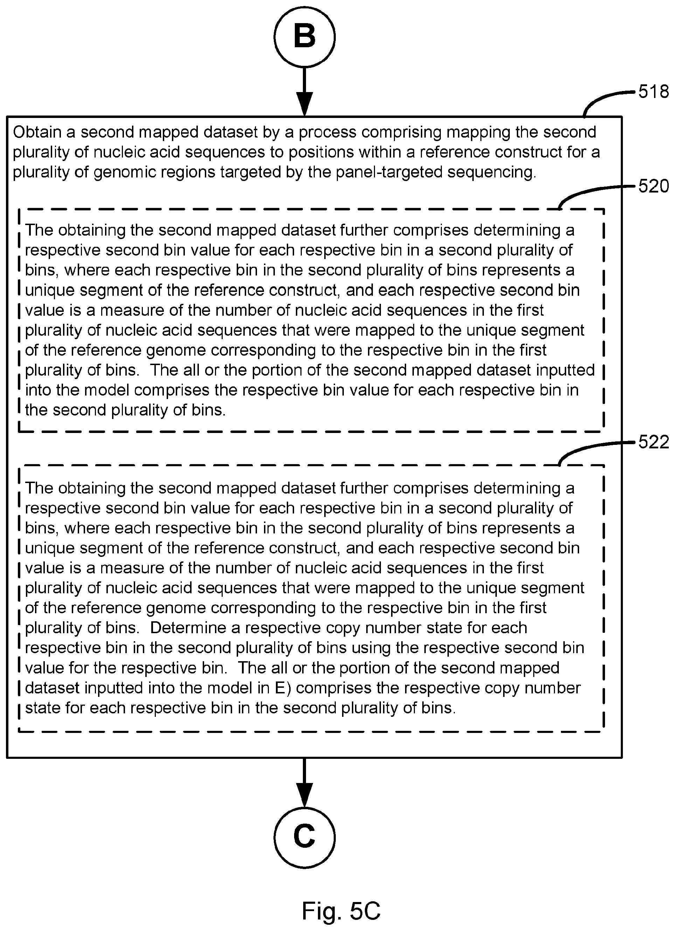

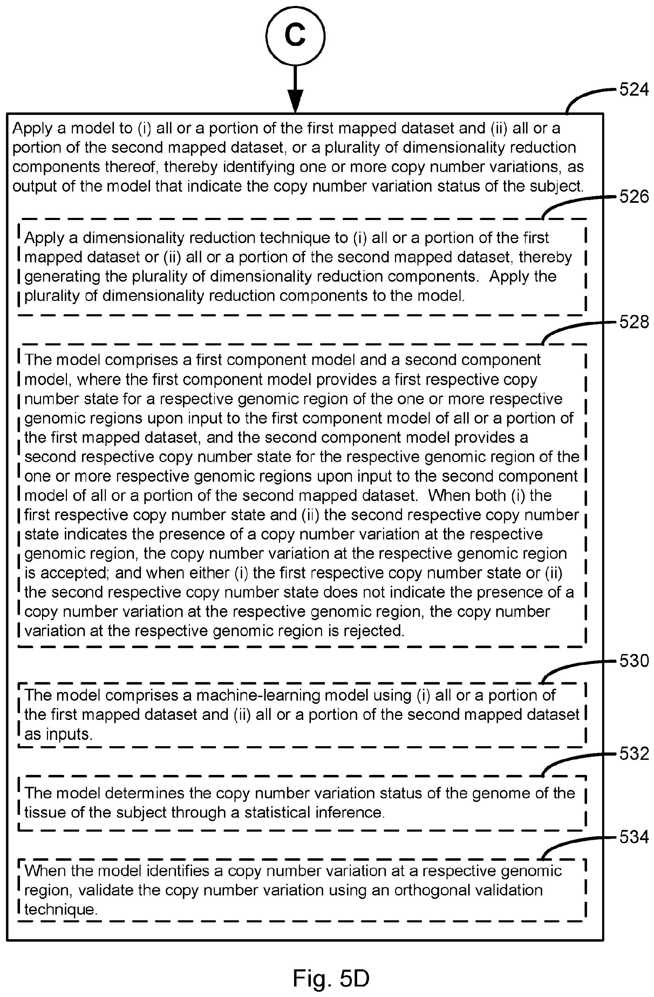

**Figures 5A****,** **5B****,** **5C****,** and **5D** collectively provide a flow chart of processes and features for determining a copy number variation status of a subject, in which dashed boxes represent optional portions of the method.

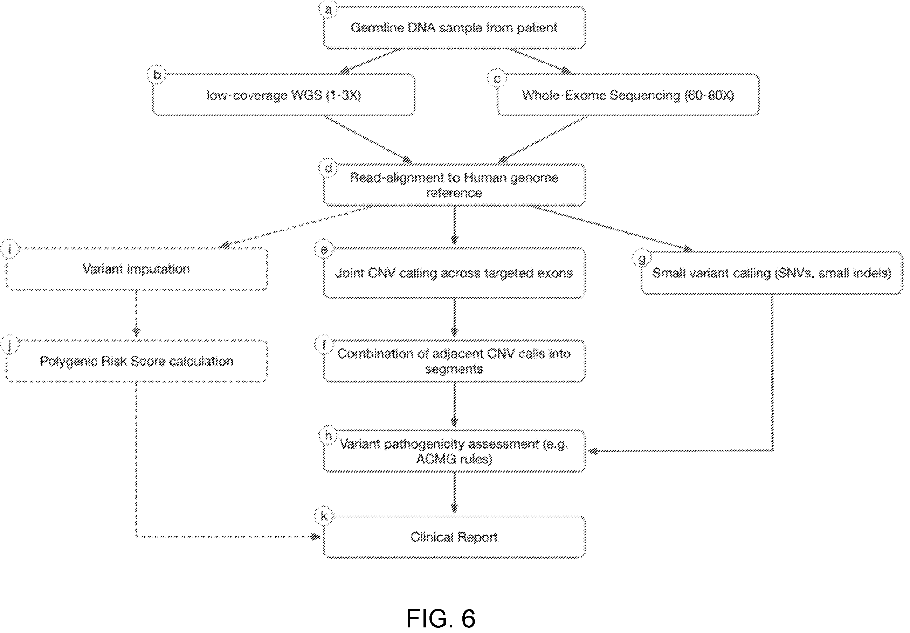

**Figure 6** illustrates an example workflow of a method for clinical reporting combining low-coverage whole genome sequencing (Ic-WGS) and whole exome sequencing (WES) data. Steps in dotted lines/boxes are optional and can be added to provide genetic disease risk prediction in the clinical report.

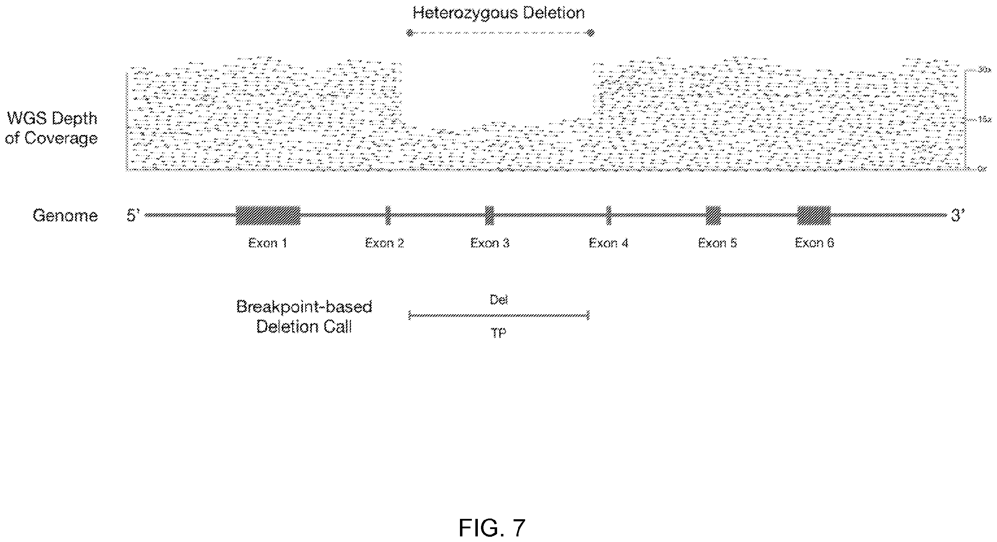

**Figure 7** illustrates an example analysis of CNVs (deletions/insertions) from WGS data. Deletions, duplications, and more general structural variants can be detected in WGS data using coverage depth analysis as well as identification of split reads, where, due to breakpoints, a first end of a respective read maps in one location and a second end of the respective read maps in a different location, resulting in discordance in apparent fragment inset size between pair-end reads. Because PCR-free WGS libraries result in close to random shotgun sequencing of the DNA templates, depth of coverage is fairly uniform in WGS alignments, although some systematic biases remain. This may allow more accurate analysis of depth for CNV detection.

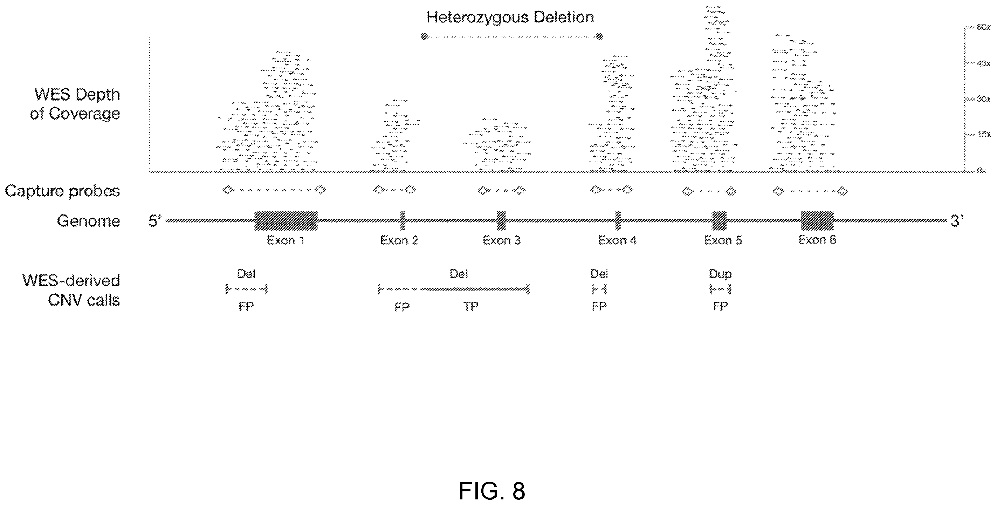

**Figure 8** illustrates an example analysis of CNVs (deletions/insertions) from WES data. In WES, exons are captured for sequencing but depth of coverage across these regions is much more variable than in WGS due to biases in the capture of DNA fragments by the assay probes. This makes it difficult to determine when deletions or duplications occur and creates false positives and negatives in CNV detection, as exemplified in the calls shown at the bottom (FP = false positive; FN = false negative segments). Single exon events are more difficult, because only reads in 150-300 base pairs of the span of the exon are available. In addition, most breakpoints of structural variants occur in intronic or intergenic regions, by chance. Small events that affect a single exon may actually span several kilobases of intronic segments but are only manifested and detectable in the targeted exon region in the WES assay.

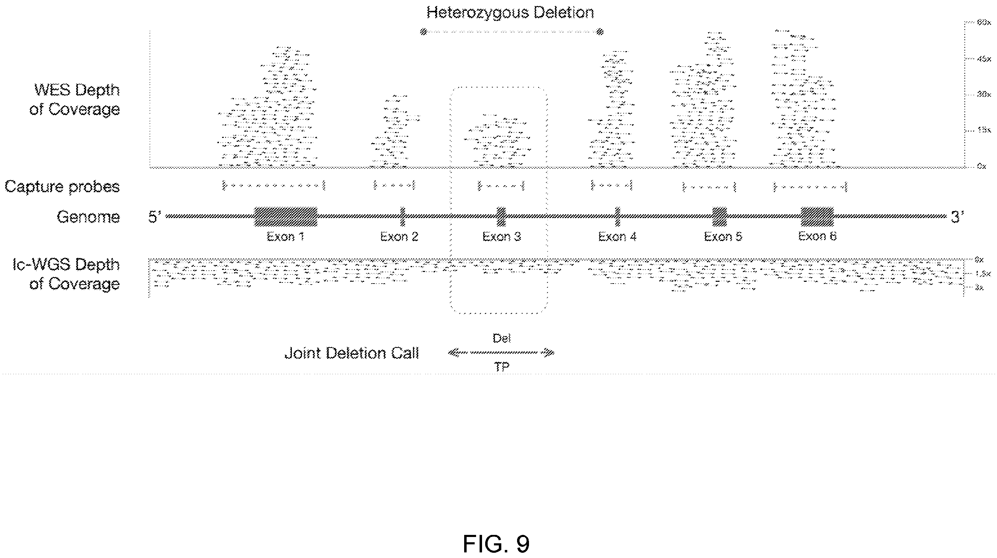

**Figure 9** illustrates an example of joint calling of CNVs (deletions/insertions) combining WES and Ic-WGS data. The pattern of WES sequence depth is quite variable and makes it difficult for most algorithms to find the single exon deletion (reads depicted in upper chart). On the other hand, the Ic-WGS signal is weak and by itself can lead to low sensitivity to small events and spurious results (reads depicted in lower chart). By combining both signals, a properly trained and/or calibrated algorithm can improve sensitivity and specificity and be able to reject false positives and detect single exon deletion (or duplications) which span more base pairs than are contained within the exon itself, even if the actual boundaries predicted are imprecise (e.g., due to the lack of sequence reads covering a breakpoint).

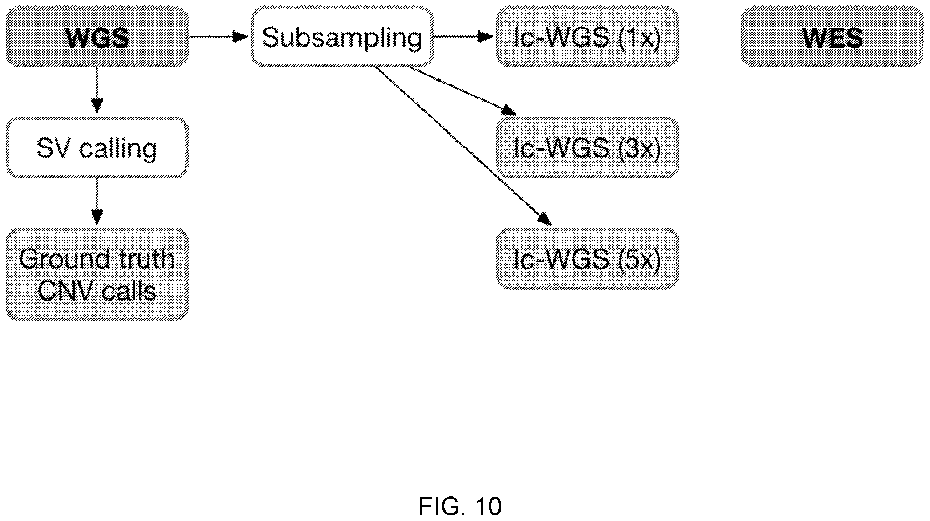

**Figure 10** illustrates an example schema for preparing training data for model development and validation. For training and validating joint CNV calling by machine learning (ML) or other methods, a set of human genomes is sequenced by WGS and WES. Standard depth WGS data (*e.g.,* 30-50X) generates a ground truth dataset of CNV calls. This data can also be subsampled to simulate Ic-WGS for training. A second input to the model includes WES data at standard depth (~60X). Full depth WGS and WES data for the 2,500 samples of the 1,000 Genomes Project (1KGP) were obtained from the New York Genome Center (NYGC) and Google Genomics. These data can be used for model training and cross validation.

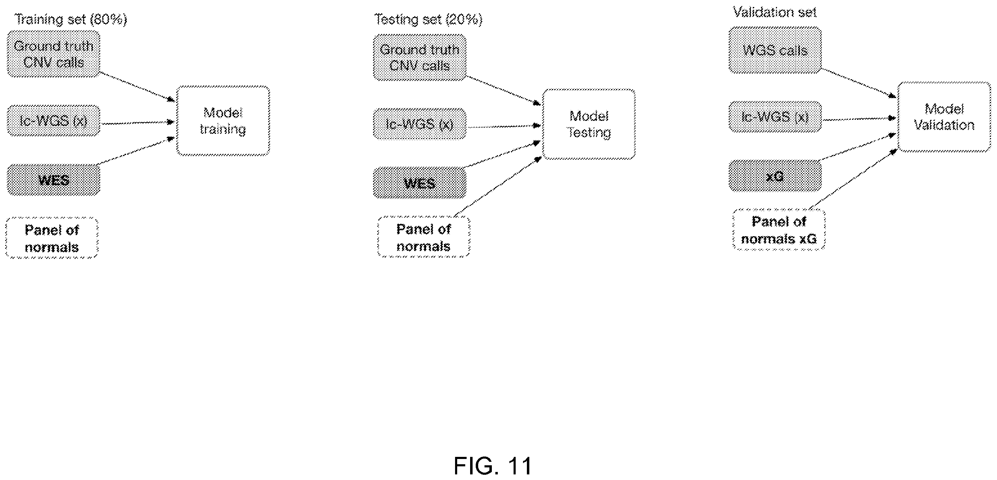

**Figure 11** illustrates an example schema for model training, testing, and validation for joint CNV calling. Training, testing, and validation steps can be optionally aided by a panel of normal samples for a region of interest.

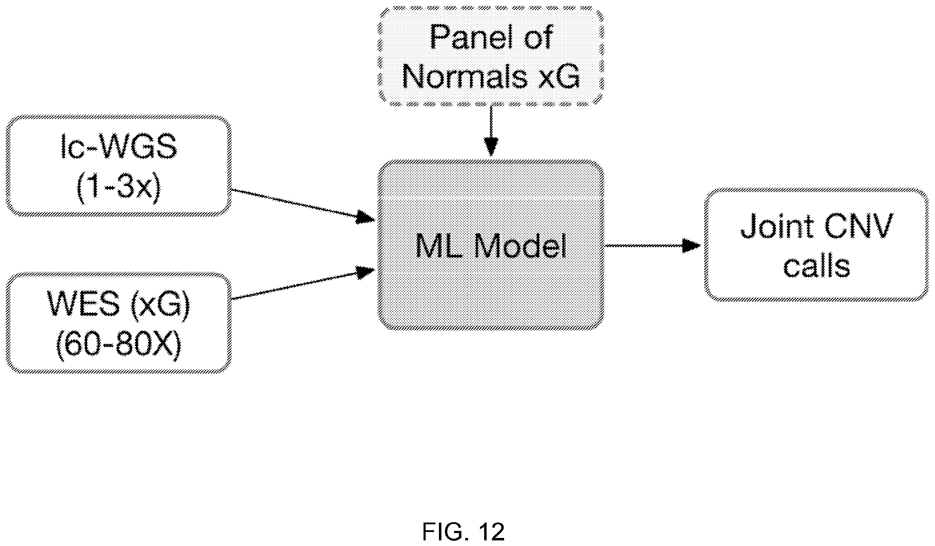

**Figure 12** illustrates an example of an operational phase using a ML model for joint CNV calling. Once a model is validated, new sample data is used to generate joint CNV calls in production mode, optionally using a panel of normal samples sequenced with the same assay.

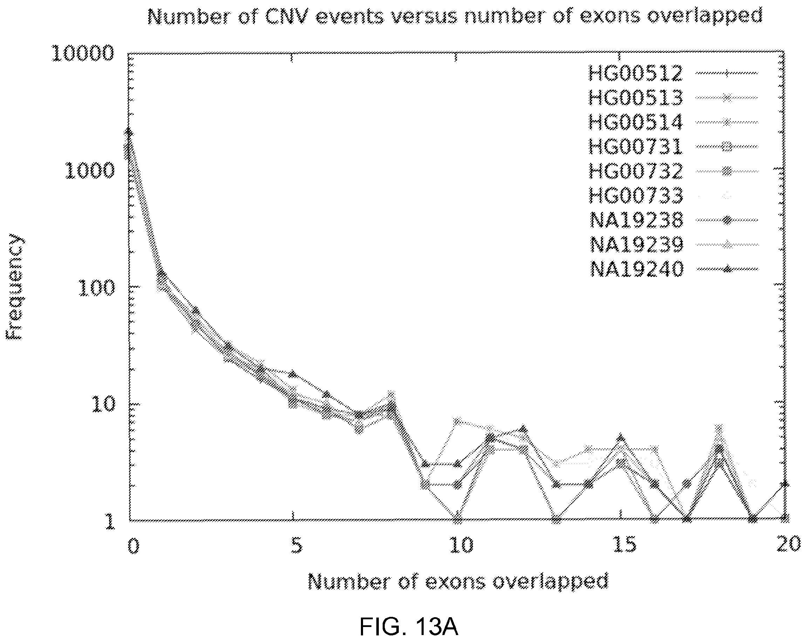

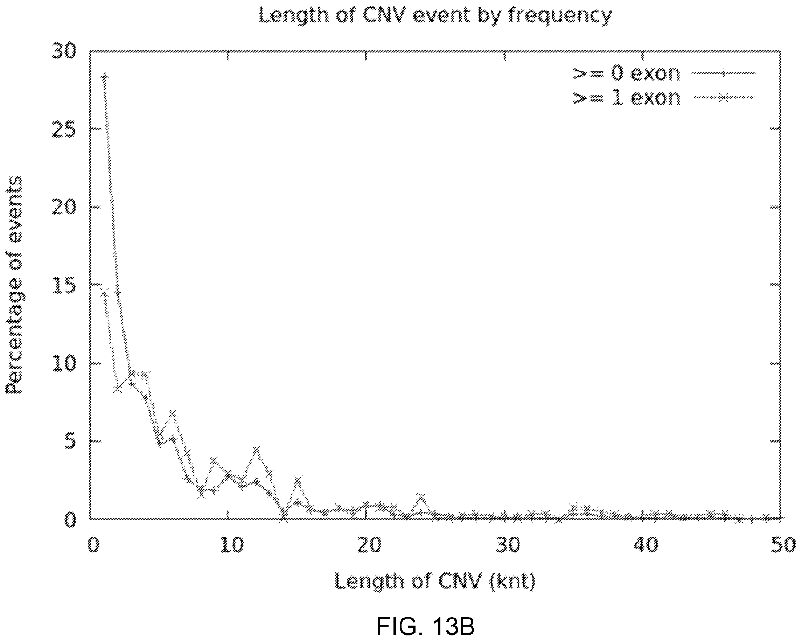

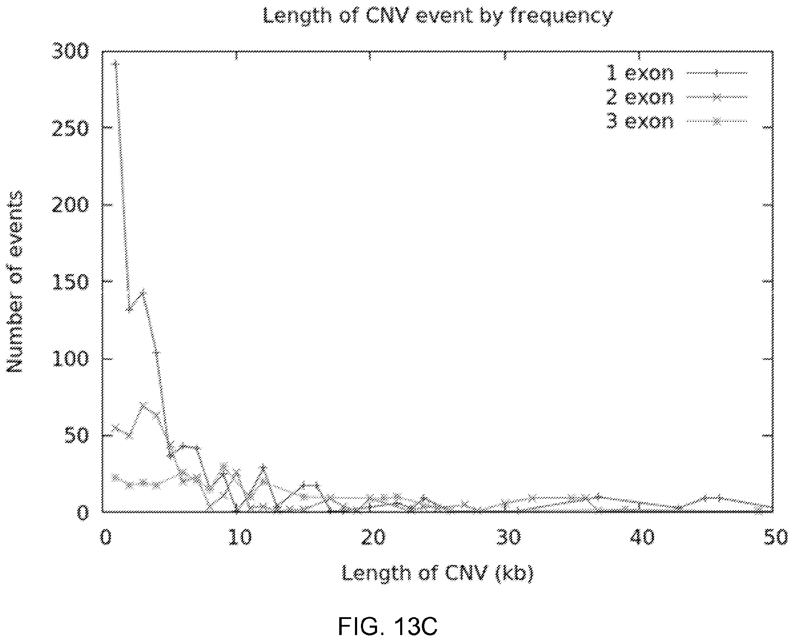

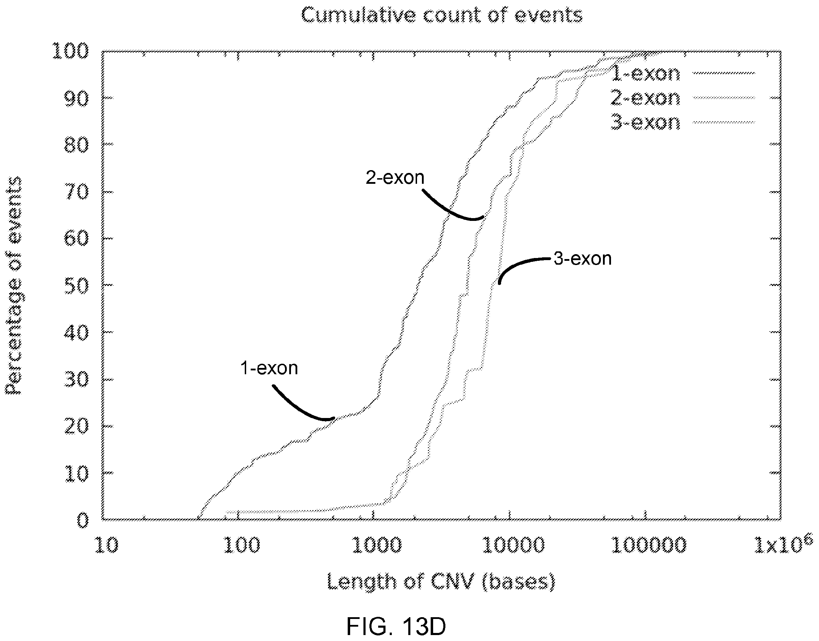

**Figures 13A****,** **13B****,** **13C****,** and **13D** illustrate plots characterizing CNVs in nine biological samples. Figure 13A illustrates the frequency of CNV events overlapping a given number of exons, for each of the nine samples. Figure 13B illustrates the frequency of CNV events overlapping 1 or more exons relative to CNV length, compared to all CNV events (overlapping 0 or more exons), using amalgamated counts for all nine samples. Figure 13C illustrates the frequency of CNV events overlapping 1, 2, or 3 exons relative to CNV length, using amalgamated counts for all nine samples. Figure 13D illustrates the cumulative count of CNV events of varying lengths overlapping 1, 2, or 3 exons, using amalgamated counts for all nine samples.

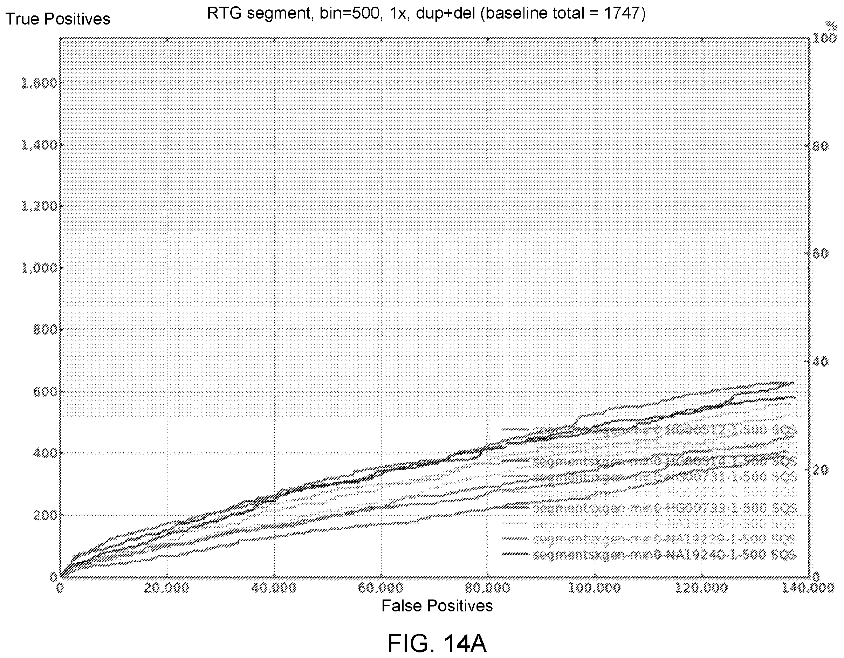

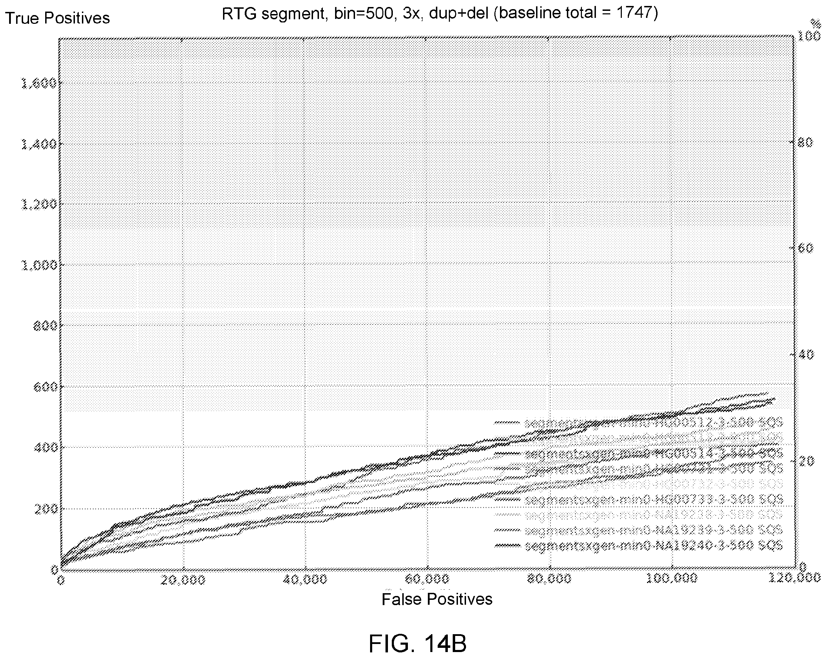

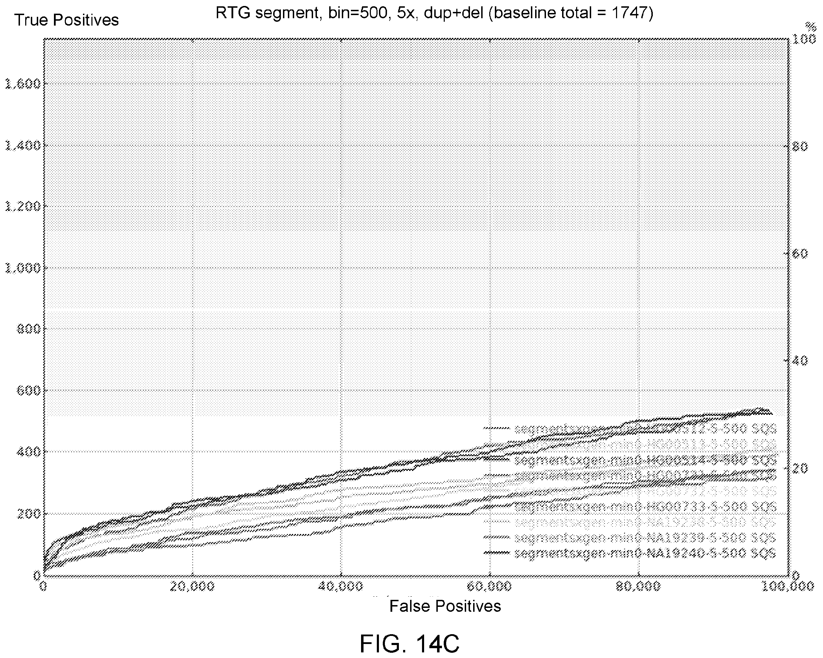

**Figures 14A****,** **14B****,** and **14C** illustrate CNV calling using the RealTimeGenomics (RTG) segment CNV caller, using a bin size of 500 base pairs and simulated coverage of 1X (Figure 14A), 3X (Figure 14B), and 5X (Figure 14C).

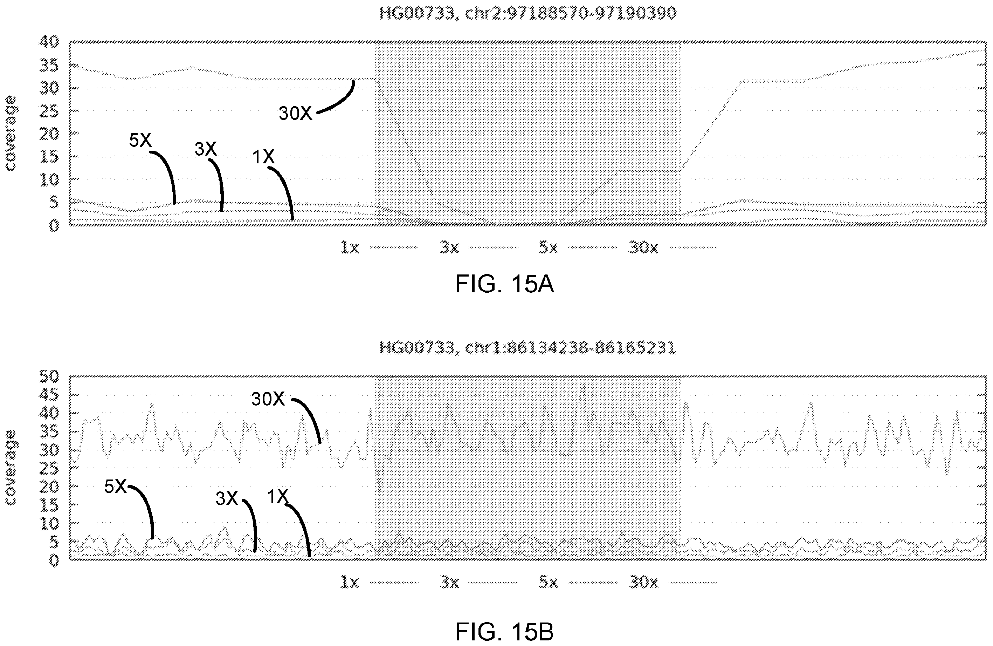

**Figures 15A** and **15B** show examples of CNV calls obtained using the RTG segment CNV caller at varying simulated and full coverages (1X to 30X). Shaded regions of the plot indicate locations of nominal deletions, as provided by baseline calls obtained from a "truth set." Figure 15A illustrates a deletion event that is discernible in the 3X to 30X coverage range. Figure 15B illustrates a deletion event that is not discernible even at 30X coverage.

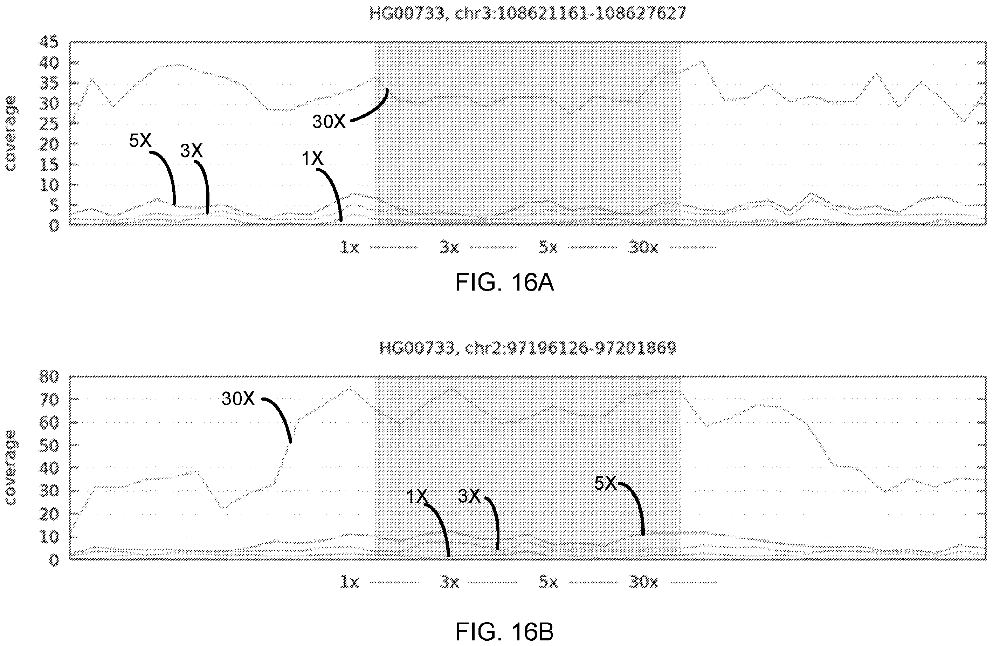

**Figures 16A** and **16B** show examples of CNV calls obtained using the RTG segment CNV caller at varying simulated and full coverages (1X to 30X). Shaded regions of the plot indicate locations of nominal duplications, as provided by baseline calls obtained from a "truth set." Figure 16A illustrates a duplication event that is not discernible even at 30X coverage. Figure 16B illustrates a duplication event that is discernible in the 3X to 30X coverage range.

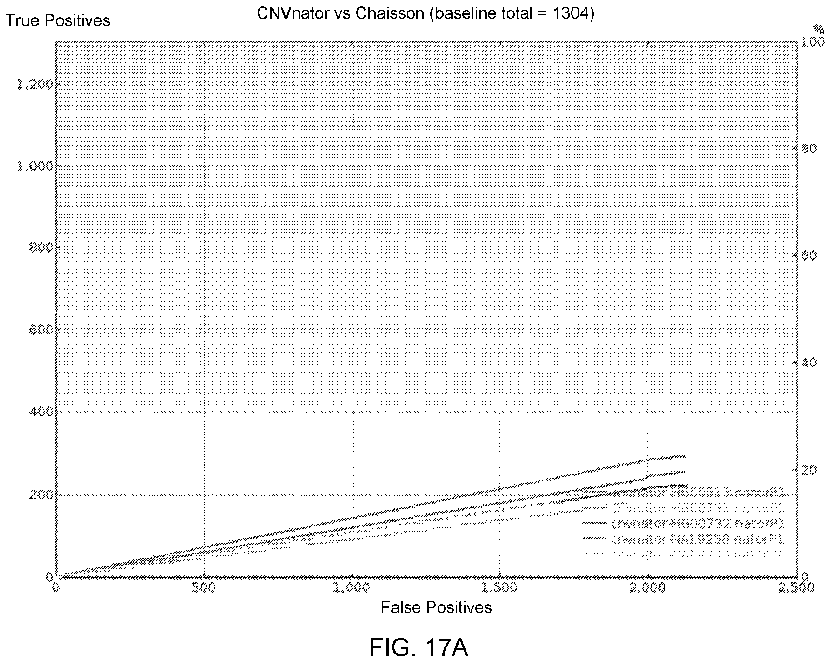

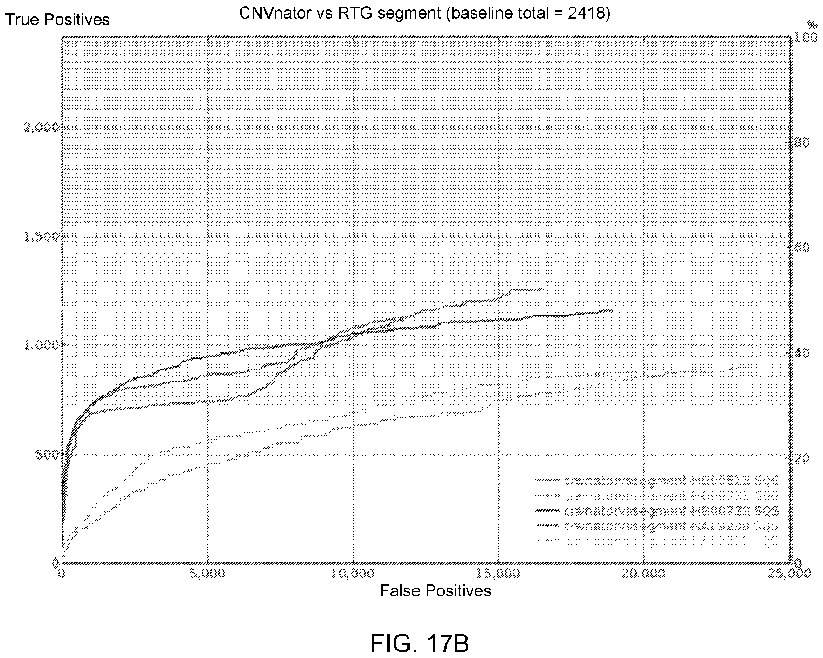

**Figures 17A** and **17B** show examples of CNV calls obtained using the CNVnator CNV caller, using five of the nine samples with known CNV events. Figure 17A shows that CNV calls obtained using CNVnator show poor concordance with a "truth set." Figure 17B shows that CNV calls obtained using CNVnator show moderate concordance with CNV calls obtained using the RTG segment CNV caller at 30X coverage.

### DETAILED DESCRIPTION

### Introduction.

Accurate CNV detection is necessary to improve clinical diagnostics, genetic risk screening, and Mendelian disease tests. A single exon deletion can be highly deleterious and, if missed, patients can be misdiagnosed (false negative). A spurious false positive test could lead to unnecessary medical procedures and cost to the healthcare systems. Advantageously, the systems and methods described herein facilitate higher sensitivity and specificity for the detection of CNVs in disease gene panels, *e.g.,* inherited cancer risk panels including BRCA1, BRCA2, and other genes.

Identification ("calling") of structural variants (SV) from whole genome sequencing data from short reads platform is not without its challenges but can be achieved for most regions of the genome. These structural variants include large deletions and insertions (*e.g.,* by convention, greater than about 50 bp), duplications (increases in the copy number of a genomic region over the copy number of a normal diploid genome), inversions, and translocations. The collection of deletions and duplications are also called copy number variants (CNVs). CNV calling in whole genome sequencing data can be achieved by analysis of depth of coverage, mapping of breakpoint reads, and discordance in apparent insert size for paired-end reads and can be quite accurate for events over a few hundred bases in length to large segments of the chromosome arms, at least for deletions. Some problematic regions include repeats and segmental duplications, but for most coding genes this is not a major problem. Long read sequencing and optical maps can be used to identify SVs in such complex regions.

On the other hand, detecting small CNVs from targeted short-read sequencing data can be challenging. Most conventional methods for detecting CNV events from next-generation sequencing (NGS) rely on detecting changes in the mean or median depth of coverage that such events are expected to cause (*e.g.,* deletions would result in a reduction in depth of coverage, and vice versa for duplications). However, it is particularly difficult to differentiate actual changes in sequencing depth from several types of technical artifacts that change the depth profile irrespective of gene dosage changes, including, but not limited to, a) sequencing biases due to GC content, b) read mapping biases due to repeats, c) segmental duplications, e) paralogous regions, and/or f) systematic capture biases prevalent in targeted sequencing chemistries. Methods to overcome such problems include GC and mappability normalization across "bins" of arbitrary length (*e.g.,* 100 bp), as well as comparing sequencing data to diploid normal samples, bin by bin, sequenced with the same assay. In some embodiments, biases are expected to be similar in the control and test samples, such that, by calculating the depth ratio in the bins, it is possible to derive whether an underlying CNV is present. Adjacent bins deemed to have the same CNV status can be combined, resulting in potentially larger CNV "calls" as outputs, which can then be interpreted for their impact and pathogenicity. While longer structural variants can be more reliably detected in this fashion, small structural variants, particularly those encompassing a single bin, are difficult to differentiate from false positives due to random or systematic read depth fluctuation.

While many of these problems affect both WGS and targeted sequencing, sequencing biases are further exacerbated in targeted sequencing reactions due to differences in probe capture efficiencies and genetic variation under probes, resulting in a much more variable "normal" depth profile in gene panels as compared to WGS. An additional complexity in targeted sequencing is that it often only targets the exons of the genes, as well as a small portion of adjacent upstream and/or downstream intronic sequences to ensure coverage of splice regions. Since the average exon length is only about 150 bp, these sequenced regions may encompass only about 300 bp on average. This means that a single exon deletion is difficult to detect, as sequencing data are typically available from only one or two bins whereas conventional CNV calling algorithms generally require a minimum of 2-3 concordant bins to call a variant. In germline testing, the typical sequencing depth on targeted gene panels is about 200-300X. At this sequencing depth the sensitivity to detect single exon events drops to 50% or less, which is not reliable enough for use in clinical diagnosis. To overcome this loss of sensitivity, the sequencing depth could be increased. However, increasing sequencing depth to a level that supports the sensitivity needed for clinical diagnostics incurs significant economic and throughput costs. For instance, it has been suggested that sensitivity can be substantially increased by sequencing at a depth of about 500-1000X. However, this makes the clinical test prohibitively expensive with current technology. Similarly, while WGS can be used effectively, it is also too expensive for routine gene-panel testing. Yet others have used targeted long read sequencing of entire genes to overcome this problem. But this is not an easily generalizable approach, as custom targeted sequencing assays are needed for each gene, and WGS uses a different sequencing platform than conventionally used for single nucleotide variant identification, increasing logistical costs.

More particularly, the actual genetic variants responsible for single exon CNVs are often much larger than the 150 bp average exon length, and in fact are typically several kb in length (*see, e.g.,* Figure 7). However, the breakpoints for these deletions commonly reside deep in the intronic regions and, thus, a large fraction of such events span regions that are likely invisible to targeted sequencing (*see, e.g.,* Figure 8). Accordingly, WGS is generally more sensitive to such events, but conventional methodologies for WGS at an average sequencing depth of 30-50X are still too expensive to be used as an assay for gene panel tests.

On the other hand, low-coverage (or low-pass/depth) WGS (lc-WGS or LPWGS) has been proposed as an alternative, inexpensive assay to identify gross level chromosomal rearrangements and structural variants (SV), and, through variant imputation, to genotype common variants to perform genome-wide wide association studies (GWAS) or to calculate polygenic risk scores (PRS) derived from such studies. When Ic-WGS is performed to 0.5-1X, it adds just $50 in cost per sample and is sufficient to detect large SVs and perform GWAS/PRS analysis. Thus, it has been proposed as an assay to replace genotyping microarray for such studies and CGC arrays in cytogenetic testing. While SV/CNV data derived from Ic-WGS data is useful for cytogenetics, it lacks the sensitivity and specificity needed for the clinical testing of small events.

What is needed in the art are improved methods and systems for identifying short CNVs, preferably from sequencing data that can also be used to identify single nucleotide variants. Particularly, methods and systems for identifying short CNVs over a panel-targeted (*e.g.,* whole exome or a subset of genes thereof) sequencing backbone at medium to low sequencing depth are desired.

Advantageously, the disclosure provides systems and methods that combine data obtained from panel-targeted sequencing gene panels with signals from Ic-WGS to improve sensitivity and specificity of CNV detection for gene-panel testing in a cost-effective fashion. By combining signals that alone would be likely to have insufficient specificity to call small, exon-level CNVs, the combined assay accomplishes clinical grade variant calling of CNVs, with a sensitivity that is at least equivalent to, if not better than, targeted sequencing performed at a sequencing depth of 1000X or WGS performed at a sequencing depth of 30X. Briefly, the systems and methods provided herein utilize both WES and Ic-WGS data for the same sample to remove false positives and provide accurate CNV calls down to the single exon level. See Figure 9, e.g., in comparison with Figure 8. Lc-WGS data further provides other useful readouts, such as disease risk prediction obtained by calculating polygenic risk scores from imputed variants from Ic-WGS data.

In one aspect, the disclosure provides a method for accurately identifying both small variants (*e.g.,* SNVs and small indels) and CNVs across coding regions for clinical diagnosis and assessment of genetic risk. In some embodiments, risk prediction for common disease can also be provided through calculation of polygenic risk scores and combined with highly penetrant variants for absolute risk predictions.

In some embodiments, such a method combines data from low-coverage WGS (1-3X, which is cost effective) with WES data performed at cost-effective depths (*e.g.,* 60-80X) and jointly analyzes the alignments from both assays to provide accurate CNV calls down to the single exon level. An example implementation of such a method is illustrated in Figure 6.

In some embodiments, methods and systems are provided for calling CNV using combined Ic-WGS and WES data, *e.g.,* as illustrated in step e of Figure 6. In some embodiments, joint CNV calling can be performed using one of a variety of algorithms, including machine learning models, Bayesian PCA models, probabilistic methods, heuristic methods, *etc.* For instance, Figures 10-12 illustrate examples of training, testing, and operating a machine learning method for this task.

In some embodiments, the methods described herein further use reference data generated for a panel of normal samples, *e.g.,* samples, previously analyzed using the same assays, that were determined to be CNV negative for one or more genes of interest.

In some embodiments, the systems and methods described herein facilitate development of gene panel tests for germline testing of inherited disease risk, *e.g.,* inherited breast, ovarian, colon, prostate, or other cancers) derived from rare, highly penetrant pathogenic CNV variants, by combining whole-exome sequencing and/or gene-panel targeted sequencing at cost effective depths (60-80X for WES; 200-300X for smaller panels), with an inexpensive Ic-WGS assay (1-3X).

In some embodiments, the systems and methods described herein are used to complement WES to identify small pathogenic CNV events more accurately for Mendelian disease diagnostics, newborn screening, carrier screening, CDC Tier-1 condition screening, and other disease panels screening.

### Definitions.

The terminology used in the present disclosure is for the purpose of describing particular embodiments only and is not intended to be limiting of the invention. As used in the description of the invention and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the claims, such terms are intended to be inclusive in a manner similar to the term "comprising."

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

It will also be understood that, although the terms first, second, etc. may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

As used herein, the term "subject" refers to any living or non-living human. In some embodiments, a subject is a male or female of any stage (*e.g.,* a man, a woman or a child).

As used herein, the terms "control," "control sample," "reference," "reference sample," "normal," and "normal sample" describe a sample from a subject that does not have a particular condition or is otherwise healthy. In an example, a method as disclosed herein can be performed on a subject having a tumor, where the reference sample is a sample taken from a healthy tissue of the subject. A reference sample can be obtained from the subject, or from a database. The reference can be, *e.g.,* a reference genome that is used to map sequence reads obtained from sequencing a sample from the subject. A reference genome can refer to a haploid or diploid genome to which sequence reads from the biological sample and a constitutional sample can be aligned and compared. An example of a constitutional sample can be DNA of whole blood or blood cells obtained from the subject. For a haploid genome, there can be only one nucleotide at each locus. For a diploid genome, heterozygous loci can be identified, each heterozygous locus can have two alleles, where either allele can allow a match for alignment to the locus.

As used herein, the term "locus" refers to a position (*e.g.,* a site) within a genome, *e.g.,* on a particular chromosome. In some embodiments, a locus refers to a single nucleotide position within a genome, i.e., on a particular chromosome. In some embodiments, a locus refers to a small group of nucleotide positions within a genome, *e.g.,* as defined by a mutation (*e.g.,* substitution, insertion, or deletion) of consecutive nucleotides within a cancer genome. Because normal mammalian cells have diploid genomes, a normal mammalian genome (*e.g.,* a human genome) will generally have two copies of every locus in the genome, or at least two copies of every locus located on the autosomal chromosomes, *e.g.,* one copy on the maternal autosomal chromosome and one copy on the paternal autosomal chromosome.

As used herein, the term "allele" refers to a particular sequence of one or more nucleotides at a chromosomal locus.

As used herein, the term "reference allele" refers to the sequence of one or more nucleotides at a chromosomal locus that is either the predominant allele represented at that chromosomal locus within the population of the species (*e.g.,* the "wild-type" sequence), or an allele that is predefined within a reference genome for the species.

As used herein, the term "variant allele" refers to a sequence of one or more nucleotides at a chromosomal locus that is either not the predominant allele represented at that chromosomal locus within the population of the species (*e.g.,* not the "wild-type" sequence), or not an allele that is predefined within a reference genome for the species.

As used herein, the term "single nucleotide variant" or "SNV" refers to a substitution of one nucleotide to a different nucleotide at a position (*e.g.,* site) of a nucleotide sequence, *e.g.,* a sequence read from an individual. A substitution from a first nucleobase X to a second nucleobase Y may be denoted as "X>Y." For example, a cytosine to thymine SNV may be denoted as "C>T."

As used herein, the term "mutation" or "variant" refers to a detectable change in the genetic material of one or more cells. In a particular example, one or more mutations can be found in, and can identify, cancer cells (*e.g.,* driver and passenger mutations). A mutation can be transmitted from a parent cell to a daughter cell. A person having skill in the art will appreciate that a genetic mutation (*e.g.,* a driver mutation) in a parent cell can induce additional, different mutations (*e.g.,* passenger mutations) in a daughter cell. A mutation generally occurs in a nucleic acid. In a particular example, a mutation can be a detectable change in one or more deoxyribonucleic acids or fragments thereof. A mutation generally refers to nucleotides that are added, deleted, substituted for, inverted, or transposed to a new position in a nucleic acid. A mutation can be a spontaneous mutation or an experimentally induced mutation. A mutation in the sequence of a particular tissue is an example of a "tissue-specific allele." For example, a tumor can have a mutation that results in an allele at a locus that does not occur in normal cells. Another example of a "tissue-specific allele" is a fetal-specific allele that occurs in the fetal tissue, but not the maternal tissue.

As used herein, the term "loss of heterozygosity" refers to the loss of one copy of a segment (*e.g.,* including part or all of one or more genes) of the genome of a diploid subject (*e.g.,* a human) or loss of one copy of a sequence encoding a functional gene product in the genome of the diploid subject, in a tissue, *e.g.,* a cancerous tissue, of the subject. As used herein, when referring to a metric representing loss of heterozygosity across the entire genome of the subject, loss of heterozygosity is caused by the loss of one copy of various segments in the genome of the subject. Loss of heterozygosity across the entire genome may be estimated without sequencing the entire genome of a subject, and such methods for such estimations based on gene panel targeting-based sequencing methodologies are described in the art. Accordingly, in some embodiments, a metric representing loss of heterozygosity across the entire genome of a tissue of a subject is represented as a single value, *e.g.,* a percentage or fraction of the genome. In some cases, a tumor is composed of various sub-clonal populations, each of which may have a different degree of loss of heterozygosity across their respective genomes. Accordingly, in some embodiments, loss of heterozygosity across the entire genome of a cancerous tissue refers to an average loss of heterozygosity across a heterogeneous tumor population. As used herein, when referring to a metric for loss of heterozygosity in a particular gene, *e.g.,* a DNA repair protein such as a protein involved in the homologous DNA recombination pathway (*e.g.,* BRCA1 or BRCA2), loss of heterozygosity refers to complete or partial loss of one copy of the gene encoding the protein in the genome of the tissue and/or a mutation in one copy of the gene that prevents translation of a full-length gene product, *e.g.,* a frameshift or truncating (creating a premature stop codon in the gene) mutation in the gene of interest. In some cases, a tumor is composed of various sub-clonal populations, each of which may have a different mutational status in a gene of interest. Accordingly, in some embodiments, loss of heterozygosity for a particular gene of interest is represented by an average value for loss of heterozygosity for the gene across all sequenced sub-clonal populations of the cancerous tissue. In other embodiments, loss of heterozygosity for a particular gene of interest is represented by a count of the number of unique incidences of loss of heterozygosity in the gene of interest across all sequenced sub-clonal populations of the cancerous tissue (*e.g.,* the number of unique frame-shift and/or truncating mutations in the gene identified in the sequencing data).

As used herein the term "cancer," "cancerous tissue," or "tumor" refers to an abnormal mass of tissue in which the growth of the mass surpasses and is not coordinated with the growth of normal tissue. A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein. A cancerous tissue can refer to blood cells if the cancer is a hematological (blood) cancer.

As used herein, the terms "sequencing," "sequence determination," and the like as used herein refers generally to any and all biochemical processes that may be used to determine the order of biological macromolecules such as nucleic acids or proteins. For example, sequencing data can include all or a portion of the nucleotide bases in a nucleic acid molecule such as an mRNA transcript or a genomic locus.

As used herein, the term "sequence reads" or "reads" refers to nucleotide sequences produced by any sequencing process described herein or known in the art. Reads can be generated from one end of nucleic acid fragments ("single-end reads"), and sometimes are generated from both ends of nucleic acids (*e.g.,* paired-end reads, double-end reads). The length of the sequence read is often associated with the particular sequencing technology. High-throughput methods, for example, provide sequence reads that can vary in size from tens to hundreds of base pairs (bp). In some embodiments, the sequence reads are of a mean, median or average length of about 15 bp to 900 bp long (*e.g.,* about 20 bp, about 25 bp, about 30 bp, about 35 bp, about 40 bp, about 45 bp, about 50 bp, about 55 bp, about 60 bp, about 65 bp, about 70 bp, about 75 bp, about 80 bp, about 85 bp, about 90 bp, about 95 bp, about 100 bp, about 110 bp, about 120 bp, about 130, about 140 bp, about 150 bp, about 200 bp, about 250 bp, about 300 bp, about 350 bp, about 400 bp, about 450 bp, or about 500 bp. In some embodiments, the sequence reads are of a mean, median or average length of about 1000 bp, 2000 bp, 5000 bp, 10,000 bp, or 50,000 bp or more. Nanopore sequencing, for example, can provide sequence reads that can vary in size from tens to hundreds to thousands of base pairs. Illumina parallel sequencing can provide sequence reads that do not vary as much, for example, most of the sequence reads can be smaller than 200 bp. A sequence read (or sequencing read) can refer to sequence information corresponding to a nucleic acid molecule (*e.g.,* a string of nucleotides). For example, a sequence read can correspond to a string of nucleotides (*e.g.,* about 20 to about 150) from part of a nucleic acid fragment, can correspond to a string of nucleotides at one or both ends of a nucleic acid fragment, or can correspond to nucleotides of the entire nucleic acid fragment. A sequence read can be obtained in a variety of ways, *e.g.,* using sequencing techniques or using probes, *e.g.,* in hybridization arrays or capture probes, or amplification techniques, such as the polymerase chain reaction (PCR) or linear amplification using a single primer or isothermal amplification.

As used herein, the term "read segment" or "read" refers to any nucleotide sequences including sequence reads obtained from an individual and/or nucleotide sequences derived from the initial sequence read from a sample obtained from an individual. For example, a read segment can refer to an aligned sequence read, a collapsed sequence read, or a stitched read. Furthermore, a read segment can refer to an individual nucleotide base, such as a single nucleotide variant.

As used herein, the term, "reference exome" refers to any particular known, sequenced or characterized exome, whether partial or complete, of any tissue from any organism or pathogen that may be used to reference identified sequences from a subject. Example reference exomes used for human subjects as well as many other organisms are provided in the on-line genome browser hosted by the National Center for Biotechnology Information ("NCBI").

As used herein, the term "reference genome" refers to any particular known, sequenced or characterized genome, whether partial or complete, of any organism or pathogen that may be used to reference identified sequences from a subject. Exemplary reference genomes used for human subjects as well as many other organisms are provided in the on-line genome browser hosted by the National Center for Biotechnology Information ("NCBI") or the University of California, Santa Cruz (UCSC). A "genome" refers to the complete genetic information of an organism or pathogen, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. The reference genome can be viewed as a representative example of a species' set of genes. In some embodiments, a reference genome comprises sequences assigned to chromosomes. Exemplary human reference genomes include but are not limited to NCBI build 34 (UCSC equivalent: hg16), NCBI build 35 (UCSC equivalent: hg17), NCBI build 36.1 (UCSC equivalent: hg18), GRCh37 (UCSC equivalent: hg19), and GRCh38 (UCSC equivalent: hg38).

As used herein, the term "assay" refers to a technique for determining a property of a substance, *e.g.,* a nucleic acid, a protein, a cell, a tissue, or an organ. An assay (*e.g.,* a first assay or a second assay) can comprise a technique for determining the copy number variation of nucleic acids in a sample, the methylation status of nucleic acids in a sample, the fragment size distribution of nucleic acids in a sample, the mutational status of nucleic acids in a sample, or the fragmentation pattern of nucleic acids in a sample. Any assay known to a person having ordinary skill in the art can be used to detect any of the properties of nucleic acids mentioned herein. Properties of a nucleic acids can include a sequence, genomic identity, copy number, methylation state at one or more nucleotide positions, size of the nucleic acid, presence or absence of a mutation in the nucleic acid at one or more nucleotide positions, and pattern of fragmentation of a nucleic acid (*e.g.,* the nucleotide position(s) at which a nucleic acid fragments). An assay or method can have a particular sensitivity and/or specificity, and their relative usefulness as a diagnostic tool can be measured using ROC-AUC statistics.

The term "classification" can refer to any number(s) or other characters(s) that are associated with a particular property of a sample. For example, in some embodiments, the term "classification" can refer to a type of cancer in a subject or sample, a stage of cancer in a subject or sample, a prognosis for a cancer in a subject or sample, a tumor load in a subject, a presence of tumor metastasis in a subject, and the like. The classification can be binary (*e.g.,* positive or negative) or have more levels of classification (*e.g.,* a scale from 1 to 10 or 0 to 1). The terms "cutoff" and "threshold" can refer to predetermined numbers used in an operation. For example, a cutoff size can refer to a size above which fragments are excluded. A threshold value can be a value above or below which a particular classification applies. Either of these terms can be used in either of these contexts.

As used interchangeably herein, the term "classifier" or "model" refers to a machine learning model or algorithm.

In some embodiments, a classifier is an unsupervised learning algorithm. One example of an unsupervised learning algorithm is cluster analysis.

In some embodiments, a classifier is supervised machine learning. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, GradientBoosting, mixture models, hidden Markov models, Gaussian NB algorithms, linear discriminant analysis, or any combinations thereof. In some embodiments, a classifier is a multinomial classifier algorithm. In some embodiments, a classifier is a 2-stage stochastic gradient descent (SGD) model. In some embodiments, a classifier is a deep neural network (*e.g.,* a deep-and-wide sample-level classifier).

*Neural networks.* In some embodiments, the classifier is a neural network (*e.g.,* a convolutional neural network and/or a residual neural network). Neural network algorithms, also known as artificial neural networks (ANNs), include convolutional and/or residual neural network algorithms (deep learning algorithms). Neural networks can be machine learning algorithms that may be trained to map an input data set to an output data set, where the neural network comprises an interconnected group of nodes organized into multiple layers of nodes. For example, the neural network architecture may comprise at least an input layer, one or more hidden layers, and an output layer. The neural network may comprise any total number of layers, and any number of hidden layers, where the hidden layers function as trainable feature extractors that allow mapping of a set of input data to an output value or set of output values. As used herein, a deep learning algorithm (DNN) can be a neural network comprising a plurality of hidden layers, *e.g.,* two or more hidden layers. Each layer of the neural network can comprise a number of nodes (or "neurons"). A node can receive input that comes either directly from the input data or the output of nodes in previous layers, and perform a specific operation, *e.g.,* a summation operation. In some embodiments, a connection from an input to a node is associated with a parameter (*e.g.,* a weight and/or weighting factor). In some embodiments, the node may sum up the products of all pairs of inputs, xᵢ, and their associated parameters. In some embodiments, the weighted sum is offset with a bias, b. In some embodiments, the output of a node or neuron may be gated using a threshold or activation function, f, which may be a linear or non-linear function. The activation function may be, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

The weighting factors, bias values, and threshold values, or other computational parameters of the neural network, may be "taught" or "learned" in a training phase using one or more sets of training data. For example, the parameters may be trained using the input data from a training data set and a gradient descent or backward propagation method so that the output value(s) that the ANN computes are consistent with the examples included in the training data set. The parameters may be obtained from a back propagation neural network training process.

Any of a variety of neural networks may be suitable for use in the present disclosure. Examples can include, but are not limited to, feedforward neural networks, radial basis function networks, recurrent neural networks, residual neural networks, convolutional neural networks, residual convolutional neural networks, and the like, or any combination thereof. In some embodiments, the machine learning makes use of a pre-trained and/or transfer-learned ANN or deep learning architecture. Convolutional and/or residual neural networks can be used in the present disclosure.

For instance, a deep neural network classifier comprises an input layer, a plurality of individually parameterized (*e.g.,* weighted) convolutional layers, and an output scorer. The parameters (*e.g.,* weights) of each of the convolutional layers as well as the input layer contribute to the plurality of parameters (*e.g.,* weights) associated with the deep neural network classifier. In some embodiments, at least 100 parameters, at least 1000 parameters, at least 2000 parameters or at least 5000 parameters are associated with the deep neural network classifier. As such, deep neural network classifiers require a computer to be used because they cannot be mentally solved. In other words, given an input to the classifier, the classifier output needs to be determined using a computer rather than mentally in such embodiments. See, for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press.

Neural network algorithms, including convolutional neural network algorithms, suitable for use as classifiers are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology. Additional example neural networks suitable for use as classifiers are disclosed in Duda et al., 2001, Pattern Classification, Second Edition, John Wiley & Sons, Inc., New York; and Hastie et al., 2001, The Elements of Statistical Learning, Springer-Verlag, New Yorkin its entirety. Additional example neural networks suitable for use as classifiers are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New Yorkin its entirety.

*Support vector machines.* In some embodiments, the classifier is a support vector machine (SVM). SVM algorithms suitable for use as classifiers are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space can correspond to a non-linear decision boundary in the input space. In some embodiments, the plurality of parameters (*e.g.,* weights) associated with the SVM define the hyper-plane. In some embodiments, the hyper-plane is defined by at least 10, at least 20, at least 50, or at least 100 parameters and the SVM classifier requires a computer to calculate because it cannot be mentally solved.

*Naïve Bayes algorithms.* In some embodiments, the classifier is a Naive Bayes algorithm. Naïve Bayes classifiers suitable for use as classifiers are disclosed, for example, in Ng et al., 2002, "On discriminative vs. generative classifiers: A comparison of logistic regression and naive Bayes," Advances in Neural Information Processing Systems, 14. A Naive Bayes classifier is any classifier in a family of "probabilistic classifiers" based on applying Bayes' theorem with strong (naïve) independence assumptions between the features. In some embodiments, they are coupled with Kernel density estimation. See, for example, Hastie et al., 2001, The elements of statistical learning : data mining, inference, and prediction, eds. Tibshirani and Friedman, Springer, New York.

*Nearest neighbor algorithms.* In some embodiments, a classifier is a nearest neighbor algorithm. Nearest neighbor classifiers can be memory-based and include no classifier to be fit. For nearest neighbors, given a query point x₀ (a test subject), the k training points x_{(*r*)}, r, .. , k (here the training subjects) closest in distance to x₀ are identified and then the point x₀ is classified using the k nearest neighbors. Here, the distance to these neighbors is a function of the abundance values of the discriminating gene set. In some embodiments, Euclidean distance in feature space is used to determine distance as *d*_{(*i*)} = ∥*x*_{(*i*)} - *x*_{(*o*)}∥. Typically, when the nearest neighbor algorithm is used, the abundance data used to compute the linear discriminant is standardized to have mean zero and variance 1. The nearest neighbor rule can be refined to address issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York.

A k-nearest neighbor classifier is a non-parametric machine learning method in which the input consists of the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. See, Duda et al., 2001, Pattern Classification, Second Edition, John Wiley & Sons. In some embodiments, the number of distance calculations needed to solve the k-nearest neighbor classifier is such that a computer is used to solve the classifier for a given input because it cannot be mentally performed.

*Random forest, decision tree, and boosted tree algorithms.* In some embodiments, the classifier is a decision tree. Decision trees suitable for use as classifiers are described generally by Duda, 2001, Pattern Classification, John Wiley & Sons, Inc., New York, pp. 395-396. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412. CART, MART, and C4.5 are described in Hastie et al., 2001, The Elements of Statistical Learning, Springer-Verlag, New York, Chapter 9. Random Forests are described in Breiman, 1999, "Random ForestsRandom Features," Technical Report 567, Statistics Department, U.C. Berkeley, September 1999. In some embodiments, the decision tree classifier includes at least 10, at least 20, at least 50, or at least 100 parameters (*e.g.,* weights and/or decisions) and requires a computer to calculate because it cannot be mentally solved.

*Regression.* In some embodiments, the classifier uses a regression algorithm. A regression algorithm can be any type of regression. For example, in some embodiments, the regression algorithm is logistic regression. In some embodiments, the regression algorithm is logistic regression with lasso, L2 or elastic net regularization. In some embodiments, those extracted features that have a corresponding regression coefficient that fails to satisfy a threshold value are pruned (removed from) consideration. In some embodiments, a generalization of the logistic regression model that handles multicategory responses is used as the classifier. Logistic regression algorithms are disclosed in Agresti, An Introduction to Categorical Data Analysis, 1996, Chapter 5, pp. 103-144, John Wiley & Son, New York. In some embodiments, the classifier makes use of a regression model disclosed in Hastie et al., 2001, The Elements of Statistical Learning, Springer-Verlag, New York. In some embodiments, the logistic regression classifier includes at least 10, at least 20, at least 50, at least 100, or at least 1000 parameters (*e.g.,* weights) and requires a computer to calculate because it cannot be mentally solved.

*Linear discriminant analysis algorithms.* Linear discriminant analysis (LDA), normal discriminant analysis (NDA), or discriminant function analysis can be a generalization of Fisher's linear discriminant, a method used in statistics, pattern recognition, and machine learning to find a linear combination of features that characterizes or separates two or more classes of objects or events. The resulting combination can be used as the classifier (linear classifier) in some embodiments of the present disclosure.

*Mixture model and Hidden Markov model.* In some embodiments, the classifier is a mixture model, such as that described in McLachlan et al., Bioinformatics 18(3):413-422, 2002. In some embodiments, in particular, those embodiments including a temporal component, the classifier is a hidden Markov model such as described by Schliep et al., 2003, Bioinformatics 19(1):i255-i263.

*Clustering.* In some embodiments, the classifier is an unsupervised clustering model. In some embodiments, the classifier is a supervised clustering model. Clustering algorithms suitable for use as classifiers are described, for example, at pages 211-256 of Duda and Hart, Pattern Classification and Scene Analysis, 1973, John Wiley & Sons, Inc., New York, (hereinafter "Duda 1973"). The clustering problem can be described as one of finding natural groupings in a dataset. To identify natural groupings, two issues can be addressed. First, a way to measure similarity (or dissimilarity) between two samples can be determined. This metric (*e.g.,* similarity measure) can be used to ensure that the samples in one cluster are more like one another than they are to samples in other clusters. Second, a mechanism for partitioning the data into clusters using the similarity measure can be determined. One way to begin a clustering investigation can be to define a distance function and to compute the matrix of distances between all pairs of samples in the training set. If distance is a good measure of similarity, then the distance between reference entities in the same cluster can be significantly less than the distance between the reference entities in different clusters. However, clustering may not use a distance metric. For example, a nonmetric similarity function s(x, x') can be used to compare two vectors x and x'. s(x, x') can be a symmetric function whose value is large when x and x' are somehow "similar." Once a method for measuring "similarity" or "dissimilarity" between points in a dataset has been selected, clustering can use a criterion function that measures the clustering quality of any partition of the data. Partitions of the data set that extremize the criterion function can be used to cluster the data. Particular exemplary clustering techniques that can be used in the present disclosure can include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering (*e.g.,* with no preconceived number of clusters and/or no predetermination of cluster assignments).

*Ensembles of classifiers and boosting.* In some embodiments, an ensemble (two or more) of classifiers is used. In some embodiments, a boosting technique such as AdaBoost is used in conjunction with many other types of learning algorithms to improve the performance of the classifier. In this approach, the output of any of the classifiers disclosed herein, or their equivalents, is combined into a weighted sum that represents the final output of the boosted classifier. In some embodiments, the plurality of outputs from the classifiers is combined using any measure of central tendency known in the art, including but not limited to a mean, median, mode, a weighted mean, weighted median, weighted mode, etc. In some embodiments, the plurality of outputs is combined using a voting method. In some embodiments, a respective classifier in the ensemble of classifiers is weighted or unweighted.

As used herein, the term "parameter" refers to any coefficient or, similarly, any value of an internal or external element (*e.g.,* a weight and/or a hyperparameter) in an algorithm, model, regressor, and/or classifier that can affect (*e.g.,* modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (*e.g.,* a feature) to an algorithm, model, regressor, and/or classifier. As a nonlimiting example, in some embodiments, a parameter is used to increase or decrease the influence of a node (*e.g.,* of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (*e.g.,* by error minimization and/or backpropagation methods). In some embodiments, an algorithm, model, regressor, and/or classifier of the present disclosure includes a plurality of parameters. In some embodiments, the plurality of parameters is n parameters, where: n ≥ 2; n ≥ 5; n ≥ 10; n ≥ 25; n ≥ 40; n ≥ 50; n ≥ 75; n ≥ 100; n ≥ 125; n ≥ 150; n ≥ 200; n ≥ 225; n ≥ 250; n ≥ 350; n ≥ 500; n ≥ 600; n ≥ 750; n ≥ 1,000; n ≥ 2,000; n ≥ 4,000; n ≥ 5,000; n ≥ 7,500; n ≥ 10,000; n ≥ 20,000; n ≥ 40,000; n ≥ 75,000; n ≥ 100,000; n ≥ 200,000; n ≥ 500,000, n ≥ 1 x 10⁶, n ≥ 5 x 10⁶, or n ≥ 1 x 10⁷. As such, the algorithms, models, regressors, and/or classifiers of the present disclosure cannot be mentally performed. In some embodiments n is between 10,000 and 1 x 10⁷, between 100,000 and 5 x 10⁶, or between 500,000 and 1 x 10⁶. In some embodiments, the algorithms, models, regressors, and/or classifier of the present disclosure operate in a k-dimensional space, where k is a positive integer of 5 or greater (*e.g.,* 5, 6, 7, 8, 9, 10, etc.). As such, the algorithms, models, regressors, and/or classifiers of the present disclosure cannot be mentally performed.

Several aspects are described herein with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

Reference is made herein to embodiments, examples of which are illustrated in the accompanying drawings. In the present disclosure, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

### Example System Embodiments.

Now that an overview of some aspects of the present disclosure and some definitions used in the present disclosure have been provided, details of an exemplary system for providing clinical support for personalized therapy for various diseases and disorders (*e.g.,* cardiovascular conditions, neurological conditions, cancers, etc.) are now described in conjunction with Figures 1A, 1B, 1C, 1D, and 1E. Figures 1A, 1B, 1C, 1D, and 1E collectively illustrate the topology of an example system for providing clinical support for personalized therapy. Advantageously, the example system illustrated in Figures 1A, 1B, 1C, 1D, and 1E improves upon conventional methods for providing clinical support for personalized therapy by improving detection of copy number variations, and particularly by identifying CNVs overlapping with only one or two exons, *e.g.,* from panel sequencing data that is also useful for identifying single nucleotide variants.

Figure 1A is a block diagram illustrating a system in accordance with some implementations. The device 100 in some implementations includes one or more processing units CPU(s) 102 (also referred to as processors), one or more network interfaces 104, a user interface 106, *e.g.,* including a display 108 and/or an input 110 (*e.g.,* a mouse, touchpad, keyboard, *etc*.)*,* a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:
- an operating system 116, which includes procedures for handling various basic system services and for performing hardware dependent tasks;
- a network communication module (or instructions) 118 for connecting the system 100 with other devices and/or a communication network 105;
- a test patient data store 120 for storing one or more collections of features from patients (*e.g.,* subjects);
- a bioinformatics module 140 for processing sequencing data and extracting features from sequencing data, *e.g.,* from liquid biopsy sequencing assays;
- a feature analysis module 160 for evaluating patient features, e*.g.,* genomic alterations, compound genomic features, and clinical features; and
- a reporting module 180 for generating and transmitting reports that provide clinical support for personalized cancer therapy.

Although Figures 1A, 1B, 1C, 1D, and 1E depict various components of a "system 100," the figures are intended more as a functional description of the various features that may be present in computer systems than as a structural schematic of the implementations described herein. In practice, items shown separately could be combined and some items could be separated. Moreover, although Figure 1 depicts certain data and modules in non-persistent memory 111, some or all of these data and modules may be in persistent memory 112. For example, in various implementations, one or more of the above identified elements are stored in one or more of the previously mentioned memory devices and correspond to a set of instructions for performing a function described above. The above identified modules, data, or programs (*e.g.,* sets of instructions) need not be implemented as separate software programs, procedures, datasets, or modules, and thus various subsets of these modules and data may be combined or otherwise re-arranged in various implementations.

In some implementations, the non-persistent memory 111 optionally stores a subset of the modules and data structures identified above. Furthermore, in some embodiments, the memory stores additional modules and data structures not described above. In some embodiments, one or more of the above-identified elements is stored in a computer system, other than that of system 100, that is addressable by system 100 so that system 100 may retrieve all or a portion of such data when needed.

For purposes of illustration in Figure 1A, system 100 is represented as a single computer that includes all of the functionality for providing clinical support for personalized cancer therapy. However, while a single machine is illustrated, the term "system" shall also be taken to include any collection of machines that individually or jointly execute a set (or multiple sets) of instructions to perform any one or more of the methodologies discussed herein.

For example, in some embodiments, system 100 includes one or more computers. In some embodiments, the functionality for providing clinical support for personalized cancer therapy is spread across any number of networked computers and/or resides on each of several networked computers and/or is hosted on one or more virtual machines at a remote location accessible across the communications network 105. For example, different portions of the various modules and data stores illustrated in Figures 1A, 1B, 1C, 1D, and 1E can be stored and/or executed on the various instances of a processing device and/or processing server/database in the distributed diagnostic environment 210 illustrated in Figure 2B (*e.g.,* processing devices 224, 234, 244, and 254, processing server 262, and database 264).

The system may operate in the capacity of a server or a client machine in a client-server network environment, as a peer machine in a peer-to-peer (or distributed) network environment, or as a server or a client machine in a cloud computing infrastructure or environment. The system may be a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations may involve specialized hardware, software, or a combination of hardware and software.

One of skill in the art will appreciate that any of a wide array of different computer topologies are used for the application and all such topologies are within the scope of the present disclosure.

### Test Patient Data Store (120)

Referring to Figure 1B, in some embodiments, the system (*e.g.,* system 100) includes a patient data store 120 that stores data for patients 121-1 to 121-M including one or more sequencing data 122, feature data 125, and clinical assessments 139. These data are used and/or generated by the various processes stored in the bioinformatics module 140 and feature analysis module 160 of system 100, to ultimately generate a report providing clinical support for personalized therapy of a patient. While the feature scope of patient data 121 across all patients may be informationally dense, an individual patient's feature set may be sparsely populated across the entirety of the collective feature scope of all features across all patients. That is to say, the data stored for one patient may include a different set of features that the data stored for another patient. Further, while illustrated as a single data construct in Figure 1B, different sets of patient data may be stored in different databases or modules spread across one or more system memories.

In some embodiments, sequencing data 122 from one or more sequencing reactions 122-i, including a plurality of sequence reads 123-1 to 123-K, is stored in the test patient data store 120. The data store may include different sets of sequencing data from a single subject, corresponding to different samples from the patient, *e.g.,* salivary samples, blood samples, solid tissue samples, tumor samples, and/or to samples acquired at different times, *e.g.,* while monitoring the progression, regression, remission, and/or recurrence of a disease or disorder in a subject. The sequence reads may be in any suitable file format, *e.g.,* BCL, FASTA, FASTQ, *etc.* In some embodiments, sequencing data 122 is accessed by a sequencing data processing module 141, which performs various pre-processing, genome alignment, and demultiplexing operations, as described in detail below with reference to bioinformatics module 140. In some embodiments, sequence data that has been aligned to a reference construct, *e.g.,* BAM file 124, is stored in test patient data store 120.

In some embodiments, the test patient data store 120 includes feature data 125, *e.g.,* that is useful for identifying clinical support for personalized therapy. In some embodiments, the feature data 125 includes personal characteristics 126 of the patient, such as patient name, date of birth, gender, ethnicity, physical address, smoking status, alcohol consumption characteristic, anthropomorphic data, *etc.*

In some embodiments, the feature data 125 includes medical history data 127 for the patient, (*e.g.,* date of initial disorder diagnosis, previous treatments and outcomes, adverse effects of therapy, therapy group history, clinical trial history, previous and current medications, surgical history, *etc*.), previous or current symptoms, previous or current therapies, previous treatment outcomes, previous disease diagnoses, diagnoses of depression, diagnoses of other physical or mental maladies, and family medical history. In some embodiments, the feature data 125 includes clinical features 128, such as pathology data 128-1, medical imaging data 128-2, and tissue culture and/or tissue organoid culture data 128-3.

In some embodiments, yet other clinical features, such as previous laboratory testing results, are stored in the test patient data store 120. Medical history data 127 and clinical features may be collected from various sources, including at intake directly from the patient, from an electronic medical record (EMR) or electronic health record (EHR) for the patient, or curated from other sources, such as fields from various testing records (*e.g.,* genetic sequencing reports).

In some embodiments, the feature data 125 includes genomic features 131 for the patient. Non-limiting examples of genomic features include allelic states 132 (*e.g.,* the identity of alleles at one or more loci, support for wild type or variant alleles at one or more loci, support for SNVs/MNVs at one or more loci, support for indels at one or more loci, and/or support for gene rearrangements at one or more loci), methylation states 134 (*e.g.,* a distribution of methylation patterns at one or more loci and/or support for aberrant methylation patterns at one or more loci), genomic copy numbers 135 (*e.g.,* a copy number value at one or more loci and/or support for an aberrant (increased or decreased) copy number at one or more loci). In some embodiments, e.g., when the methods and systems described herein are used for precision oncology, the feature data includes one or more tumor-specific genomic features, e.g., allelic fractions (*e.g*., ratios of variant to reference alleles (or vice versa), tumor mutational burden (*e.g.,* a measure of the number of mutations in the cancer genome of the subject), microsatellite instability status (*e.g.,* a measure of the repeated unit length at one or more microsatellite loci and/or a classification of the MSI status for the patient's cancer), tumor ploidy, and homologous recombination deficiency (HRD) status.

In some embodiments, one or more of the genomic features 131 (e.g., that are used to generate the mapped data sets applied to the joint CNV calling models) are determined by a nucleic acid bioinformatics pipeline, *e.g.,* as described in detail below with reference to Figure 4. For example, in some embodiments, the feature data 125 includes bin values 135-bv derived from sequence reads 123 from low-pass whole genome sequencing and/or targeted panel sequencing reactions (e.g., bin values 135-wgs-bv and 135-ps-bv, as illustrated in Figure 1C). Similarly, in some embodiments, the feature data 125 includes bin copy number states 135-cns, e.g., derived from bin values 135-bv, from low-pass whole genome sequencing and/or targeted panel sequencing reactions (e.g., bin values 135-wgs-cns and 135-ps-cns, as illustrated in Figure 1C).

For example, in some embodiments, the feature data 125 include genomic copy numbers 135 (*e.g.,* 135-1 for Patient 1 121-1), as determined using a bioinformatics pipeline as described in further detail below with reference to Figures 1, 4, and 5. In some embodiments, one or more of the genomic features 131 are obtained from an external source, *e.g.,* not connected to the bioinformatics pipeline as described below.

Referring again to Figure 1B, in some embodiments, the feature data 125 further includes data 138 from other -omics fields of study. Non-limiting examples of -omics fields of study that may yield feature data useful for providing clinical support for personalized cancer therapy include transcriptomics, epigenomics, proteomics, metabolomics, metabonomics, microbiomics, lipidomics, glycomics, cellomics, and organoidomics.

In some embodiments, yet other features may include features derived from machine learning approaches, *e.g.,* based at least in part on evaluation of any relevant molecular or clinical features, considered alone or in combination, not limited to those listed above. For instance, in some embodiments, one or more latent features learned from evaluation of cancer patient training datasets improve the diagnostic and prognostic power of the various analysis algorithms in the feature analysis module 160.

The skilled artisan will know of other types of features useful for providing clinical support for personalized cancer therapy. The listing of features above is merely representative and should not be construed to be limiting.

In some embodiments, a test patient data store 120 includes clinical assessment data 139 for patients, *e.g.,* based on the feature data 125 collected for the subject. In some embodiments, the clinical assessment data 139 includes a catalogue of actionable variants and characteristics 139-1 (*e.g*., genomic alterations such as CNV, focal CNV, SNV, MNV, as well as compound metrics thereof, known or believed to be targetable by one or more specific therapies), matched therapies 139-2 (*e.g.,* the therapies known or believed to be particularly beneficial for treatment of subjects having actionable variants), and/or clinical reports 139-3 generated for the subject, *e.g.,* based on identified actionable variants and characteristics 139-1, and/or matched therapies 139-2, and/or matched clinical trials.

In some embodiments, clinical assessment data 139 is generated by analysis of feature data 125 using the various algorithms of feature analysis module 160, as described in further detail below. In some embodiments, clinical assessment data 139 is generated, modified, and/or validated by evaluation of feature data 125 by a clinician, *e.g.,* an oncologist. For instance, in some embodiments, a clinician (*e.g.,* at clinical environment 220) uses feature analysis module 160, or accesses test patient data store 120 directly, to evaluate feature data 125 to make recommendations for personalized treatment of a patient. Similarly, in some embodiments, a clinician (*e.g.,* at clinical environment 220) reviews recommendations determined using feature analysis module 160 and approves, rejects, or modifies the recommendations, *e.g.,* prior to the recommendations being sent to a medical professional treating the patient.

### Bioinformatics Module (140)

Referring again to Figure 1A, the system (*e.g.,* system 100) includes a bioinformatics module 140 that includes a feature extraction module 145 and optional ancillary data processing constructs, such as a sequence data processing module 141 and/or one or more reference sequence constructs 158 (*e.g.,* a reference genome, exome, or targeted-panel construct that includes reference sequences for a plurality of loci targeted by a sequencing panel).

In some embodiments, bioinformatics module 140 includes a sequence data processing module 141 that includes instructions for processing sequence reads, *e.g.,* raw sequence reads 123 from one or more sequencing reactions 122-i, prior to analysis by the various feature extraction algorithms, as described in detail below. In some embodiments, sequence data processing module 141 includes one or more pre-processing algorithms 142 that prepare the data for analysis. In some embodiments, the pre-processing algorithms 142 include instructions for converting the file format of the sequence reads from the output of the sequencer (*e.g.,* a BCL file format) into a file format compatible with downstream analysis of the sequences (*e.g.,* a FASTQ or FASTA file format). In some embodiments, the pre-processing algorithms 142 include instructions for evaluating the quality of the sequence reads (*e.g.,* by interrogating quality metrics like Phred score, base-calling error probabilities, Quality (Q) scores, and the like) and/or removing sequence reads that do not satisfy a threshold quality (*e.g.,* an inferred base call accuracy of at least 80%, at least 90%, at least 95%, at least 99%, at least 99.5%, at least 99.9%, or higher). In some embodiments, the pre-processing algorithms 142 include instructions for filtering the sequence reads for one or more properties, *e.g.,* removing sequences failing to satisfy a lower or upper size threshold or removing duplicate sequence reads.

In some embodiments, sequence data processing module 141 includes one or more alignment algorithms 143, for aligning pre-processed sequence reads 123 to a reference sequence construct 158, *e.g.,* a reference genome, exome, or targeted-panel construct. Many algorithms for aligning sequencing data to a reference construct are known in the art, for example, BWA, Blat, SHRiMP, LastZ, and MAQ. One example of a sequence read alignment package is the Burrows-Wheeler Alignment tool (BWA), which uses a Burrows-Wheeler Transform (BWT) to align short sequence reads against a large reference construct, allowing for mismatches and gaps. Li and Durbin, Bioinformatics, 25(14):1754-60 (2009). Sequence read alignment packages import raw or pre-processed sequence reads 122, *e.g.,* in BCL, FASTA, or FASTQ file formats, and output aligned sequence reads 124, *e.g.,* in SAM or BAM file formats. Generally, any known alignment methodology, including pseudoalignment methodologies, find use in the methods and systems described herein.

In some embodiments, sequence data processing module 141 includes one or more demultiplexing algorithms 144, for dividing sequence read or sequence alignment files generated from sequencing reactions of pooled nucleic acids into separate sequence read or sequence alignment files, each of which corresponds to a different source of nucleic acids in the nucleic acid sequencing pool. For instance, because of the cost of sequencing reactions, it is common practice to pool nucleic acids from a plurality of samples into a single sequencing reaction. The nucleic acids from each sample are tagged with a sample-specific and/or molecule-specific sequence tag (*e.g.,* a UMI), which is sequenced along with the molecule. In some embodiments, demultiplexing algorithms 144 sort these sequence tags in the sequence read or sequence alignment files to demultiplex the sequencing data into separate files for each of the samples included in the sequencing reaction.

Bioinformatics module 140 includes a feature extraction module 145, which includes instructions for identifying diagnostic features, *e.g.,* genomic features 131, from sequencing data 122 of biological samples from a subject. For instance, in some embodiments, a feature extraction algorithm compares the identity of one or more nucleotides at a locus from the sequencing data 122 to the identity of the nucleotides at that locus in a reference sequence construct (*e.g.,* a reference genome, exome, or targeted-panel construct) to determine whether the subject has a variant at that locus. In some embodiments, a feature extraction algorithm evaluates data other than the raw sequence, e.g., copy number, to identify a genomic alteration in the subject, *e.g.,* a copy number variation (CNV).

For instance, in some embodiments, feature extraction module 145 includes one or more variant identification modules 146 that include instructions for various variant calling processes. In some embodiments, the variant identification module includes instructions for identifying one or more of nucleotide variants (*e.g.,* single nucleotide variants (SNV) and multi-nucleotide variants (MNV)) using one or more SNV/MNV calling algorithms (*e.g.,* algorithm 147), indels (*e.g.,* insertions or deletions of nucleotides) using one or more indel calling algorithms (*e.g.,* algorithm 148), and genomic rearrangements (*e.g.,* inversions, translocation, and fusions of nucleotide sequences) using one or more genomic rearrangement calling algorithms (*e.g.,* algorithm 149).

In some embodiments where the disease or disorder is a cancer, variants are identified in both the germline of the subject (e.g., germline variants) and in a cancer genome (*e.g.,* somatic variants) of the subject, *e.g.,* using the variant identification module 146. In some embodiments, separate germline and somatic variant identification modules are used, while in some embodiments they are integrated into a single module.

A SNV/MNV algorithm 147 may identify a substitution of a single nucleotide that occurs at a specific position in the genome. For example, at a specific base position, or locus, in the human genome, the C nucleotide may appear in most individuals, but in a minority of individuals, the position is occupied by an A. This means that there is a SNP at this specific position and the two possible nucleotide variations, C or A, are said to be alleles for this position. SNPs underlie differences in human susceptibility to a wide range of diseases (*e.g.,* sickle-cell anemia, β-thalassemia and cystic fibrosis result from SNPs). The severity of illness and the way the body responds to treatments are also manifestations of genetic variations. For example, a single-base mutation in the APOE (apolipoprotein E) gene is associated with a lower risk for Alzheimer's disease. A single-nucleotide variant (SNV) is a variation in a single nucleotide without any limitations of frequency and may arise in somatic cells. A somatic single-nucleotide variation (*e.g.,* caused by cancer) may also be called a single-nucleotide alteration. An MNP (Multiple-nucleotide polymorphisms) module may identify the substitution of consecutive nucleotides at a specific position in the genome.

An indel calling algorithm 148 may identify an insertion or deletion of bases in the genome of an organism classified among small genetic variations. While indels usually measure from 1 to 10 000 base pairs in length, a microindel is defined as an indel that results in a net change of 1 to 50 nucleotides. Indels can be contrasted with a SNP or point mutation. An indel inserts and/or deletes nucleotides from a sequence, while a point mutation is a form of substitution that replaces one of the nucleotides without changing the overall number in the DNA. Indels, being insertions and/or deletions, can be used as genetic markers in natural populations, especially in phylogenetic studies. Indel frequency tends to be markedly lower than that of single nucleotide polymorphisms (SNP), except near highly repetitive regions, including homopolymers and microsatellites.

A genomic rearrangement algorithm 149 may identify hybrid genes formed from two previously separate genes. It can occur as a result of translocation, interstitial deletion, or chromosomal inversion. Gene fusion can play an important role in tumorigenesis. Fusion genes can contribute to tumor formation because fusion genes can produce much more active abnormal protein than non-fusion genes. Often, fusion genes are oncogenes that cause cancer; these include BCR-ABL, TEL-AML1 (ALL with t(12 ; 21)), AML1-ETO (M2 AML with t(8 ; 21)), and TMPRSS2-ERG with an interstitial deletion on chromosome 21, often occurring in prostate cancer. In the case of TMPRSS2-ERG, by disrupting androgen receptor (AR) signaling and inhibiting AR expression by oncogenic ETS transcription factor, the fusion product regulates prostate cancer. Most fusion genes are found from hematological cancers, sarcomas, and prostate cancer. BCAM-AKT2 is a fusion gene that is specific and unique to high-grade serous ovarian cancer. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. The latter is common in lymphomas, where oncogenes are juxtaposed to the promoters of the immunoglobulin genes. Oncogenic fusion transcripts may also be caused by trans-splicing or read-through events. Since chromosomal translocations play such a significant role in neoplasia, a specialized database of chromosomal aberrations and gene fusions in cancer has been created. This database is called Mitelman Database of Chromosome Aberrations and Gene Fusions in Cancer.

In some embodiments where the disease or disorder is a cancer, feature extraction module 145 includes cancer-specific modules 150 (e.g., as illustrated in Figure 1E) for identifying one or more complex genomic alterations (*e.g.,* features that incorporate more than a change in the primary sequence of the genome) in a genome of the subject. For instance, in some embodiments, feature extraction module 145 includes modules for identifying one or more of variant allele fraction (*e.g.,* variant allele fraction module 151), methylation status (*e.g.,* methylation analysis module 152), microsatellite instability status (*e.g.,* microsatellite instability analysis module 154), tumor mutational burden (*e.g.,* tumor mutational burden analysis module 155), tumor ploidy (*e.g.,* tumor ploidy analysis module 156), and homologous recombination pathway deficiencies (*e.g.,* homologous recombination pathway analysis module 157).

In some embodiments, referring to Figures 1D, the copy number variation analysis module 153 determines a copy number variant status of a subject,. In some embodiments, the module obtains sequencing data for a first plurality of DNA molecules from a first biological sample of the subject generated by whole genome sequencing (*e.g.,* low-pass whole genome sequencing (LPWGS)) and sequencing data for a second plurality of DNA molecules from a second biological sample of the subject generated by targeted-panel sequencing, e.g., from a sequencing data store such as sequence reads 123 or aligned sequences 124 stored in test patient data store 120 as illustrated in Figure 1B.

In some embodiments, the copy number variation analysis module 153 generates a first mapped dataset using the LPWGS sequencing data and a second mapped dataset using the targeted-panel sequencing data. In some embodiments, the first and second datasets are a single data set. That is, in some embodiments, the copy number variation analysis module 153 generates a single mapped dataset using both the LPWGS sequencing data and the targeted-panel sequencing data. In some embodiments, the mapped dataset(s) include a plurality of aligned sequences 124 generated from the LPWGS sequencing data and/or from the targeted-panel sequencing data.

In some embodiments, the copy number variation analysis module 153 bins sequences from the LPWGS sequencing data according to the positions to which each sequence maps to a reference genome in the species of the subject. For example, in some embodiments, the copy number variation analysis module 153 bins aligned sequences 124 from LPWGS sequencing data to generate a first plurality of bin values 135-wgs-bv, e.g., using bin value determination module 153-b, as illustrated in Figure 1. In some embodiments, each respective bin in the first plurality of bins represents a unique segment of the reference genome, and each respective first bin value is a measure of the number of nucleic acid sequences in the first plurality of nucleic acid sequences that were mapped to the unique segment of the reference genome corresponding to the respective bin in the first plurality of bins.

Similarly, in some embodiments, the copy number variation analysis module 153 bins sequences from the targeted-panel sequencing data according to the positions to which each sequence maps to a reference construct for the species of the subject, e.g., a reference genome or a construct specific to the enrichment panel used for sequencing. For example, in some embodiments, the copy number variation analysis module 153 bins aligned sequences 124 from targeted-panel sequencing data to generate a second plurality of bin values 135-ps-bv, e.g., using bin value determination module 153-b, as illustrated in Figure 1. In some embodiments, each respective bin in the second plurality of bins represents a unique segment of the reference construct, and each respective second bin value is a measure of the number of nucleic acid sequences in the first plurality of nucleic acid sequences that were mapped to the unique segment of the reference genome corresponding to the respective bin in the first plurality of bins.

Accordingly, in some embodiments, the mapped dataset(s) (e.g., WGS mapped dataset 153-f-1 and Targeted-panel mapped dataset 153-f-2, or a combined mapped dataset thereof) include a plurality of bin values 135-bv generated from the LPWGS sequencing data and/or from the targeted-panel sequencing data.

In some embodiments, the copy number variation analysis module 153 determines a copy number state for the genomic location corresponding to each bin from the LPWGS sequencing data. For example, in some embodiments, the copy number variation analysis module 153 analyzes bin values 135-wgs-bv for the LPWGS sequencing data to generate bin copy number states 135-wgs-cns, e.g., using copy number state determination module 153-d, as illustrated in Figure 1. Similarly, in some embodiments, the copy number variation analysis module 153 determines a copy number state for the genomic location corresponding to each bin from the targeted-panel sequencing data. For example, in some embodiments, the copy number variation analysis module 153 analyzes bin values 135-ps-bv for the targeted-panel sequencing data to generate bin copy number states 135-ps-cns, e.g., using copy number state determination module 153-d, as illustrated in Figure 1. Various methods for determining copy number state are described herein. Generally, any method of copy number state determination can be used in conjunction with the method and systems described herein. Accordingly, in some embodiments, the mapped dataset(s) (e.g., WGS mapped dataset 153-f-1 and Targeted-panel mapped dataset 153-f-2, or a combined mapped dataset thereof) include a plurality of bin copy number states 135-cns generated from the LPWGS sequencing data and/or from the targeted-panel sequencing data.

In some embodiments, the mapped dataset(s) (e.g., WGS mapped dataset 153-f-1 and Targeted-panel mapped dataset 153-f-2, or a combined mapped dataset thereof) include a plurality of aligned sequences 124-wgs, a plurality of bin values 135-wgs-bv, and/or a plurality of bin copy number states 135-wgs-cns for the LPWGS sequencing data and a plurality of aligned sequences 124-ps, a plurality of bin values 135-bv-ps, and/or a plurality of bin copy number states 135-cns-ps for the targeted-panel sequencing data.

In yet other embodiments, the mapped dataset(s) includes a plurality of dimensionality reduced component values prepared from a plurality of aligned sequences 124-wgs, a plurality of bin values 135-wgs-bv, and/or a plurality of bin copy number states 135-wgs-cns for the LPWGS sequencing data and/or a plurality of aligned sequences 124-ps, a plurality of bin values 135-bv-ps, and/or a plurality of bin copy number states 135-cns-ps for the targeted-panel sequencing data.

In some embodiments, copy number variation analysis module 153 applies a model 153-h, such as a model implemented by classification construct 153-g in CNV analysis module 153, to (i) all or a portion of the first mapped dataset and (ii) all or a portion of the second mapped dataset, or a plurality of dimensionality reduction components thereof. The all or a portion of the first mapped dataset and the all or a portion of the second mapped dataset can be stored, for example, in input data store 153-f, comprising one or more of a bin value data structure 153-f-1, a copy number state data structure 153-f-2, and a dimensionality reduction module 153-f-3. The classification construct 153-g thereby identifies one or more copy number variations, as output of the model, thus indicating the copy number variation status of the subject.

Further details and specific embodiments regarding methods for determining a copy number variation status of a subject are provided below with reference to Figures 4F and 5A-D.

### Feature Analysis Module (160)

Referring again to Figure 1A, the system (*e.g.,* system 100) includes a feature analysis module 160 that includes one or more genomic alteration interpretation algorithms 161, one or more optional clinical data analysis algorithms 165, an optional therapeutic curation algorithm 165, and an optional recommendation validation module 167. In some embodiments, feature analysis module 160 identifies actionable variants and characteristics 139-1 and corresponding matched therapies 139-2 and/or clinical trials using one or more analysis algorithms (*e.g.,* algorithms 162, 163, 164, and 165) to evaluate feature data 125. The identified actionable variants and characteristics 139-1 and corresponding matched therapies 139-2, which are optionally stored in test patient data store 120, are then curated by feature analysis module 160 to generate a clinical report 139-3, which is optionally validated by a user, *e.g.,* a clinician, before being transmitted to a medical professional, *e.g.,* an oncologist, treating the patient.

In some embodiments, the genomic alteration interpretation algorithms 161 include instructions for evaluating the effect that one or more genomic features 131 of the subject, *e.g.,* as identified by feature extraction module 145, have on the characteristics of the patient's cancer and/or whether one or more targeted cancer therapies may improve the clinical outcome for the patient. For example, in some embodiments, one or more genomic variant analysis algorithms 163 evaluate various genomic features 131 by querying a database, *e.g.,* a look-up-table ("LUT") of actionable genomic alterations, targeted therapies associated with the actionable genomic alterations, and any other conditions that should be met before administering the targeted therapy to a subject having the actionable genomic alteration. For instance, evidence suggests that depatuxizumab mafodotin (an anti-EGFR mAb conjugated to monomethyl auristatin F) has improved efficacy for the treatment of recurrent glioblastomas having EGFR focal amplifications. van den Bent M. et al., Cancer Chemother Pharmacol., 80(6):1209-17 (2017). Accordingly, the actionable genomic alteration LUT would have an entry for the focal amplification of the EGFR gene indicating that depatuxizumab mafodotin is a targeted therapy for glioblastomas (*e.g.,* recurrent glioblastomas) having a focal gene amplification. In some instances, the LUT may also include counter indications for the associated targeted therapy, *e.g.,* adverse drug interactions or personal characteristics that are counter-indicated for administration of the particular targeted therapy.

In some embodiments, a genomic alteration interpretation algorithm 161 determines whether a particular genomic feature 131 should be reported to a medical professional treating the cancer patient. In some embodiments, genomic features 131 (*e.g*., genomic alterations and compound features) are reported when there is clinical evidence that the feature significantly impacts the biology of the disease or disorder, impacts the prognosis for the disease or disorder, and/or impacts pharmacogenomics, *e.g.,* by indicating or counter-indicating particular therapeutic approaches. For instance, a genomic alteration interpretation algorithm 161 may classify a particular CNV feature 135 as "Reportable," *e.g.,* meaning that the CNV has been identified as influencing the character of the disease or disorder, the overall disease state, and/or pharmacogenomics, as "Not Reportable," *e.g.,* meaning that the CNV has not been identified as influencing the character of the disease or disorder, the overall disease state, and/or pharmacogenomics, as "No Evidence," *e.g.,* meaning that no evidence exists supporting that the CNV is "Reportable" or "Not Reportable," or as "Conflicting Evidence," *e.g.,* meaning that evidence exists supporting both that the CNV is "Reportable" and that the CNV is "Not Reportable."

In some embodiments, the genomic alteration interpretation algorithms 161 include one or more pathogenic variant analysis algorithms 162, which evaluate various genomic features to identify the presence of a pathogen associated with the patient's disease or disorder and/or targeted therapies associated with a pathogenic infection in the disease or disorder. For instance, RNA expression patterns of some cancers are associated with the presence of an oncogenic pathogen that is helping to drive the cancer. See, for example, U.S. Patent Application Serial No. 16/802,126, filed February 26, 2020. In some instances, the recommended therapy for the disease or disorder is different when the disease or disorder is associated with the pathogenic infection than when it is not. Accordingly, in some embodiments, *e.g.,* where feature data 125 includes RNA abundance data, one or more pathogenic variant analysis algorithms 162 evaluate the RNA abundance data to determine whether a signature exists in the data that indicates the presence of the pathogen in the disease or disorder. Similarly, in some embodiments, bioinformatics module 140 includes an algorithm that searches for the presence of pathogenic nucleic acid sequences in sequencing data 122. See, for example, U.S. Provisional Patent Application Serial No. 62/978,067, filed February 18, 2020. Accordingly, in some embodiments, one or more pathogenic variant analysis algorithms 162 evaluates whether the presence of a pathogen in a subject is associated with an actionable therapy. In some embodiments, system 100 queries a database, *e.g.,* a look-up-table ("LUT"), of actionable pathogenic infections, targeted therapies associated with the actionable infections, and any other conditions that should be met before administering the targeted therapy to a subject that is infected with the pathogen. In some instances, the LUT may also include counter indications for the associated targeted therapy, *e.g.,* adverse drug interactions or personal characteristics that are counter-indicated for administration of the particular targeted therapy.

In some embodiments, the genomic alteration interpretation algorithms 161 include one or more multi-feature analysis algorithms 164 that evaluate a plurality of features to classify a disease or disorder with respect to the effects of one or more targeted therapies. For instance, in some embodiments, feature analysis module 160 includes one or more classifiers trained against feature data, one or more clinical therapies, and their associated clinical outcomes for a plurality of training subjects to classify a disease or disorder based on their predicted clinical outcomes following one or more therapies.

In some embodiments, the classifier is implemented as an artificial intelligence engine and may include gradient boosting models, random forest models, neural networks (NN), regression models, Naive Bayes models, and/or machine learning algorithms (MLA). An MLA or a NN may be trained from a training data set that includes one or more features 125, including personal characteristics 126, medical history 127, clinical features 128, genomic features 131, and/or other -omic features 138. MLAs include supervised algorithms (such as algorithms where the features/classifications in the data set are annotated) using linear regression, logistic regression, decision trees, classification and regression trees, naïve Bayes, nearest neighbor clustering; unsupervised algorithms (such as algorithms where no features/classification in the data set are annotated) using Apriori, means clustering, principal component analysis, random forest, adaptive boosting; and semi-supervised algorithms (such as algorithms where an incomplete number of features/classifications in the data set are annotated) using generative approach (such as a mixture of Gaussian distributions, mixture of multinomial distributions, hidden Markov models), low density separation, graph-based approaches (such as mincut, harmonic function, manifold regularization), heuristic approaches, or support vector machines.

NNs include conditional random fields, convolutional neural networks, attention based neural networks, deep learning, long short term memory networks, or other neural models where the training data set includes a plurality of tumor samples, RNA expression data for each sample, and pathology reports covering imaging data for each sample.

While MLA and neural networks identify distinct approaches to machine learning, the terms may be used interchangeably herein. Thus, a mention of MLA may include a corresponding NN or a mention of NN may include a corresponding MLA unless explicitly stated otherwise. Training may include providing optimized datasets, labeling these traits as they occur in patient records, and training the MLA to predict or classify based on new inputs. Artificial NNs are efficient computing models which have shown their strengths in solving hard problems in artificial intelligence. They have also been shown to be universal approximators, that is, they can represent a wide variety of functions when given appropriate parameters.

In some embodiments, system 100 includes a classifier training module that includes instructions for training one or more untrained or partially trained classifiers based on feature data from a training dataset. In some embodiments, system 100 also includes a database of training data for use in training the one or more classifiers. In other embodiments, the classifier training module accesses a remote storage device hosting training data. In some embodiments, the training data includes a set of training features, including but not limited to, various types of the feature data 125 illustrated in Figure 1B. In some embodiments, the classifier training module uses patient data 121, e.g., when test patient data store 120 also stores a record of treatments administered to the patient and patient outcomes following therapy.

In some embodiments, feature analysis module 160 includes one or more clinical data analysis algorithms 165, which evaluate clinical features 128 of a disease or disorder to identify targeted therapies which may benefit the subject. For example, in some embodiments, *e.g.,* where feature data 125 includes pathology data 128-1, one or more clinical data analysis algorithms 165 evaluate the data to determine whether an actionable therapy is indicated based on the histopathology of a tumor biopsy from the subject, *e.g.,* which is indicative of a particular cancer type and/or stage of cancer. In some embodiments, system 100 queries a database, *e.g.,* a look-up-table ("LUT"), of actionable clinical features (*e.g.,* pathology features), targeted therapies associated with the actionable features, and any other conditions that should be met before administering the targeted therapy to a subject associated with the actionable clinical features 128 (*e.g.,* pathology features 128-1). In some embodiments, system 100 evaluates the clinical features 128 (*e.g.,* pathology features 128-1) directly to determine whether the patient's disease or disorder is sensitive to a particular therapeutic agent. Further details on example methods, systems, and algorithms for classifying cancer and identifying targeted therapies based on clinical data, such as pathology data 128-1, imaging data 138-2, and/or tissue culture/organoid data 128-3 are discussed, for example, in U.S. Patent Application No. 16/830,186, filed on March 25, 2020, U.S. Patent Application No. 16/789,363, filed on Feb. 12, 2020, and U.S. Patent Application No. 17/227,120, filed on April 9, 2021.

In some embodiments, feature analysis module 160 includes a clinical trials module that evaluates test patient data 121 to determine whether the patient is eligible for inclusion in a clinical trial for treatment of a disease or disorder, *e.g.,* a clinical trial that is currently recruiting patients, a clinical trial that has not yet begun recruiting patients, and/or an ongoing clinical trial that may recruit additional patients in the future. In some embodiments, a clinical trial module evaluates test patient data 121 to determine whether the results of a clinical trial are relevant for the patient, *e.g.,* the results of an ongoing clinical trial and/or the results of a completed clinical trial. For instance, in some embodiments, system 100 queries a database, *e.g.,* a look-up-table ("LUT") of clinical trials, *e.g.,* active and/or completed clinical trials, and compares patient data 121 with inclusion criteria for the clinical trials, stored in the database, to identify clinical trials with inclusion criteria that closely match and/or exactly match the patient's data 121. In some embodiments, a record of matching clinical trials, *e.g.,* those clinical trials that the patient may be eligible for and/or that may inform personalized treatment decisions for the patient, are stored in clinical assessment database 139.

In some embodiments, feature analysis module 160 includes a therapeutic curation algorithm 166 that assembles actionable variants and characteristics 139-1, matched therapies 139-2, and/or relevant clinical trials identified for the patient, as described above. In some embodiments, a therapeutic curation algorithm 166 evaluates certain criteria related to which actionable variants and characteristics 139-1, matched therapies 139-2, and/or relevant clinical trials should be reported and/or whether certain matched therapies, considered alone or in combination, may be counter-indicated for the patient, *e.g.,* based on personal characteristics 126 of the patient and/or known drug-drug interactions. In some embodiments, the therapeutic curation algorithm then generates one or more clinical reports 139-3 for the patient. In some embodiments, the therapeutic curation algorithm generates a first clinical report 139-3-1 that is to be reported to a medical professional treating the patient and a second clinical report 139-3-2 that will not be communicated to the medical professional but may be used to improve various algorithms within the system.

In some embodiments, feature analysis module 160 includes a recommendation validation module 167 that includes an interface allowing a clinician to review, modify, and approve a clinical report 139-3 prior to the report being sent to a medical professional, *e.g.,* an oncologist, treating the patient.

In some embodiments, each of the one or more feature collections, sequencing modules, bioinformatics modules (including, *e.g.,* alteration module(s), structural variant calling and data processing modules), classification modules and outcome modules are communicatively coupled to a data bus to transfer data between each module for processing and/or storage. In some alternative embodiments, each of the feature collection, alteration module(s), structural variant and feature store are communicatively coupled to each other for independent communication without sharing the data bus.

Further details on systems and exemplary embodiments of modules and feature collections are discussed in PCT Application PCT/US19/69149, titled "A METHOD AND PROCESS FOR PREDICTING AND ANALYZING PATIENT COHORT RESPONSE, PROGRESSION, AND SURVIVAL," filed December 31, 2019.

### Example Embodiments

Now that details of a system 100 for providing clinical support for personalized cancer therapy have been disclosed, *e.g.,* with improved determination of copy number variation status, details regarding processes and features of the system are provided below. Specifically, example processes are described below with reference to Figures 2A-B, 3, 4A-B, and 5A-D. In some embodiments, such processes and features of the system are carried out by modules 118, 120, 140, 160, and/or 180, as illustrated in Figure 1A. Referring to these methods, the systems described herein (*e.g.,* system 100) include instructions for determining copy number variation status that are improved compared to conventional methods for copy number analysis.

### Figure 2B: Distributed Diagnostic and Clinical Environment

In some aspects, the methods described herein for providing clinical support for a disease or disorder are performed across a distributed diagnostic/clinical environment, *e.g.,* as illustrated in Figure 2B. However, in some embodiments, the improved methods described herein for supporting clinical decisions in personalized (*e.g.,* by determining a copy number variation status of a subject, *etc*.) are performed at a single location, *e.g.,* at a single computing system or environment, although ancillary procedures supporting the methods described herein, and/or procedures that make further use of the results of the methods described herein, may be performed across a distributed diagnostic/clinical environment.

Figure 2B illustrates an example of a distributed diagnostic/clinical environment 210. In some embodiments, the distributed diagnostic/clinical environment is connected via communication network 105. In some embodiments, one or more biological samples are collected from a subject in clinical environment 220, *e.g.,* a doctor's office, hospital, or medical clinic, or at a home health care environment (not depicted). In some embodiments, one or more biological samples, or portions thereof, are processed within the clinical environment 220 where collection occurred, using a processing device 224, *e*.g*.,* a nucleic acid sequencer for obtaining sequencing data, a microscope for obtaining pathology data, a mass spectrometer for obtaining proteomic data, *etc.* In some embodiments, one or more biological samples, or portions thereof, are sent to one or more external environments, *e.g.,* sequencing lab 230, pathology lab 240, and/or molecular biology lab 250, each of which includes a processing device 234, 244, and 254, respectively, to generate biological data 121 for the subject. Each environment includes a communications device 222, 232, 242, and 252, respectively, for communicating biological data 121 about the subject to a processing server 262 and/or database 264, which may be located in yet another environment, *e.g.,* processing/storage center 260. Thus, in some embodiments, different portions of the systems and methods described herein are fulfilled by different processing devices located in different physical environments.

Accordingly, in some embodiments, a method for providing clinical support for personalized therapy, *e.g.,* with improved determination of copy number variation status, is performed across one or more environments, as illustrated in Figure 2B. For instance, in some such embodiments, a sample is collected at clinical environment 220 or in a home healthcare environment. The sample, or a portion thereof, is sent to sequencing lab 230 where raw sequence reads 123 of nucleic acids in the sample are generated by sequencer 234. The raw sequencing data 123 is communicated, *e.g.,* from communications device 232, to database 264 at processing/storage center 260, where processing server 262 extracts features from the sequence reads by executing one or more of the processes in bioinformatics module 140, thereby generating genomic features 131 for the sample. Processing server 262 may then analyze the identified features by executing one or more of the processes in feature analysis module 160, thereby generating clinical assessment 139, including a clinical report 139-3. A clinician may access clinical report 139-3, *e.g.,* at processing/storage center 260 or through communications network 105, via recommendation validation module 167. After final approval, clinical report 139-3 is transmitted to a medical professional, *e.g.,* an oncologist, at clinical environment 220, who uses the report to support clinical decision making for personalized treatment of the patient's cancer.

### Figure 2A: Example Workflow

Figure 2A is a flowchart of an example workflow 200 for collecting and analyzing data in order to generate a clinical report 139 to support clinical decision making in personalized medicine. Advantageously, the methods described herein improve this process, for example, by improving various stages within feature extraction 206, including determining copy number variation status. Workflow 200 is tailored for a precision oncology application, but the skilled artisan will know how to tailor such workflows to provide clinical support for other diseases and disorders.

Briefly, the workflow begins with patient intake and sample collection 201, where one or more liquid biopsy samples, one or more tumor biopsy, and one or more normal and/or control tissue samples are collected from the patient (e.g., at a clinical environment 220 or home healthcare environment, as illustrated in Figure 2B). In some embodiments, personal data 126 corresponding to the patient and a record of the one or more biological samples obtained (*e.g.,* patient identifiers, patient clinical data, sample type, sample identifiers, cancer conditions, *etc*.) are entered into a data analysis platform, *e.g.,* test patient data store 120. Accordingly, in some embodiments, the methods disclosed herein include obtaining one or more biological samples from one or more subjects, *e.g.,* cancer patients. In some embodiments, the subject is a human, *e.g.,* a human cancer patient.

In some embodiments, one or more of the biological samples obtained from the patient is a biological liquid sample, also referred to as a liquid biopsy sample. In some embodiments, one or more of the biological samples obtained from the patient are selected from blood, plasma, serum, urine, vaginal fluid, fluid from a hydrocele *(e.g.,* of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (*e.g.,* thyroid, breast), etc. In some embodiments, the liquid biopsy sample includes blood and/or saliva. In some embodiments, the liquid biopsy sample is peripheral blood. In some embodiments, blood samples are collected from patients in commercial blood collection containers, e.g., using a PAXgene^{®} Blood DNA Tubes. In some embodiments, saliva samples are collected from patients in commercial saliva collection containers, e.g., using an Oragene^{®} DNA Saliva Kit.

In some embodiments, one or more biological samples collected from the patient is a solid tissue sample, e.g., a solid tumor sample or a solid normal tissue sample. Methods for obtaining solid tissue samples, e.g., of cancerous and/or normal tissue are known in the art and are dependent upon the type of tissue being sampled. For example, bone marrow biopsies and isolation of circulating tumor cells can be used to obtain samples of blood cancers, endoscopic biopsies can be used to obtain samples of cancers of the digestive tract, bladder, and lungs, needle biopsies (*e.g*., fine-needle aspiration, core needle aspiration, vacuum-assisted biopsy, and image-guided biopsy, can be used to obtain samples of subdermal tumors, skin biopsies, *e.g.,* shave biopsy, punch biopsy, incisional biopsy, and excisional biopsy, can be used to obtain samples of dermal cancers, and surgical biopsies can be used to obtain samples of cancers affecting internal organs of a patient. In some embodiments, a solid tissue sample is a formalin-fixed tissue (FFT). In some embodiments, a solid tissue sample is a macro-dissected formalin fixed paraffin embedded (FFPE) tissue. In some embodiments, a solid tissue sample is a fresh frozen tissue sample.

In some embodiments, a dedicated normal sample is collected from the patient, for co-processing with a liquid biopsy sample. Generally, the normal sample is of a non-cancerous tissue, and can be collected using any tissue collection means described above. In some embodiments, buccal cells collected from the inside of a patient's cheeks are used as a normal sample. Buccal cells can be collected by placing an absorbent material, *e.g.,* a swab, in the subject's mouth and rubbing it against their cheek, *e.g.,* for at least 15 second or for at least 30 seconds. The swab is then removed from the patient's mouth and inserted into a tube, such that the tip of the tube is submerged into a liquid that serves to extract the buccal cells off of the absorbent material. An example of buccal cell recovery and collection devices is provided in U.S. Patent No. 9,138,205. In some embodiments, the buccal swab DNA is used as a source of normal DNA in circulating heme malignancies.

The biological samples collected from the patient are, optionally, sent to various analytical environments (*e.g.,* sequencing lab 230, pathology lab 240, and/or molecular biology lab 250) for processing (*e.g.,* data collection) and/or analysis (*e.g.,* feature extraction). Wet lab processing 204 may include cataloguing samples (*e.g.,* accessioning), examining clinical features of one or more samples (*e.g.,* pathology review), and nucleic acid sequence analysis (*e.g.,* extraction, library prep, capture + hybridize, pooling, and sequencing). In some embodiments, the workflow includes clinical analysis of one or more biological samples collected from the subject, *e.g.,* at a pathology lab 240 and/or a molecular and cellular biology lab 250, to generate clinical features such as pathology features 128-3, imaging data 128-3, and/or tissue culture / organoid data 128-3.

In some embodiments, the pathology data 128-1 collected during clinical evaluation includes visual features identified by a pathologist's inspection of a specimen (*e.g.,* a solid tumor biopsy), *e.g.,* of stained H&E or IHC slides. In some embodiments, the sample is a solid tissue biopsy sample. In some embodiments, the tissue biopsy sample is a formalin-fixed tissue (FFT), *e.g.,* a formalin-fixed paraffin-embedded (FFPE) tissue. In some embodiments, the tissue biopsy sample is an FFPE or FFT block. In some embodiments, the tissue biopsy sample is a fresh-frozen tissue biopsy. The tissue biopsy sample can be prepared in thin sections (*e.g.,* by cutting and/or affixing to a slide), to facilitate pathology review (*e.g.,* by staining with immunohistochemistry stain for IHC review and/or with hematoxylin and eosin stain for H&E pathology review). For instance, analysis of slides for H&E staining or IHC staining may reveal features such as tumor infiltration, programmed death-ligand 1 (PD-L1) status, human leukocyte antigen (HLA) status, or other immunological features.

In some embodiments, a liquid sample (*e.g.,* blood) collected from the patient (*e.g.,* in EDTA-containing collection tubes) is prepared on a slide (*e.g.,* by smearing) for pathology review. In some embodiments, macrodissected FFPE tissue sections, which may be mounted on a histopathology slide, from solid tissue samples (*e.g.,* tumor or normal tissue) are analyzed by pathologists. In some embodiments, tumor samples are evaluated to determine, *e.g.,* the tumor purity of the sample, the percent tumor cellularity as a ratio of tumor to normal nuclei, etc. For each section, background tissue may be excluded or removed such that the section meets a tumor purity threshold, *e.g.,* where at least 20% of the nuclei in the section are tumor nuclei, or where at least 25%, 30%, 40%, 50%, 60%, 70%, 80%, 90%, or more of the nuclei in the section are tumor nuclei.

Further details on methods, systems, and algorithms for using pathology data to classify cancer and identify targeted therapies are discussed, for example, in are discussed, for example, in U.S. Patent Application No. 16/830,186, filed on March 25, 2020, and U.S. Patent Application No. 17/227,120, filed on April 9, 2021.

In some embodiments, imaging data 128-2 collected during clinical evaluation includes features identified by review of *in vitro* and/or *in vivo* imaging results (*e.g.,* of a tumor site), for example a size of a tumor, tumor size differentials over time (such as during treatment or during other periods of change). In some embodiments, imaging data 128-2 includes features determined using machine learning algorithms to evaluate imaging data collected as described above.

Further details on methods, systems, and algorithms for using medical imaging to classify cancer and identify targeted therapies are discussed, for example, in are discussed, for example, in U.S. Patent Application No. 16/830,186, filed on March 25, 2020, and U.S. Patent Application No. 17/227,120, filed on April 9, 2021.

In some embodiments, tissue culture / organoid data 128-3 collected during clinical evaluation includes features identified by evaluation of cultured tissue from the subject. For instance, in some embodiments, tissue samples obtained from the patients (*e.g.,* tumor tissue, normal tissue, or both) are cultured (*e.g.,* in liquid culture, solid-phase culture, and/or organoid culture) and various features, such as cell morphology, growth characteristics, genomic alterations, and/or drug sensitivity, are evaluated. In some embodiments, tissue culture / organoid data 128-3 includes features determined using machine learning algorithms to evaluate tissue culture / organoid data collected as described above. Examples of tissue organoid (*e.g.,* personal tumor organoid) culturing and feature extractions thereof are described in PCT/US20/56930, filed on October 22, 2020, and U.S. Patent Application Serial No. 16/693,117, filed on November 22, 2019.

Nucleic acid sequencing of one or more samples collected from the subject is performed, *e.g.,* at sequencing lab 230, during wet lab processing 204. An example workflow for nucleic acid sequencing is illustrated in Figure 3. In some embodiments, the one or more biological samples obtained at the sequencing lab 230 are accessioned (302), to track the sample and data through the sequencing process.

Next, nucleic acids, *e.g.,* RNA and/or DNA are extracted (304) from the one or more biological samples. Methods for isolating nucleic acids from biological samples are known in the art and are dependent upon the type of nucleic acid being isolated (*e.g.,* cfDNA, DNA, and/or RNA) and the type of sample from which the nucleic acids are being isolated (*e.g.,* liquid biopsy samples, white blood cell buffy coat preparations, formalin-fixed paraffin-embedded (FFPE) solid tissue samples, and fresh frozen solid tissue samples). The selection of any particular nucleic acid isolation technique for use in conjunction with the embodiments described herein is well within the skill of the person having ordinary skill in the art, who will consider the sample type, the state of the sample, the type of nucleic acid being sequenced, and the sequencing technology being used.

For instance, many techniques for DNA isolation, *e.g.,* genomic DNA isolation, from a tissue sample are known in the art, such as organic extraction, silica adsorption, and anion exchange chromatography. Likewise, many techniques for RNA isolation, *e.g.,* mRNA isolation, from a tissue sample are known in the art. For example, acid guanidinium thiocyanate-phenol-chloroform extraction (see, for example, Chomczynski and Sacchi, 2006, Nat Protoc, 1(2):581-85), and silica bead/glass fiber adsorption (*see,* for example, Poeckh, T. et al., 2008, Anal Biochem., 373(2):253-62). The selection of any particular DNA or RNA isolation technique for use in conjunction with the embodiments described herein is well within the skill of the person having ordinary skill in the art, who will consider the tissue type, the state of the tissue, *e.g.,* fresh, frozen, formalin-fixed, paraffin-embedded (FFPE), and the type of nucleic acid analysis that is to be performed.

In some embodiments, isolated DNA molecules are mechanically sheared to an average length using an ultrasonicator (for example, a Covaris ultrasonicator). In some embodiments, isolated nucleic acid molecules are analyzed to determine their fragment size, *e.g.,* through gel electrophoresis techniques and/or the use of a device such as a LabChip GX Touch. The skilled artisan will know of an appropriate range of fragment sizes, based on the sequencing technique being employed, as different sequencing techniques have differing fragment size requirements for robust sequencing. In some embodiments, quality control testing is performed on the extracted nucleic acids (*e.g.,* DNA and/or RNA), *e.g.,* to assess the nucleic acid concentration and/or fragment size. For example, sizing of DNA fragments provides valuable information used for downstream processing, such as determining whether DNA fragments require additional shearing prior to sequencing.

Wet lab processing 204 then includes preparing a nucleic acid library from the isolated nucleic acids *(e.g.,* cfDNA, DNA, and/or RNA). For example, in some embodiments, DNA libraries (*e.g.,* gDNA and/or cfDNA libraries) are prepared from isolated DNA from the one or more biological samples. In some embodiments, the DNA libraries are prepared using a commercial library preparation kit, *e.g.,* the KAPA Hyper Prep Kit, a New England Biolabs (NEB) kit, or a similar kit.

In some embodiments, during library preparation, adapters (*e.g.,* UDI adapters, such as Roche SeqCap dual end adapters, or UMI adapters such as full length or stubby Y adapters) are ligated onto the nucleic acid molecules. In some embodiments, the adapters include unique molecular identifiers (UMIs), which are short nucleic acid sequences *(e.g.,* 3-10 base pairs) that are added to ends of DNA fragments during adapter ligation. In some embodiments, UMIs are degenerate base pairs that serve as a unique tag that can be used to identify sequence reads originating from a specific DNA fragment. In some embodiments, *e.g.,* when multiplex sequencing will be used to sequence DNA from a plurality of samples (*e.g.,* from the same or different subjects) in a single sequencing reaction, a patient-specific index is also added to the nucleic acid molecules. In some embodiments, the patient specific index is a short nucleic acid sequence (*e.g.,* 3-20 nucleotides) that are added to ends of DNA fragments during library construction, that serve as a unique tag that can be used to identify sequence reads originating from a specific patient sample. Examples of identifier sequences are described, for example, in Kivioja et al., Nat. Methods 9(1):72-74 (2011) and Islam et al., Nat. Methods 11(2):163-66 (2014).

In some embodiments, an adapter includes a PCR primer landing site, designed for efficient binding of a PCR or second-strand synthesis primer used during the sequencing reaction. In some embodiments, an adapter includes an anchor binding site, to facilitate binding of the DNA molecule to anchor oligonucleotide molecules on a sequencer flow cell, serving as a seed for the sequencing process by providing a starting point for the sequencing reaction. During PCR amplification following adapter ligation, the UMIs, patient indexes, and binding sites are replicated along with the attached DNA fragment. This provides a way to identify sequence reads that came from the same original fragment in downstream analysis.

In some embodiments, DNA libraries are amplified and purified using commercial reagents, (*e.g.,* Axygen MAG PCR clean up beads). In some such embodiments, the concentration and/or quantity of the DNA molecules are then quantified using a fluorescent dye and a fluorescence microplate reader, standard spectrofluorometer, or filter fluorometer. In some embodiments, library amplification is performed on a device (*e.g.,* an Illumina C-Bot2) and the resulting flow cell containing amplified target-captured DNA libraries is sequenced on a next generation sequencer (*e.g.,* an Illumina HiSeq 4000 or an Illumina NovaSeq 6000) to a unique on-target depth selected by the user. In some embodiments, DNA library preparation is performed with an automated system, using a liquid handling robot (*e.g.,* a SciClone NGSx).

In some embodiments, where feature data 125 includes methylation states 132 for one or more genomic locations, nucleic acids isolated from the biological sample (*e.g.,* cfDNA and/or DNA) are treated to convert unmethylated cytosines to uracils, e*.g.,* prior to generating the sequencing library. Accordingly, when the nucleic acids are sequenced, all cytosines called in the sequencing reaction were necessarily methylated, since the unmethylated cytosines were converted to uracils and accordingly would have been called as thymidines, rather than cytosines, in the sequencing reaction. Commercial kits are available for bisulfite-mediated conversion of methylated cytosines to uracils, for instance, the EZ DNA MethylationTM-Gold, EZ DNA Methylation^{™}-Direct, and EZ DNA Methylation^{™}-Lightning kit (available from Zymo Research Corp (Irvine, CA)). Commercial kits are also available for enzymatic conversion of methylated cytosines to uracils, for example, the APOBEC-Seq kit (available from NEBiolabs, Ipswich, MA).

In some embodiments, wet lab processing 204 includes pooling (308) DNA molecules from a plurality of libraries, corresponding to different samples from the same and/or different patients, to forming a sequencing pool of DNA libraries. When the pool of DNA libraries is sequenced, the resulting sequence reads correspond to nucleic acids isolated from multiple samples. The sequence reads can be separated into different sequence read files, corresponding to the various samples represented in the sequencing read based on the unique identifiers present in the added nucleic acid fragments. In this fashion, a single sequencing reaction can generate sequence reads from multiple samples. Advantageously, this allows for the processing of more samples per sequencing reaction.

In some embodiments, wet lab processing 204 includes enriching (310) a sequencing library, or pool of sequencing libraries, for target nucleic acids, *e.g.,* nucleic acids encompassing loci that are informative for precision oncology and/or used as internal controls for the sequencing or bioinformatics processes. In some embodiments, enrichment is achieved by hybridizing target nucleic acids in the sequencing library to probes that hybridize to the target sequences, and then isolating the captured nucleic acids away from off-target nucleic acids that are not bound by the capture probes. In some embodiments, one or more off-target nucleic acids will remain in the final sequencing pool.

Advantageously, enriching for target sequences prior to sequencing nucleic acids significantly reduces the costs and time associated with sequencing, facilitates multiplex sequencing by allowing multiple samples to be mixed together for a single sequencing reaction, and significantly reduces the computation burden of aligning the resulting sequence reads, as a result of significantly reducing the total amount of nucleic acids analyzed from each sample.

In some embodiments, the enrichment is performed prior to pooling multiple nucleic acid sequencing libraries. However, in other embodiments, the enrichment is performed after pooling nucleic acid sequencing libraries, which has the advantage of reducing the number of enrichment assays that have to be performed.

In some embodiments, the enrichment is performed prior to generating a nucleic acid sequencing library. This has the advantage that fewer reagents are needed to perform both the enrichment (because there are fewer target sequences at this point, prior to library amplification) and the library production (because there are fewer nucleic acid molecules to tag and amplify after the enrichment). However, this raises the possibility of pull-down bias and/or that small variations in the enrichment protocol will result in less consistent results.

In some embodiments, nucleic acid libraries are pooled (two or more DNA libraries may be mixed to create a pool) and treated with reagents to reduce off-target capture, for example Human COT-1 and/or IDT xGen Universal Blockers. Pools may be dried in a vacufuge and resuspended. DNA libraries or pools may be hybridized to a probe set (for example, a probe set specific to a panel that includes loci from at least 100, 600, 1,000, 10,000, etc. of the 19,000 known human genes) and amplified with commercially available reagents (for example, the KAPA HiFi HotStart ReadyMix). For example, in some embodiments, a pool is incubated in an incubator, PCR machine, water bath, or other temperature-modulating device to allow probes to hybridize. Pools may then be mixed with Streptavidin-coated beads or another means for capturing hybridized DNA-probe molecules, such as DNA molecules representing exons of the human genome and/or genes selected for a genetic panel.

Pools may be amplified and purified more than once using commercially available reagents, for example, the KAPA HiFi Library Amplification kit and Axygen MAG PCR clean up beads, respectively. The pools or DNA libraries may be analyzed to determine the concentration or quantity of DNA molecules, for example by using a fluorescent dye (for example, PicoGreen pool quantification) and a fluorescence microplate reader, standard spectrofluorometer, or filter fluorometer. In one example, the DNA library preparation and/or capture is performed with an automated system, using a liquid handling robot (for example, a SciClone NGSx).

In some embodiments, *e.g.,* where a whole genome sequencing method will be used, nucleic acid sequencing libraries are not target-enriched prior to sequencing, in order to obtain sequencing data on substantially all of the competent nucleic acids in the sequencing library. Similarly, in some embodiments, *e.g.,* where a whole genome sequencing method will be used, nucleic acid sequencing libraries are not mixed, because of bandwidth limitations related to obtaining significant sequencing depth across an entire genome. However, in other embodiments, *e.g.,* where a low-pass whole genome sequencing (LPWGS) methodology will be used, nucleic acid sequencing libraries can still be pooled, because very low average sequencing coverage is achieved across a respective genome, *e.g.,* between about 0.5X and about 5X.

In some embodiments, a plurality of nucleic acid probes (*e.g.,* a probe set) is used to enrich one or more target sequences in a nucleic acid sample (*e.g.,* an isolated nucleic acid sample or a nucleic acid sequencing library), *e.g.,* where one or more target sequences is informative for precision oncology. For instance, in some embodiments, one or more of the target sequences encompasses a locus that is associated with an actionable allele. That is, variations of the target sequence are associated with targeted therapeutic approaches. In some embodiments, one or more of the target sequences and/or a property of one or more of the target sequences is used in a classifier trained to distinguish two or more cancer states.

In some embodiments, the probe set includes probes targeting one or more gene loci, *e.g.,* exon or intron loci. In some embodiments, the probe set includes probes targeting one or more loci not encoding a protein, *e*.g*.,* regulatory loci, miRNA loci, and other non-coding loci, *e.g.,* that have been found to be associated with cancer. In some embodiments, the plurality of loci includes at least 25, 50, 100, 150, 200, 250, 300, 350, 400, 500, 750, 1000, 2500, 5000, or more human genomic loci. In some embodiments, the probe set is a whole exome sequencing panel.

Generally, probes for enrichment of nucleic acids include DNA, RNA, or a modified nucleic acid structure with a base sequence that is complementary to a locus of interest. For instance, a probe designed to hybridize to a locus in a DNA molecule can contain a sequence that is complementary to either strand, because the DNA molecules are double stranded. In some embodiments, each probe in the plurality of probes includes a nucleic acid sequence that is identical or complementary to at least 10, at least 11, at least 12, at least 13, at least 14, or at least 15 consecutive bases of a locus of interest. In some embodiments, each probe in the plurality of probes includes a nucleic acid sequence that is identical or complementary to at least 20, 25, 30, 40, 50, 75, 100, 150, 200, or more consecutive bases of a locus of interest.

Targeted panels provide several benefits for nucleic acid sequencing. For example, in some embodiments, algorithms for discriminating between, *e.g.,* a first and second disease or disorder condition can be trained on smaller, more informative data sets (*e.g.,* fewer genes), which leads to more computationally efficient training of classifiers that discriminate between the first and second cancer states. Such improvements in computational efficiency, owing to the reduced size of the discriminating gene set, can advantageously either be used to speed up classifier training or be used to improve the performance of such classifiers (*e.g.,* through more extensive training of the classifier).

In some embodiments, the gene panel is a whole-exome panel that analyzes the exomes of a biological sample. In some embodiments, the gene panel is a whole genome panel that analyzes the genome of a specimen.

In some embodiments, the probes include additional nucleic acid sequences that do not share any homology to the locus of interest. For example, in some embodiments, the probes also include nucleic acid sequences containing an identifier sequence, *e.g.,* a unique molecular identifier (UMI), *e.g.,* that is unique to a particular sample or subject. Examples of identifier sequences are described, for example, in Kivioja et al., 2011, Nat. Methods 9(1), pp. 72-74 and Islam et al., 2014, Nat. Methods 11(2), pp. 163-66. Similarly, in some embodiments, the probes also include primer nucleic acid sequences useful for amplifying the nucleic acid molecule of interest, *e.g.,* using PCR. In some embodiments, the probes also include a capture sequence designed to hybridize to an anti-capture sequence for recovering the nucleic acid molecule of interest from the sample.

Likewise, in some embodiments, the probes each include a non-nucleic acid affinity moiety covalently attached to nucleic acid molecule that is complementary to the locus of interest, for recovering the nucleic acid molecule of interest. Non-limited examples of non-nucleic acid affinity moieties include biotin, digoxigenin, and dinitrophenol. In some embodiments, the probe is attached to a solid-state surface or particle, *e.g.,* a dipstick or magnetic bead, for recovering the nucleic acid of interest. In some embodiments, the methods described herein include amplifying the nucleic acids that bound to the probe set prior to further analysis, *e.g.,* sequencing. Methods for amplifying nucleic acids, *e.g.,* by PCR, are well known in the art.

Sequence reads are then generated (312) from the sequencing library or pool of sequencing libraries. Sequencing data may be acquired by any methodology known in the art. For example, next generation sequencing (NGS) techniques such as sequencing-by-synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators. In some embodiments, sequencing is performed using next generation sequencing technologies, such as short-read technologies. In other embodiments, long-read sequencing or another sequencing method known in the art is used.

Next-generation sequencing produces millions of short reads (*e.g.,* sequence reads) for each biological sample. Accordingly, in some embodiments, the plurality of sequence reads obtained by next-generation sequencing of nucleic acid molecules are DNA sequence reads. In some embodiments, the sequence reads have an average length of at least fifty nucleotides. In other embodiments, the sequence reads have an average length of at least 50, 60, 70, 80, 90, 100, 150, 200, 250, 300, or more nucleotides.

In some embodiments, sequencing is performed after enriching for nucleic acids (*e.g.,* cfDNA, gDNA, and/or RNA) encompassing a plurality of predetermined target sequences, *e.g.,* human genes and/or non-coding sequences associated with cancer. Advantageously, sequencing a nucleic acid sample that has been enriched for target nucleic acids, rather than all nucleic acids isolated from a biological sample, significantly reduces the average time and cost of the sequencing reaction. Accordingly, in some embodiments, the methods described herein include obtaining a plurality of sequence reads of nucleic acids that have been hybridized to a probe set for hybrid-capture enrichment (*e.g.,* of one or more genes listed in Table 1, List 1, and/or List 2).

In some embodiments, panel-targeting sequencing is performed to an average on-target depth of at least 30X, at least 40X, at least 50X, at least 60X, at least 70X, at least 80X, at least 90X, at least 100X, at least 500X, at least 750X, at least 1000X, at least 2500X, at least 500X, at least 10,000X, or greater depth. In some embodiments, samples are further assessed for uniformity above a sequencing depth threshold (*e.g.,* 95% of all targeted base pairs at 300X sequencing depth). In some embodiments, the sequencing depth threshold is a minimum depth selected by a user or practitioner.

In some embodiments, the sequence reads are obtained by a whole genome sequencing methodology. As described herein, the whole genome sequencing is performed at lower sequencing depth than smaller target-panel sequencing reactions, because many more loci are being sequenced. For example, in some embodiments, whole genome sequencing is performed to an average sequencing depth of at least 0.2X, at least 0.5X, at least 1X, at least 1.5X, at least 2X, at least 2.5X, at least 3X, at least 3.5X, at least 4X, at least 4.5X, or greater. In some embodiments, whole genome sequencing is performed to an average sequencing depth of no more than 7.5X, no more than 7X, no more than 6.5X, no more than 6X, no more than 5.5X, no more than 5X, no more than 4.5X, no more than 4X, no more than 3.5X, no more than 3X, no more than 2.5X, no more than 2X, no more than 1.5X, no more than 1X, or less. In some embodiments, low-pass whole genome sequencing (LPWGS) is performed to an average sequencing depth of about 0.25X to about 5X, or to an average sequencing depth of about 0.5X to about 5X, or to an average sequencing depth of about 1X to about 5X, or to an average sequencing depth of about 2X to about 5X, or to an average sequencing depth of about 3X to about 5X, or to an average sequencing depth of about 1X to about 4X, or to an average sequencing depth of about 1X to about 3X, or to an average sequencing depth of about 1.5X to about 4X, or to an average sequencing depth of about 1.5X to about 3X, or to an average sequencing depth of about 2X to about 3X.

In some embodiments, the raw sequence reads resulting from the sequencing reaction are output from the sequencer in a native file format, *e.g.,* a BCL file. In some embodiments, the native file is passed directly to a bioinformatics pipeline (*e.g.,* variant analysis 206), components of which are described in detail below. In other embodiments, pre-processing is performed prior to passing the sequences to the bioinformatics platform. For instance, in some embodiments, the format of the sequence read file is converted from the native file format (*e.g.,* BCL) to a file format compatible with one or more algorithms used in the bioinformatics pipeline (*e.g.,* FASTQ or FASTA). In some embodiments, the raw sequence reads are filtered to remove sequences that do not meet one or more quality thresholds. In some embodiments, raw sequence reads generated from the same unique nucleic acid molecule in the sequencing read are collapsed into a single sequence read representing the molecule, *e.g.,* using UMIs as described above. In some embodiments, one or more of these pre-processing activities is performed within the bioinformatics pipeline itself.

In one example, a sequencer may generate a BCL file. A BCL file may include raw image data of a plurality of patient specimens which are sequenced. BCL image data is an image of the flow cell across each cycle during sequencing. A cycle may be implemented by illuminating a patient specimen with a specific wavelength of electromagnetic radiation, generating a plurality of images which may be processed into base calls via BCL to FASTQ processing algorithms which identify which base pairs are present at each cycle. The resulting FASTQ file includes the entirety of reads for each patient specimen paired with a quality metric, *e.g.,* in a range from 0 to 64 where a 64 is the best quality and a 0 is the worst quality. In embodiments where both a diseased tissue sample and a non-diseased tissue sample are sequenced, sequence reads in the corresponding FASTQ files may be matched, such that a diseased-normal analysis may be performed.

FASTQ format is a text-based format for storing both a biological sequence, such as a nucleotide sequence, and its corresponding quality scores. These FASTQ files are analyzed to determine what genetic variants or copy number changes are present in the sample. Each FASTQ file contains reads that may be paired-end or single reads and may be short-reads or long-reads, where each read represents one detected sequence of nucleotides in a nucleic acid molecule that was isolated from the patient sample or a copy of the nucleic acid molecule, detected by the sequencer. Each read in the FASTQ file is also associated with a quality rating. The quality rating may reflect the likelihood that an error occurred during the sequencing procedure that affected the associated read. In some embodiments, the results of paired-end sequencing of each isolated nucleic acid sample are contained in a split pair of FASTQ files, for efficiency. Thus, in some embodiments, forward (Read 1) and reverse (Read 2) sequences of each isolated nucleic acid sample are stored separately but in the same order and under the same identifier.

In various embodiments, the bioinformatics pipeline may filter FASTQ data from the corresponding sequence data file for each respective biological sample. Such filtering may include correcting or masking sequencer errors and removing (trimming) low quality sequences or bases, adapter sequences, contaminations, chimeric reads, overrepresented sequences, biases caused by library preparation, amplification, or capture, and other errors.

While workflow 200 illustrates obtaining a biological sample, extracting nucleic acids from the biological sample, and sequencing the isolated nucleic acids, in some embodiments, sequencing data used in the improved systems and methods described herein (*e.g.,* which include improved methods for determining copy number variation status) is obtained by receiving previously generated sequence reads, in electronic form.

Figure 4A illustrates an example bioinformatics pipeline 206 (*e.g.,* as used for feature extraction in the various workflows illustrated in the Figures and described herein) for providing clinical support for treatment of a disease or disorder. As shown in Figure 4A, sequencing data 122 obtained from the wet lab processing 204 (*e.g.,* sequence reads 314) is input into the pipeline. The pipeline may detect SNVs, INDELs, copy number amplifications/deletions and genomic rearrangements (for example, fusions). The pipeline may employ unique molecular index (UMI)-based consensus base calling as a method of error suppression as well as a Bayesian tri-nucleotide context-based position level error suppression. In various embodiments, it is able to detect variants having a 0.1%, 0.15%, 0.2%, 0.25%, 0.3%, 0.4%, or 0.5% variant allele fraction.

In some embodiments, the sequencing data is processed (*e.g.,* using sequence data processing module 141) to prepare it for genomic feature identification 385. For instance, in some embodiments as described above, the sequencing data is present in a native file format provided by the sequencer. Accordingly, in some embodiments, the system (*e.g.,* system 100) applies a pre-processing algorithm 142 to convert the file format (318) to one that is recognized by one or more upstream processing algorithms. For example, BCL file outputs from a sequencer can be converted to a FASTQ file format using the bcl2fastq or bcl2fastq2 conversion software (Illumina^{®}). FASTQ format is a text-based format for storing both a biological sequence, such as nucleotide sequence, and its corresponding quality scores. These FASTQ files are analyzed to determine what genetic variants, copy number changes, etc., are present in the sample.

In some embodiments, other preprocessing functions are performed, *e.g.,* filtering sequence reads 122 based on a desired quality, *e.g.,* size and/or quality of the base calling. In some embodiments, quality control checks are performed to ensure the data is sufficient for variant calling. For instance, entire reads, individual nucleotides, or multiple nucleotides that are likely to have errors may be discarded based on the quality rating associated with the read in the FASTQ file, the known error rate of the sequencer, and/or a comparison between each nucleotide in the read and one or more nucleotides in other reads that has been aligned to the same location in the reference genome. Filtering may be done in part or in its entirety by various software tools, for example, a software tool such as Skewer. See, Jiang, H. et al., BMC Bioinformatics 15(182):1-12 (2014). FASTQ files may be analyzed for rapid assessment of quality control and reads, for example, by a sequencing data QC software such as AfterQC, Kraken, RNA-SeQC, FastQC, or another similar software program. For paired end reads, reads may be merged.

In some embodiments, two FASTQ output files are generated, one for the WGS data and one for the targeted-panel sequencing data. If two or more patient samples are processed simultaneously on the same sequencer flow cell, *e.g.,* a WGS reaction and a targeted panel sequencing reaction, a difference in the sequence of the adapters used for each patient sample barcodes nucleic acids extracted from both samples, to associate each read with the correct patient sample and facilitate assignment to the correct FASTQ file.

For efficiency, in some embodiments, the results of paired-end sequencing of each isolate are contained in a split pair of FASTQ files. Forward (Read 1) and reverse (Read 2) sequences of each sequencing run are stored separately but in the same order and under the same identifier. In various embodiments, the bioinformatics pipeline may filter FASTQ data from each isolate. Such filtering may include correcting or masking sequencer errors and removing (trimming) low quality sequences or bases, adapter sequences, contaminations, chimeric reads, overrepresented sequences, biases caused by library preparation, amplification, or capture, and other errors.

Similarly, in some embodiments, sequencing (312) is performed on a pool of nucleic acid sequencing libraries prepared from different biological samples, *e.g.,* from the same or different patients. Accordingly, in some embodiments, the system demultiplexes (320) the data (*e.g.,* using demultiplexing algorithm 144) to separate sequence reads into separate files for each sequencing library included in the sequencing pool, *e.g.,* based on UMI or patient identifier sequences added to the nucleic acid fragments during sequencing library preparation, as described above. In some embodiments, the demultiplexing algorithm is part of the same software package as one or more pre-processing algorithms 142. For instance, the bcl2fastq or bcl2fastq2 conversion software (Illumina^{®}) include instructions for both converting the native file format output from the sequencer and demultiplexing sequence reads 122 output from the reaction.

In some embodiments, the sequence reads are then aligned (322), *e.g.,* using an alignment algorithm 143, to a reference sequence construct 158, *e.g.,* a reference genome, reference exome, or other reference construct prepared for a particular targeted-panel sequencing reaction. For example, in some embodiments, individual sequence reads 123, in electronic form (*e.g.,* in FASTQ files), are aligned against a reference sequence construct for the species of the subject (*e.g.,* a reference human genome) by identifying a sequence in a region of the reference sequence construct that best matches the sequence of nucleotides in the sequence read. In some embodiments, the sequence reads are aligned to a reference exome or reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene. Any of a variety of alignment tools can be used for this task.

For instance, local sequence alignment algorithms compare subsequences of different lengths in the query sequence (*e.g.,* sequence read) to subsequences in the subject sequence (*e.g.,* reference construct) to create the best alignment for each portion of the query sequence. In contrast, global sequence alignment algorithms align the entirety of the sequences, *e.g.,* end to end. Examples of local sequence alignment algorithms include the Smith-Waterman algorithm (see, *for example,* Smith and Waterman, J Mol. Biol., 147(1):195-97 (1981)), Lalign (see, *for example,* Huang and Miller, Adv. Appl. Math, 12:337-57 (1991)), and PatternHunter (see, *for example,* Ma B. et al., Bioinformatics, 18(3):440-45 (2002)).

In some embodiments, the read mapping process starts by building an index of either the reference genome or the reads, which is then used to retrieve the set of positions in the reference sequence where the reads are more likely to align. Once this subset of possible mapping locations has been identified, alignment is performed in these candidate regions with slower and more sensitive algorithms. See, for example, Hatem et al., 2013, "Benchmarking short sequence mapping tools," BMC Bioinformatics 14: p. 184; and Flicek and Birney, 2009, "Sense from sequence reads: methods for alignment and assembly," Nat Methods 6(Suppl. 11), S6-S12. In some embodiments, the mapping tools methodology makes use of a hash table or a Burrows-Wheeler transform (BWT). See, for example, Li and Homer, 2010, "A survey of sequence alignment algorithms for next-generation sequencing," Brief Bioinformatics 11, pp. 473-483.

Other software programs designed to align reads include, for example, Novoalign (Novocraft, Inc.), Bowtie, Burrows Wheeler Aligner (BWA), and/or programs that use a Smith-Waterman algorithm. Candidate reference genomes include, for example, hg19, GRCh38, hg38, GRCh37, and/or other reference genomes developed by the Genome Reference Consortium. In some embodiments, the alignment generates a SAM file, which stores the locations of the start and end of each read according to coordinates in the reference genome and the coverage (number of reads) for each nucleotide in the reference genome.

For example, in some embodiments, each read of a FASTQ file is aligned to a location in the human genome having a sequence that best matches the sequence of nucleotides in the read. There are many software programs designed to align reads, for example, Novoalign (Novocraft, Inc.), Bowtie, Burrows Wheeler Aligner (BWA), programs that use a Smith-Waterman algorithm, etc. Alignment may be directed using a reference genome (for example, hg19, GRCh38, hg38, GRCh37, other reference genomes developed by the Genome Reference Consortium, *etc*.) by comparing the nucleotide sequences in each read with portions of the nucleotide sequence in the reference genome to determine the portion of the reference genome sequence that is most likely to correspond to the sequence in the read. In some embodiments, one or more SAM files are generated for the alignment, which store the locations of the start and end of each read according to coordinates in the reference genome and the coverage (number of reads) for each nucleotide in the reference genome. The SAM files may be converted to BAM files. In some embodiments, the BAM files are sorted, and duplicate reads are marked for deletion, resulting in de-duplicated BAM files.

In some embodiments, adapter-trimmed FASTQ files are aligned to the 19th edition of the human reference genome build (HG19) using Burrows-Wheeler Aligner (BWA, Li and Durbin, Bioinformatics, 25(14):1754-60 (2009). Following alignment, reads are grouped by alignment position and UMI family and collapsed into consensus sequences, for example, using fgbio tools (*e.g.,* available on the internet at fulcrumgenomics.github.io/fgbio/). Bases with insufficient quality or significant disagreement among family members (for example, when it is uncertain whether the base is an adenine, cytosine, guanine, *etc*.) may be replaced by N's to represent a wildcard nucleotide type. PHRED scores are then scaled based on initial base calling estimates combined across all family members. Following single-strand consensus generation, duplex consensus sequences are generated by comparing the forward and reverse oriented PCR products with mirrored UMI sequences. In various embodiments, a consensus can be generated across read pairs. Otherwise, single-strand consensus calls will be used. Following consensus calling, filtering is performed to remove low-quality consensus fragments. The consensus fragments are then realigned to the human reference genome using BWA. A BAM output file is generated after the re-alignment, then sorted by alignment position, and indexed.

In some embodiments, this process produces a WGS BAM file (*e.g.,* WGS BAM 124-1-i-w) and a targeted-panel sequencing BAM file (*e.g.,* Targeted-panel BAM 124-1-i-p), as illustrated in Figure 4A. In various embodiments, BAM files may be analyzed to detect genetic variants and other genetic features, including single nucleotide variants (SNVs), copy number variants (CNVs), gene rearrangements, etc.

In some embodiments, the sequencing data is normalized, *e.g.,* to account for pull-down, amplification, and/or sequencing bias (*e.g.,* mappability, GC bias etc.). See, for example, Schwartz et al., PLoS ONE 6(1):e16685 (2011) and Benjamini and Speed, Nucleic Acids Research 40(10):e72 (2012).

In some embodiments, SAM files generated after alignment are converted to BAM files 124. Thus, after preprocessing sequencing data generated for a pooled sequencing reaction, BAM files are generated for each of the sequencing libraries present in the master sequencing pools. In some embodiments, one or more samples acquired from one or more additional subjects at time j (*e.g.,* WGS BAM 124-2-j-w corresponding to alignments of sequence reads of nucleic acids isolated from a sample from subject 2). In some embodiments, BAM files are sorted, and duplicate reads are marked for deletion, resulting in de-duplicated BAM files. For example, tools like SamBAMBA mark and filter duplicate alignments in the sorted BAM files.

Generally, the methods and systems described herein are independent and, thus, not reliant upon any particular sequencing data generation methods, *e.g.,* sample preparation, sequencing, and/or data pre-processing methodologies. However, in some embodiments, the methods described below include one or more features 204 of generating sequencing data, as illustrated in Figures 2A and 3.

Alignment files prepared as described above (*e.g.,* BAM files 124) are then passed to a feature extraction module 145, where the sequences are analyzed (324) to identify genomic alterations (*e.g.,* SNVs/MNVs, indels, genomic rearrangements, copy number variations, *etc*.) and/or determine various characteristics of the patient's disease or disorder. Many software packages for identifying genomic alterations are known in the art, for example, freebayes, PolyBayse, samtools, GATK, pindel, SAMtools, Breakdancer, Cortex, Crest, Delly, Gridss, Hydra, Lumpy, Manta, and Socrates. For a review of many of these variant calling packages see, for example, Cameron, D.L. et al., Nat. Commun., 10(3240):1-11 (2019). Generally, these software packages identify variants in sorted SAM or BAM files 124, relative to one or more reference sequence constructs 158. The software packages then output a file *e.g.,* a raw VCF (variant call format), listing the variants (*e.g.,* genomic features 131) called and identifying their location relevant to the reference sequence construct (*e.g.,* where the sequence of the sample nucleic acids differ from the corresponding sequence in the reference construct). In some embodiments, system 100 digests the contents of the native output file to populate feature data 125 in test patient data store 120. In other embodiments, the native output file serves as the record of these genomic features 131 in test patient data store 120.

Generally, the systems described herein can employ any combination of available variant calling software packages and internally developed variant identification algorithms. In some embodiments, the output of a particular algorithm of a variant calling software is further evaluated, *e.g.,* to improve variant identification. Accordingly, in some embodiments, system 100 employs an available variant calling software package to perform some of all of the functionality of one or more of the algorithms shown in feature extraction module 145.

In various aspects, the detected genetic variants and genetic features are analyzed as a form of quality control. For example, a pattern of detected genetic variants or features may indicate an issue related to the sample, sequencing procedure, and/or bioinformatics pipeline (*e.g.,* example, contamination of the sample, mislabeling of the sample, a change in reagents, a change in the sequencing procedure and/or bioinformatics pipeline, *etc*.).

Generally, any combination of the modules and algorithms of feature extraction module 145, *e.g.,* illustrated in Figure 1A, can be used for a bioinformatics pipeline used in conjunction with the methods and systems described herein. For instance, in some embodiments, an architecture useful for the methods and systems described herein includes at least one of the modules or variant calling algorithms shown in feature extraction module 145. In some embodiments, an architecture includes at least 2, 3, 4, 5, 6, 7, 8, 9, 10, or more of the modules or variant calling algorithms shown in feature extraction module 145. Further, in some embodiments, feature extraction modules and/or algorithms not illustrated in Figure 1A find use in the methods and systems described herein.

### Variant Identification

In some embodiments, variant analysis of aligned sequence reads, *e.g.,* in SAM or BAM format, includes identification of single nucleotide variants (SNVs), multiple nucleotide variants (MNVs), indels (*e.g.,* nucleotide additions and deletions), and/or genomic rearrangements (*e.g.,* inversions, translocations, and gene fusions) using variant identification module 146, *e.g.,* which includes a SNV/MNV calling algorithm (*e.g.,* SNV/MNV calling algorithm 147), an indel calling algorithm (*e.g.,* indel calling algorithm 148), and/or one or more genomic rearrangement calling algorithms (*e.g.,* genomic rearrangement calling algorithm 149). In some embodiments, the module first identifies a difference between the sequence of an aligned sequence read 124 and the reference sequence to which the sequence read is aligned (*e.g.,* an SNV/MNV, an indel, or a genomic rearrangement) and makes a record of the variant, *e.g.,* in a variant call format (VCF) file. For instance, software packages such as freebayes and pindel are used to call variants using sorted BAM files and reference BED files as the input. For a review of variant calling packages see, for example, Cameron, D.L. et al., Nat. Commun., 10(3240):1-11 (2019). A raw VCF file (variant call format) file is output, showing the locations where the nucleotide base in the sample is not the same as the nucleotide base in that position in the reference sequence construct.

In some embodiments, SNV/INDEL detection is accomplished using VarDict (available on the internet at github.com/AstraZeneca-NGS/VarDictJava). Both SNVs and INDELs are called and then sorted, deduplicated, normalized and annotated. The annotation uses SnpEff to add transcript information, 1000 genomes minor allele frequencies, COSMIC reference names and counts, ExAC allele frequencies, and Kaviar population allele frequencies. The annotated variants are then classified as germline, somatic, or uncertain using a Bayesian model based on prior expectations informed by databases of germline and cancer variants. In some embodiments, uncertain variants are treated as somatic for filtering and reporting purposes.

In some embodiments, genomic rearrangements (*e.g.,* inversions, translocations, and gene fusions) are detected following de-multiplexing by aligning tumor FASTQ files against a human reference genome using a local alignment algorithm, such as BWA. In some embodiments, DNA reads are sorted and duplicates may be marked with a software, for example, SAMBlaster. Discordant and split reads may be further identified and separated. These data may be read into a software, for example, LUMPY, for structural variant detection. In some embodiments, structural alterations are grouped by type, recurrence, and presence and stored within a database and displayed through a fusion viewer software tool. The fusion viewer software tool may reference a database, for example, Ensembl, to determine the gene and proximal exons surrounding the breakpoint for any possible transcript generated across the breakpoint. The fusion viewer tool may then place the breakpoint 5' or 3' to the subsequent exon in the direction of transcription. For inversions, this orientation may be reversed for the inverted gene. After positioning of the breakpoint, the translated amino acid sequences may be generated for both genes in the chimeric protein, and a plot may be generated containing the remaining functional domains for each protein, as returned from a database, for example, Uniprot.

For instance, in an example implementation, gene rearrangements are detected using the SpeedSeq analysis pipeline. Chiang et al., 2015, "SpeedSeq: ultra-fast personal genome analysis and interpretation," Nat Methods, (12), pg. 966. Briefly, FASTQ files are aligned to hg19 using BWA. Split reads mapped to multiple positions and read pairs mapped to discordant positions are identified and separated, then utilized to detect gene rearrangements by LUMPY. Layer et al., 2014, "LUMPY: a probabilistic framework for structural variant discovery," Genome Biol, (15), pg. 84. Fusions can then be filtered according to the number of supporting reads.

### Allelic Fraction Determination

In some embodiments, the analysis of aligned sequence reads, *e.g.,* in SAM or BAM format, includes determination of variant allele fractions (133) for one or more of the variant alleles 132 identified as described above. In some embodiments, a variant allele fraction module 151 tallies the instances that each allele is represented by a unique sequence read encompassing the variant locus of interest, generating a count for each allele represented at that locus. In some embodiments, these tallies are used to determine the ratio of the variant allele, *e.g.,* an allele other than the most prevalent allele in the subject's population for a respective locus, to a reference allele. This variant allele fraction 133 can be used in several places in the feature extraction 206 workflow. For instance, in some embodiments, a variant allele fraction is used during annotations of identified variants, *e.g.,* when determining whether the allele originated from a germline cell or a somatic cell. In other instances, a variant allele fraction is used in a process for estimating a tumor fraction for a liquid biopsy sample or a tumor purity for a solid tumor fraction. For instance, variant allele fractions for a plurality of somatic alleles can be used to estimate the percentage of sequence reads originating from one copy of a cancerous chromosome. Assuming a 100% tumor purity and that each cancer cell carries one copy of the variant allele, the overall purity of the tumor can be estimated. This estimate can be further corrected based on other information extracted from the sequencing data, such as copy number alterations, tumor ploidy aberrations, tumor heterozygosity, *etc.*

### Methylation Determination

In some embodiments, where nucleic acid sequencing library was processed by bi-sulfite treatment or enzymatic methyl-cytosine conversion, as described above, the analysis of aligned sequence reads, *e.g.,* in SAM or BAM format, includes determination of methylation states 132 for one or more loci in the genome of the patient. In some embodiments, methylation sequencing data is aligned to a reference sequence construct 158 in a different fashion than non-methylation sequencing, because non-methylated cytosines are converted to uracils, and the resulting uracils are ultimately sequenced as thymines, whereas methylated cytosine are not converted and sequenced as cytosine. Different approaches, therefore, have to be used to align these modified sequences to a reference sequence construct, such as seeding alignments with shorter regions of identity or converting all cytosines to thymidines in the sequencing data and then aligning the data to reference sequence constructs for both the plus and minus strand of the sequence construct. For review of these approaches, see Zhou Q. et al., BMC Bioinformatics, 20(47):1-11 (2019). Algorithms for calling methylated bases are known in the art. For example, Bismark is able to distinguish between cytosines in CpG, CHG, and CHH contexts. Krueger F. and Andrews SR, Bioinformatics, 27(11):1571-71 (2011).

### Copy Number Variation

In some embodiments, the analysis of aligned sequence reads, *e.g.,* in SAM or BAM format, includes determination of the copy number 135 for one or more locus, using a copy number variation analysis module 153. For example, Figure 4B illustrates a workflow of an exemplary method 400 for determining copy number variation status to support clinical decision making in treating a disease or disorder.

Referring to Blocks 401-w and 401-p, in some embodiments, the methods described herein include an active step of sequencing one or more biological samples from a subject by low-pass whole genome sequencing and targeted panel sequencing. However, in some embodiments, systems for performing the methods described herein access prior sequencing data, eliminating the need to actively sequence one or more patient samples.

Referring to Blocks 402 and 404, the method comprises obtaining a first dataset *(e.g.,* 135-wgs-seq) of DNA sequencing data *(e.g.,* from a first biological sample of the subject) and a second dataset *(e.g.,* 135-pt-seq) of DNA sequencing data *(e.g.,* from a second biological sample of the subject). The sequencing data can be obtained using any of the methods and/or embodiments disclosed herein, including any of the implementations for wet lab processing 204.

Referring to Blocks 406 and 407, sequence reads obtained from the first and second datasets of DNA sequencing data are mapped to positions in a reference human construct (e.g., a reference genome, exome, or construct corresponding to a targeted panel), thus generating a plurality of aligned reads 408 and 409, respectively.

Referring to Blocks 410 and 411, the method includes obtaining mapped datasets for the WGS and targeted-panel sequencing data. As described above with reference to copy number variation (CNV) analysis module 153, illustrated in Figure 1D, in various embodiments, the mapped datasets include pluralities of mapped sequences, binned values, copy number states, and/or dimensionality-reduced component values thereof. Accordingly, in some embodiments, referring to Blocks 412 and 14, the method further comprises obtaining bin values for the first mapped dataset (*e.g.,* 135-wgs-bv) and/or obtaining bin values for the second mapped dataset (*e.g.,* 135-pt-bv). Similarly, referring to Blocks 416 and 418, in some embodiments, the method further includes obtaining copy number states for the first mapped dataset (*e.g.,* 135-wgs-cns) and/or obtaining copy number states for the second mapped dataset (*e.g.,* 135-pt-cns), e.g., using one or more of the copy number methodologies described herein.

For instance, in an example implementation, copy number variants (CNVs) are analyzed using the CNVkit package. See, Talevich et al., PLoS Comput Biol, 12:1004873 (2016). CNVkit is used for genomic region binning, coverage calculation, bias correction, normalization to a reference pool, segmentation and visualization. The log₂ ratios between the tumor sample and a pool of process matched healthy samples from the CNVkit output are then annotated and filtered using statistical models whereby the amplification status (amplified or not-amplified) of each gene is predicted and non-focal amplifications are removed.

In some embodiments, copy number variations (CNVs) are analyzed using a combination of an open-source tool, such as CNVkit, and an annotation/filtering algorithm, *e.g.,* implemented via a python script. CNVkit is used initially to perform genomic region binning, coverage calculation, bias correction, normalization to a reference pool, segmentation and, optionally, visualization. The bin-level copy ratios and segment-level copy ratios, in addition to their corresponding confidence intervals, from the CNVkit output are then used in the annotation and filtering where the copy number state (amplified, neutral, deleted) of each segment and bin are determined and non-focal amplifications/deletions are filtered out based on a set of acceptance criteria. In some embodiments, one or more copy number variations selected from amplifications in the MET, EGFR, ERBB2, CD274, CCNE1, and MYC genes, and deletions in the BRCA1 and BRCA2 genes are analyzed. However, the methods described herein is not limited to only these reportable genes.

In some embodiments, CNV analysis is performed using a tumor BAM file, a target region BED file, a pool of process matched normal samples, and inputs for initial reference pool construction. Inputs for initial reference pool construction include one or more of normal BAM files, a human reference genome file, mappable regions of the genome, and a block list that contains recurrent problematic areas of the genome.

CNVkit utilizes both targeted captured sequencing reads and non-specifically captured off-target reads to infer copy number information. The targeted genomic regions specified in the probe target BED file are divided to target bins with an average size of, *e.g.,* 100 base pairs, which can be specified by the user. The genomic regions between the target regions, *e.g.,* excluding regions that cannot be mapped reliably, are automatically divided into off-target (also referred to as anti-target) bins with an average size of, *e.g.,* 150 kbp, which again can be specified by the user. Raw log₂-transformed depths are then calculated from the alignments in the input BAM file and written to two tab-delimited .cnn files, one for each of the target and off-target bins.

A pooled reference is constructed from a panel of process matched normal samples. The raw log₂ depths of target and off-target bins in each normal sample are computed as described above, and then each are median-centered and corrected for bias including GC content, genome sequence repetitiveness, target size, and/or spacing. The corrected target and off-target log₂ depths are combined, and a weighted average and spread are calculated as Tukey's biweight location and midvariance in each bin. These values are written to a tab delimited reference .cnn file, which is used to normalize an input tumor sample as follows.

The raw log₂ depths of an input sample are median-centered and bias-corrected as described in the reference construction. The corrected log₂ depth of each bin is then subtracted by the corresponding log₂ depth in the reference file, resulting in the log₂ copy ratios (also referred to as copy ratios or log₂ ratios) between the input tumor sample and the reference pool. These values are written to a tab-delimited .cnr file.

The copy ratios are then segmented, *e.g.,* via a circular binary segmentation (CBS) algorithm or another suitable segmentation algorithm, whereby adjacent bins are grouped to larger genomic regions (segments) of equal copy number. The segment's copy ratio is calculated as the weighted mean of all bins within the segment. The confidence interval of the segment mean is estimated by bootstrapping the bin-level copy ratios within the segment. The segments' genomic ranges, copy ratios and confidence intervals are written to a tab-delimited .cns file.

In some embodiments, copy number analysis includes application of a circular binary segmentation algorithm and selection of segments with highly differential log₂ ratios between the cancer sample and its comparator (*e.g.,* a matched normal or normal pool). In some embodiments, approximate integer copy number is assessed from a combination of differential coverage in segmented regions and an estimate of stromal admixture (for example, tumor purity, or the portion of a sample that is cancerous vs. non-cancerous, such as a tumor fraction for a liquid biopsy sample) is generated by analysis of heterozygous germline SNVs. In some embodiments, the integer copy number of a genomic segment in a cancer sample is used to assign a copy number status annotation to the genomic segment (*e.g.,* amplified, neutral, deleted) based on a comparison with the integer copy number of a corresponding genomic segment in a reference pool.

Any suitable method for determining copy number state is contemplated for use in the present disclosure. For example, in some embodiments, a copy number state is calculated across a respective plurality of bins using a stochastic modeling algorithm and the bin value for each respective bin in the respective plurality of bins. In some embodiments, the stochastic modeling algorithm is a Hidden Markov Model algorithm.

In some embodiments, a copy number state is determined using a single sample approach (*e.g.,* without a reference or a control sample). In some such embodiments, sequence reads are mapped to a reference genome to form a plurality of genomic regions. Genomic regions are binned into variable-sized bins and read coverage is determined for each bin. For coverage normalization, the variable-sized bins are selected to contain a constant number of mappable positions (such an approach can smooth stochastic sampling noise). For an exemplary reference sequence, the mappability for various sequencing methodologies (*e.g.,* fragment or mate pair) and read lengths can be determined. This can be used to predict, for each position in the reference sequence, whether it is likely to be capable of having reads uniquely map there or not based on the degree of homology or repetitiveness elsewhere in the reference sequence. Within these bins, coverage can be further normalized based on predicted mappability and GC content of the bins. In various embodiments, a Hidden Markov Model (HMM) can be used for segmentation, applying empirically derived filters to one or more contiguous bins to call copy number states. In some such embodiments, the copy number states of the bins are determined, and any copy number variations present can be detected for each genomic region.

In some embodiments, a copy number state is performed using a paired-sample approach. In some such embodiments, rather than comparing to the predicted mappability of the reference sequence, the coverage of the sample of the subject can be normalized by comparing it to the coverage of a control sample. Using such an approach can, in some instances, address systematic issues such as mappability and/or GC content, which may be expected to be similar between both samples, thus simplifying normalization. In some such embodiments, nucleic acid sequence reads are obtained for the sample of the subject and a control sample. For each sample, the plurality of nucleic acid sequence reads is aligned to a reference sequence and the aligned reads form a plurality of genomic regions. In various embodiments, the subject sample and the control sample nucleic acid sequence reads can be stored in a single nucleic acid sequence data file. Nucleic acid sequence read coverage is determined for each base position of the plurality of genomic regions of the subject sample and the control sample. Each of the plurality of genomic regions of the subject sample and the control sample is binned into one or more non-overlapping fixed-size bins. In various embodiments, the bin size can be variable and determined, for example, by fixing the number of positions of a control sample with coverage. Nucleic acid sequence read coverage for each bin is determined and, to adjust for coverage differences in the samples, coverage of each bin is normalized by the mean coverage of the respective sample. Nucleic acid sequence read coverage ratios for each bin of the subject sample is determined by dividing the read coverage of each bin of the subject sample with the read coverage of a corresponding bin of the control sample. In some embodiments, a stochastic modeling algorithm (*e.g.,* a Hidden Markov Modeling (HMM) algorithm) is used to convert the normalized nucleic acid sequence read coverage ratios for each bin of the subject sample to discrete copy number states. In some embodiments, the discrete copy number states of each bin of the subject sample is utilized to identify copy number variation in the genomic regions of the subject sample. In various embodiments, adjacent bins with the same copy number are merged into segments for CNV reporting purposes. In various embodiments, bins are filtered before they are merged into a segment to meet minimum segment length requirements and/or window region mappability thresholds. *See, e.g.,* United States Patent Application US17/225,833, filed April 8, 2021.

In some embodiments, the determining the respective copy number state comprises a read count approach, a paired-end approach, and/or an assembly approach.

Read count approaches are generally performed by counting the number of nucleic acid sequence reads that are mapped to a genomic region within each frame of a non-overlapping sliding window. Read count values are used to identify regions with copy number variations. Paired-end approaches are typically used with paired-end next-generation sequencing methodologies and identify genomic aberrations based on distances between paired reads. For instance, in paired-end sequencing data, sequence reads are obtained for each of the two ends of genomic regions. The distance between pairs of paired-end reads is used as an indicator of a genomic aberration, such that genomic aberrations are detected when the distance is significantly different from the predetermined average insert size. Assembly approaches assemble genomic regions by connecting overlapping short reads (contigs). Copy number variations are detected by comparing the assembled contigs to the reference genome. Unlike read count approaches, assembly approaches do not perform an alignment of the sequence reads to the reference genome prior to assembly.

Moreover, any suitable tool for determining copy number state is contemplated for use in the present disclosure. For example, in some implementations, a respective copy number state is determined using a copy number variation detection tool. Examples of copy number variation detection tools contemplated for use in the present disclosure include, but are not limited to, ADTEx, CONTRA, cnMOPS, ExomeCNV, VarScan2, CNVkit, RTG segment, CNVnator, and/or CoNVEX. See, *e.g.,* Zare et al., "An evaluation of copy number variation detection tools for cancer using whole exome sequencing data," BMC Bioinformatics (2017) 18:286.

Referring to Block 420, the method includes applying a model, such as copy number variation model 153-h, as illustrated with respect to Figure 1D, to all or a portion of the first mapped dataset and all or a portion of the second mapped dataset, or a plurality of dimensionality reduction components thereof, thereby identifying one or more copy number variation states 434, as output of the model, that indicate the copy number variation status of the subject 424. As described in detail herein, in some embodiments, the model is applied within the framework of a heuristic gate (Block 422), where a first component model 424 is applied to all or a portion of the WGS mapped dataset and a second component model 426 is applied to all or a portion of the targeted panel sequencing mapped dataset, or dimensionally-reduced component values thereof. As described in detail herein, in some embodiments, the model is a joint model 428, e.g., a machine learning model, that considers all or a portion of the targeted panel sequencing mapped dataset and all or a portion of the targeted panel sequencing mapped dataset, or dimensionally-reduced component values thereof, together.

The status of copy number variation 434 can then be used for variant analysis 208 and clinical report generation (*e.g.,* as described in further detail below with reference to Figure 2A). For example, referring to Block 436, the method optionally comprises matching therapies and/or clinical trials based on the copy number variation status (*e.g.,* accepted or rejected). In some embodiments, the method optionally comprises generating a patient report indicating the CNV status 438, in addition to matched therapies and/or clinical trials based on the CNV status.

Other methods for determining copy number variation status of a subject are disclosed in, *e.g.,* U.S. Patent No. 11,211,144, filed February 18, 2021. Specific embodiments and further details regarding systems and methods for determining copy number variation status are provided in following sections with reference to Figures 5A-D.

### Microsatellite Instability (MSI)

In some embodiments, analysis of aligned sequence reads, *e.g.,* in SAM or BAM format, includes analysis of the microsatellite instability status 137 of a cancer, using a microsatellite instability analysis module 154. In some embodiments, an MSI classification algorithm classifies a cancer into three categories: microsatellite instability-high (MSI-H), microsatellite stable (MSS), or microsatellite equivocal (MSE). Microsatellite instability is a clinically actionable genomic indication for cancer immunotherapy. In microsatellite instability-high (MSI-H) tumors, defects in DNA mismatch repair (MMR) can cause a hypermutated phenotype where alterations accumulate in the repetitive microsatellite regions of DNA. MSI detection is conventionally performed by subjecting tumor tissue ("solid biopsy") to clinical next-generation sequencing or specific assays, such as MMR IHC or MSI PCR.

Methods for determining the MSI status of a subject are known in the art. For example, in some embodiments, microsatellite instability analysis module 154 employs an MSI evaluation methods described in U.S. Patent Application Serial No. 16/945,588, filed July 31, 2020.

### Tumor Mutational Burden (TMB)

In some embodiments, the analysis of aligned sequence reads, *e.g.,* in SAM or BAM format, includes determination of a mutation burden for the cancer (*e.g.,* a tumor mutational burden 136), using a tumor mutational burden analysis module 155. Generally, a tumor mutational burden is a measure of the mutations in a cancer per unit of the patient's genome. For example, a tumor mutational burden may be expressed as a measure of central tendency (*e.g.,* an average) of the number of somatic variants per million base pairs in the genome. In some embodiments, a tumor mutational burden refers to only a set of possible mutations, *e.g.,* one or more of SNVs, MNVs, indels, or genomic rearrangements. In some embodiments, a tumor mutational burden refers to only a subset of one or more types of possible mutations, *e.g.,* non-synonymous mutations, meaning those mutations that alter the amino acid sequence of an encoded protein. In other embodiments, for example, a tumor mutational burden refers to the number of one or more types of mutations that occur in protein coding sequences, *e.g.,* regardless of whether they change the amino acid sequence of the encoded protein.

Methods for calculating tumor mutation burden in liquid biopsy samples and/or solid tissue samples are known in the art. See, for example, Fenizia F et al., Transl Lung Cancer Res., 7(6):668-77 (2018) and Georgiadis A et al., Clin. Cancer Res., 25(23):7024-34 (2019).

### Homologous Recombination Status (HRD)

In some embodiments, analysis of aligned sequence reads, *e.g.,* in SAM or BAM format, includes analysis of whether the cancer is homologous recombination deficient (HRD status 137-3), using a homologous recombination pathway analysis module 157.

Homologous recombination (HR) is a normal, highly conserved DNA repair process that enables the exchange of genetic information between identical or closely related DNA molecules. It is most widely used by cells to accurately repair harmful breaks *(e.g.,* damage) that occur on both strands of DNA. DNA damage may occur from exogenous (external) sources like UV light, radiation, or chemical damage; or from endogenous (internal) sources like errors in DNA replication or other cellular processes that create DNA damage. Double strand breaks are a type of DNA damage. Using poly (ADP-ribose) polymerase (PARP) inhibitors in patients with HRD compromises two pathways of DNA repair, resulting in cell death (apoptosis). The efficacy of PARP inhibitors is improved not only in ovarian cancers displaying germline or somatic BRCA mutations, but also in cancers in which HRD is caused by other underlying etiologies.

Methods for determining HR status are described in U.S. Patent Application Serial No. 16/789,363, filed February 12, 2020.

### Circulating Tumor Fraction

In some embodiments, the analysis of aligned sequence reads, *e.g.,* in SAM or BAM format, includes estimation of a circulating tumor fraction for the liquid biopsy sample. Tumor fraction or circulating tumor fraction is the fraction of cell free nucleic acid molecules in the sample that originates from a cancerous tissue of the subject, rather than from a non-cancerous tissue *(e.g.,* a germline or hematopoietic tissue). Several open source analysis packages have modules for calculating tumor fraction from solid tumor samples. For instance, PureCN (Riester, M., et al., Source Code Biol Med, 11:13 (2016)) is designed to estimate tumor purity from targeted short-read sequencing data of solid tumor samples. Similarly, FACETS (Shen R, Seshan VE, Nucleic Acids Res., 44(16):e131 (2016)) is designed to estimate tumor fraction from sequencing data of solid tumor samples. However, estimating tumor fraction from a liquid biopsy sample is more difficult because of the, generally, lower tumor fraction relative to a solid tumor sample and typically small size of a targeted panel used for liquid biopsy sequencing. Indeed, packages such as PureCN and FACETS perform poorly at low tumor fractions and with sequencing data generated using small targeted-panels.

### Quality Control

In some embodiments, a positive sensitivity control sample is processed and sequenced along with one or more clinical samples. In some embodiments, the control sample is included in at least one flow cell of a multi-flow cell reaction and is processed and sequenced each time a set of samples is sequenced or periodically throughout the course of a plurality of sets of samples. In some embodiments, the control includes a pool of controls. In some embodiments, a quality control analysis requires that read metrics of variants present in the control sample fall within acceptable criteria. In some embodiments, a quality control requires approval by a pathologist before the results are reported. Examples of criteria used for such purpose are described, for example, in WO 2021/168146.

### Variant Characterization

In some embodiments, a predicted functional effect and/or clinical interpretation for one or more identified variants is curated by using information from databases. In some embodiments, a weighted-heuristic model is used to characterize each variant.

In some embodiments, identified clinical variants are labeled as "potentially actionable," "biologically relevant," "variants of unknown significance (VUSs)," or "benign." Potentially actionable alterations are protein-altering variants with an associated therapy based on evidence from the medical literature. Biologically relevant alterations are protein-altering variants that may have functional significance or have been observed in the medical literature but are not associated with a specific therapy. Variants of unknown significance (VUSs) are protein-altering variants exhibiting an unclear effect on function and/or without sufficient evidence to determine their pathogenicity. In some embodiments, benign variants are not reported. In some embodiments, variants are identified through aligning the patient's DNA sequence to the human genome reference sequence version hg19 (GRCh37).

For instance, in some embodiments, variant classification and reporting is performed, where detected variants are investigated following criteria from known evolutionary models, functional data, clinical data, literature, and other research endeavors, including tumor organoid experiments. In some embodiments, variants are prioritized and classified based on known gene-disease relationships, hotspot regions within genes, internal and external somatic databases, primary literature, and other features of somatic drivers. Variants can be added to a patient (or sample, for example, organoid sample) report based on recommendations from the AMP/ASCO/CAP guidelines. Additional guidelines may be followed. Briefly, pathogenic variants with therapeutic, diagnostic, or prognostic significance may be prioritized in the report. Non-actionable pathogenic variants may be included as biologically relevant, followed by variants of uncertain significance. Translocations may be reported based on features of known gene fusions, relevant breakpoints, and biological relevance. Evidence may be curated from public and private databases or research and presented as 1) consensus guidelines 2) clinical research, or 3) case studies, with a link to the supporting literature. Germline alterations may be reported as secondary findings in a subset of genes for consenting patients. These may include genes recommended by the ACMG and additional genes associated with cancer predisposition or drug resistance.

In some embodiments, a clinical report 139-3 includes information about clinical trials for which the patient is eligible, therapies that are specific to the patient's disease or disorder, and/or possible therapeutic adverse effects associated with the specific characteristics of the patient's disease or disorder, *e.g.,* the patient's genetic variations, epigenetic abnormalities, associated oncogenic pathogenic infections, and/or pathology abnormalities, or other characteristics of the patient's sample and/or clinical records. For example, in some embodiments, the clinical report includes such patient information and analysis metrics, including diagnosis, patient demographic and/or institution, matched therapies (*e.g.,* FDA approved and/or investigational), matched clinical trials, variants of unknown significance (VUS), genes with low coverage, panel information, specimen information, details on reported variants, patient clinical history, status and/or availability of previous test results, and/or version of bioinformatics pipeline.

In some embodiments, the results included in the report, and/or any additional results (for example, from the bioinformatics pipeline), are used to query a database of clinical data, for example, to determine whether there is a trend showing that a particular therapy was effective or ineffective in treating (*e.g.,* slowing or halting cancer progression), and/or adverse effects of such treatments in other patients having the same or similar characteristics.

As illustrated in Figure 2A, in some embodiments, a clinical report is checked for final validation, review, and sign-off by a medical practitioner. The clinical report is then sent for action, to a clinician treating the patient.

### Example Embodiments for Determining Copy Number Variation Status.

An overview of methods for providing clinical support for personalized cancer therapy is described above with reference to Figures 1-4F above. Below, systems and methods for improving validation of copy number variation in a test subject, *e.g.,* within the context of the methods and systems described above, are described with reference to Figures 4F and 5A-D.

Many of the embodiments described below, in conjunction with Figures 4F and 5A-D, relate to analyses performed using sequencing data for nucleic acid molecules obtained from samples of a subject. Generally, these embodiments are independent and, thus, not reliant upon any particular nucleic acid sequencing methods. However, in some embodiments, the methods described below include generating the sequencing data.

In one aspect, the disclosure provides a method 500 for determining a copy number variation status of a subject. In some embodiments, the method is performed on a computer system having one or more processors and memory storing one or more programs for execution by the one or more processors.

Referring to Block 502, in some embodiments, the method includes obtaining, in electronic form, a first plurality (*e.g.,* at least 100,000) of nucleic acid sequences (135-wgs-seq) for a first plurality of DNA molecules from a first biological sample of the subject generated by whole genome sequencing (*e.g.,* at an average sequencing depth of from 0.5X to 5X across at least 90% of a reference genome for the species of the subject).

In some embodiments, the first biological sample of the subject is obtained from a solid tumor sample from the subject.

In some embodiments, the first biological sample of the subject is obtained from a liquid biopsy sample from the subject. For instance, in some embodiments, the first biological sample is a saliva sample or a blood sample.

In some embodiments, the first biological sample is a non-cancerous tissue sample from the subject. For instance, in some embodiments, the systems and methods described herein are used to inform identification of small pathogenic CNV events more accurately for Mendelian disease diagnostics, newborn screening, carrier screening, CDC Tier-1 condition screening, and other disease panels screening.

In some embodiments, the first biological sample includes, but is not limited to, any of the samples disclosed herein, as described, *e.g.,* in the section entitled "Example Workflow for Precision Oncology," above.

In some embodiments, the first plurality of nucleic acid sequences comprises at least 10,000, at least 20,000, at least 50,000, at least 100,000, at least 200,000, at least 500,000, at least 800,000, at least 1 million, at least 2 million, at least 3 million, or at least 5 million nucleic acid sequences. In some embodiments, the first plurality of nucleic acid sequences comprises no more than 10 million, no more than 5 million, no more than 2 million, no more than 1 million, no more than 500,000, no more than 200,000, no more than 100,000, or no more than 50,000 nucleic acid sequences. In some embodiments, the first plurality of nucleic acid sequences comprises from 10,000 to 500,000, from 100,000 to 1 million, from 200,000 to 2 million, from 1 million to 5 million, or from 2 million to 10 million nucleic acid sequences. In some embodiments, the first plurality of nucleic acid sequences falls within another range starting no lower than 10,000 nucleic acid sequences and ending no higher than 10 million nucleic acid sequences.

In some embodiments, the first plurality of nucleic acid sequences is at least 100,000 nucleic acid sequences. In some embodiments, the first plurality of at least 100,000 nucleic acid sequences is at least 1,000,000 sequence reads.

In some embodiments, the first plurality of nucleic acid sequences collectively provides an average sequencing depth of at least 0.1X, at least 0.2X, at least 0.5X, at least 1X, at least 2X, at least 3X, at least 4X, at least 5X, at least 10X, at least 20X, at least 30X, or at least 50X. In some embodiments, the first plurality of nucleic acid sequences collectively provides an average sequencing depth of no more than 100X, no more than 50X, no more than 30X, no more than 10X, or no more than 5X. In some embodiments, the first plurality of nucleic acid sequences collectively provides an average sequencing depth of from 0.1X to 5X, from 1X to 5X, from 2X to 10X, or from 0.5X to 30X. In some embodiments, the first plurality of nucleic acid sequences collectively provides an average sequencing depth that falls within another range starting no lower than 0.1X and ending no higher than 100X.

In some embodiments, the first plurality of nucleic acid sequences collectively maps to at least 40%, at least 50%, at least 60%, at least 70%, at least 80%, at least 85%, at least 90%, at least 95%, at least 98%, or at least 99% of a reference genome for the species of the subject. In some embodiments, the first plurality of nucleic acid sequences collectively maps to no more than 99.9%, no more than 99%, no more than 98%, no more than 95%, no more than 90%, no more than 80%, no more than 70%, or no more than 60% of a reference genome for the species of the subject. In some embodiments, the first plurality of nucleic acid sequences collectively maps to from 50% to 95%, from 70% to 99%, from 80% to 99%, or from 90% to 99.9% of a reference genome for the species of the subject. In some embodiments, the first plurality of nucleic acid sequences collectively maps to another range of the reference genome, starting no lower than 40% and ending no higher than 99.9%. In some embodiments, the first plurality of nucleic acid sequences collectively maps to the entirety (100%) of the reference genome for the species of the subject.

Accordingly, referring to Block 504, in some embodiments, the first plurality of at least 100,000 nucleic acid sequences collectively provides an average sequencing depth of from 1X to 5X across at least 90% of a reference genome for the species of the subject. In some embodiments, the first plurality of at least 100,000 nucleic acid sequences collectively provides an average sequencing depth of from 2X to 3X across at least 90% of a reference genome for the species of the subject.

Optionally, in some embodiments, the method includes performing a sequencing step to obtain the first plurality of nucleic acid sequences. In particular, in some embodiments, the method includes isolating nucleic acids from the first biological sample, generating a nucleic acid library from the isolated nucleic acids, optionally amplifying the nucleic acid library, and then sequencing the nucleic acids, as described elsewhere herein (*see, e.g.,* the section entitled "Example Workflow for Precision Oncology," above).

In some embodiments, the whole genome sequencing is low-pass whole genome sequencing (LPWGS).

Referring to Block 506, the method includes obtaining, in electronic form, a second plurality of (*e.g.,* at least 10,000) nucleic acid sequences (*e.g.,* 135-pt-seq) for a second plurality of DNA molecules from a second biological sample of the subject generated by panel-targeted sequencing.

In some embodiments, the second biological sample includes, but is not limited to, any of the samples disclosed herein, as described, *e.g.,* in the section entitled "Example Workflow for Precision Oncology," above. For example, in some embodiments, the second biological sample of the subject is obtained from a solid tumor sample from the subject. In some embodiments, the second biological sample of the subject is obtained from a liquid biopsy sample from the subject. For instance, in some embodiments, the second biological sample is a saliva sample or a blood sample. In some embodiments, the second biological sample is a non-cancerous tissue sample from the subject. For instance, in some embodiments, the systems and methods described herein are used to inform identification of small pathogenic CNV events more accurately for Mendelian disease diagnostics, newborn screening, carrier screening, CDC Tier-1 condition screening, and other disease panels screening.

In some embodiments, the first and second samples are the same sample. For example, in some implementations, the first biological sample of the subject and the second biological sample of the subject are obtained from a common single solid tumor sample from the subject. In some such implementations, the first biological sample of the subject and the second biological sample of the subject are different slices (*e.g.,* tissue sections) obtained from a single solid tissue sample.

In some embodiments, the first biological sample and the second biological sample are different samples. Various embodiments for the first biological sample and the second biological sample are disclosed, *e.g.,* in the section entitled "Additional Embodiments," below.

In some embodiments, the first biological sample of the subject and the second biological sample of the subject are non-cancerous tissue samples from the subject. In some embodiments, the first biological sample of the subject and the second biological sample of the subject are germline samples from the subject. In particular, in some embodiments, the first biological sample and the second biological sample are different germline samples from the subject (*e.g.,* a blood sample and a saliva sample). Accordingly, in some embodiments, the first biological sample and the second biological sample are independently selected from a saliva sample and a blood sample.

In some embodiments, where the first sample and the second sample are different samples, the samples are collected within a certain amount of time as each other, e.g., less than 7 days apart, less than 30 days apart, less than 2 months apart, less than 3 months apart, less than 6 months apart, less than 1 year apart, etc. This may be particularly important when the samples are cancerous samples, as cancer genomes can accumulate genomic variations more quickly than non-cancerous genomes. Similarly, this may be less important when the disease or disorder is a hereditary disorder, such that the subject's germline genome will change less over time than the genomes of cancerous tissues.

In some embodiments, the second plurality of nucleic acid sequences comprises at least 1000, at least 2000, at least 5000, at least 10,000, at least 20,000, at least 50,000, at least 80,000, at least 100,000, at least 200,000, at least 500,000, at least 1 million, or at least 2 million nucleic acid sequences. In some embodiments, the second plurality of nucleic acid sequences comprises no more than 5 million, no more than 2 million, no more than 1 million, no more than 500,000, no more than 200,000, no more than 100,000, no more than 50,000, or no more than 10,000 nucleic acid sequences. In some embodiments, the second plurality of nucleic acid sequences comprises from 1000 to 50,000, from 10,000 to 1 million, from 20,000 to 2 million, from 100,000 to 500,000, or from 200,000 to 1 million nucleic acid sequences. In some embodiments, the second plurality of nucleic acid sequences falls within another range starting no lower than 1000 nucleic acid sequences and ending no higher than 5 million nucleic acid sequences.

In some embodiments, the second plurality of nucleic acid sequences comprises at least 10,000 nucleic acid sequences. In some such embodiments, the second plurality of at least 10,000 nucleic acid sequences is at least 100,000 sequence reads. In some embodiments, the number of sequence reads in the second plurality of nucleic acid sequences is dependent upon the size of an enrichment panel used for the panel-targeted sequencing, as described in further detail below.

In some embodiments, the second plurality of nucleic acid sequences collectively provides an average sequencing depth of at least 10X, at least 20X, at least 30X, at least 50X, at least 100X, at least 200X, at least 300X, or at least 500X. In some embodiments, the second plurality of nucleic acid sequences collectively provides an average sequencing depth of no more than 1000X, no more than 500X, no more than 300X, no more than 100X, or no more than 50X. In some embodiments, the second plurality of nucleic acid sequences collectively provides an average sequencing depth of from 30X to 500X, from 10X to 100X, from 40X to 200X, or from 60X to 80X. In some embodiments, the second plurality of nucleic acid sequences collectively provides an average sequencing depth that falls within another range starting no lower than 10X and ending no higher than 1000X.

Accordingly, referring to Block 508, in some embodiments, the second plurality of at least 10,000 nucleic acid sequences collectively provides an average sequencing depth of at least 40X across the genomic regions targeted by the panel-targeted sequencing. In some embodiments, the second plurality of at least 10,000 nucleic acid sequences collectively provides an average sequencing depth of from 40X to 100X across the genomic regions targeted by the panel-targeted sequencing.

In some embodiments, the first plurality of nucleic acid sequences collectively provides an average sequencing depth of at least 0.1X, at least 0.2X, at least 0.5X, at least 1X, at least 2X, at least 3X, at least 4X, at least 5X, at least 10X, at least 20X, at least 30X, or at least 50X, and the second plurality of nucleic acid sequences collectively provides an average sequencing depth of at least 10X, at least 20X, at least 30X, at least 50X, at least 100X, at least 200X, at least 300X, or at least 500X. In some embodiments, the first plurality of nucleic acid sequences collectively provides an average sequencing depth of no more than 100X, no more than 50X, no more than 30X, no more than 10X, or no more than 5X, and the second plurality of nucleic acid sequences collectively provides an average sequencing depth of no more than 1000X, no more than 500X, no more than 300X, no more than 100X, or no more than 50X. In some embodiments, the first plurality of nucleic acid sequences collectively provides an average sequencing depth of from 0.1X to 5X, from 1X to 5X, from 2X to 10X, or from 0.5X to 30X, and the second plurality of nucleic acid sequences collectively provides an average sequencing depth of from 30X to 500X, from 10X to 100X, from 40X to 200X, or from 60X to 80X. In some embodiments, the first plurality of nucleic acid sequences collectively provides an average sequencing depth that falls within another range starting no lower than 0.1X and ending no higher than 100X, and the second plurality of nucleic acid sequences collectively provides an average sequencing depth that falls within another range starting no lower than 10X and ending no higher than 1000X.

In some embodiments, the panel-targeted sequencing is performed to determine a genomic characteristic (e.g., a single nucleotide variant (SNV), an indel, a copy number variation (CNV), a pseudogene, a CG-rich region, an AT-rich region, a genetic rearrangement, a splice variant, a gene expression level, aneuploidy, and/or chromosomal trisomy) of one or more target regions in a genome (e.g., a short genomic sequence, an exon, and intron, a plurality of contiguous exons, a plurality of contiguous exons and introns, a gene, a cluster of genes, tens to hundreds of contiguous kilobases of a chromosome, a chromosome arm, and/or an entire chromosome) of a subject.

In some embodiments, the one or more regions targeted by the panel-targeted sequencing includes a nucleotide, a portion of an intron, a portion of an exon, an intron, an exon, a subset of contiguous exons for a gene, a subset of contiguous exons and introns for a gene, a gene, a portion of a chromosome, an arm of a chromosome, and/or an entire chromosome.

For instance, in some embodiments, the panel-targeted sequencing targets a plurality of genomic regions (e.g., loci) in a genome of the subject.

In some embodiments, the plurality of genomic regions comprises at least 100 regions. In some embodiments, the plurality of genomic regions is at least 10, at least 15, at least 25, at least 30, at least 40, at least 50, at least 100, at least 200, at least 250, at least 400, at least 500, at least 600, at least 700, at least 800, at least 900, at least 1000, at least 2000, at least 2500, at least 4000, at least 5000, at least 6000, at least 7000, at least 8000, at least 9000, at least 10,000, at least 15,000, or at least 20,000 regions. In some embodiments, the plurality of genomic regions is no more than 30,000, no more than 20,000, no more than 10,000, no more than 8000, no more than 7500, no more than 5000, no more than 4000, no more than 3000, no more than 2000, no more than 1000, no more than 750, no more than 500, no more than 250, no more than 100, no more than 50, or no more than 25 regions. In some embodiments, the plurality of genomic regions is from 10 to 50, from 25 to 100, from 100 to 500, from 100 to 1000, from 1000 to 2000, from 10 to 500, from 500 to 2000, from 1000 to 5000, from 5000 to 10,000, or from 10,000 to 20,000 regions. In some embodiments, the plurality of genomic regions is from 10 to 100,000 regions, from 100 to 100,000 regions, from 1000 to 100,000 regions, from 5000 to 100,000 regions, from 10,000 to 100,000 regions, or from 50,000 to 100,000 regions. In some embodiments, the plurality of genomic regions is from 10 to 50,000 regions, from 100 to 50,000 regions, from 1000 to 50,000 regions, from 5000 to 50,000 regions, or from 10,000 to 50,000 regions. In some embodiments, the plurality of genomic regions is from 10 to 30,000 regions, from 100 to 30,000 regions, from 1000 to 30,000 regions, from 5000 to 30,000 regions, or from 10,000 to 30,000 regions. In some embodiments, the plurality of genomic regions is from 10 to 10,000 regions, from 100 to 10,000 regions, from 1000 to 10,000 regions, or from 5000 to 10,000 regions. In some embodiments, the plurality of genomic regions is from 10 to 1000 regions, from 100 to 1000 regions, or from 500 to 1000 regions. In some embodiments, the plurality of genomic regions falls within another range starting no lower than 10 regions and ending no higher than 30,000 regions.

In some embodiments, a genomic region in the plurality of genomic regions is a gene. In some embodiments, each genomic region in the plurality of genomic regions is a gene. Accordingly, in some embodiments, the panel-targeted sequencing targets a plurality of genes. In some such embodiments, the panel-targeted sequencing targets at least 25 genes. In some embodiments, the panel-targeted sequencing targets a plurality of genes selected from Table 1, List 1, and/or List 2, as described above (see, e.g., the section entitled "Example Workflow for Precision Oncology," above). In some embodiments, the panel-targeted sequencing targets at least 10, at least 15, at least 20, at least 25, at least 30, at least 50, at least 75, or at least 100 genes selected from Table 1, List 1, and/or List 2. In some embodiments, the panel-targeted sequencing targets all of the genes selected from Table 1. In some embodiments, the panel-targeted sequencing targets all of the genes selected from List 1. In some embodiments, the panel-targeted sequencing targets all of the genes selected from List 2.

In some embodiments, the plurality of genes includes one or more genes selected from the group consisting of BRCA1, BRCA2, a CYP gene, CYP2D, a PMS2 pseudogene, a PMSCL pseudogene, DMD, MET, TP53, ALK, IGF1, TLR9, FLT3, and a TCR/BCR gene.

In some embodiments, the plurality of genomic regions includes a whole exome. In some embodiments, the plurality of genomic regions includes a whole human exome.

In some embodiments, the panel-targeted sequencing targets one or more chromosomes of the subject. In some such embodiments, the panel-targeted sequencing targets a portion of a chromosome, an arm of a chromosome, or an entire chromosome. In some embodiments, the plurality of genomic regions includes all, or substantially all (*e.g*., at least 98%, at least 99%, at least 99.5%, or at least 99.9%), of a chromosomal arm. For example, in some embodiments, an entire chromosomal arm is targeted by the panel-targeted sequencing except for one or more complex genomic regions, such as a telomere, telomeric region, kinetochore, kinetochoric region, large nucleotide repeat, and the like. In some embodiments, the plurality of genomic regions includes all, or substantially all (*e.g.*, at least 98%, at least 99%, at least 99.5%, or at least 99.9%), of a chromosome. For example, in some embodiments, an entire chromosome is targeted by the panel-targeted sequencing except for one or more complex genomic regions, such as a telomere, telomeric region, kinetochore, kinetochoric region, large nucleotide repeat, and the like. In some embodiments, the plurality of genomic regions includes all, or substantially all (*e.g*., at least 98%, at least 99%, at least 99.5%, or at least 99.9%), of a plurality of chromosomes. In some embodiments, the plurality of chromosomes comprises at least 2, at least 3, at least 4, at least 5, at least 6, at least 7, at least 8, at least 9, at least 10, at least 11, at least 12, at least 13, at least 20, at least 30, or at least 40 chromosomes. In some embodiments, the plurality of chromosomes comprises no more than 50, no more than 40, no more than 30, no more than 20, no more than 10, or no more than 5 chromosomes. In some embodiments, the plurality of chromosomes comprises from 2 to 5, from 3 to 10, from 2 to 20, from 10 to 50, or another range of chromosomes starting no lower than 2 chromosomes and ending no higher than 50 chromosomes.

In some embodiments, the plurality of genomic regions includes all, or substantially all, of a genome.

Optionally, in some embodiments, the method includes performing a sequencing step to obtain the second plurality of nucleic acid sequences. For instance, in some embodiments, the method includes capturing targeted nucleic acids using a plurality of probes. In particular, in some embodiments, the method includes isolating nucleic acids from the second biological sample, generating a nucleic acid library from the isolated nucleic acids, optionally amplifying the nucleic acid library, capturing targeted nucleic acids using a probe set, optionally amplifying the captured nucleic acids, and then sequencing the amplified nucleic acids, as described elsewhere herein (see, *e.g*., the section entitled "Example Workflow for Precision Oncology," above). Any suitable embodiment for a respective probe in the plurality of probes is contemplated for use in the present disclosure, as described in further herein (see, *e.g*., the section entitled "Example Workflow for Precision Oncology," above).

For instance, in some embodiments, the plurality of probes comprises a plurality of nucleic acid probe species. Each respective nucleic acid probe species (*e.g*., all nucleic acid probes that align to the same subsequence of a respective target region) in the plurality of nucleic acid probe species aligns to a different subsequence of a respective target region of a reference construct for the species of the subject. For instance, in some embodiments, a first respective set of nucleic acid probes tiles (*e.g*., via overlapping or non-overlapping tiling) a respective genomic region, such as a gene. Thus, the nucleic acid probes in the set of probes bind to different subsequences of the genomic region.

As used herein, a "nucleic acid probe species" refers to all nucleic acid probes in a composition that align to the same or substantially the same genomic sequence (*e.g*., the first 150 nucleotides of a particular exon of a gene). Generally, all probes of a particular nucleic acid probe species will have the same nucleotide sequence. However, in some embodiments, a particular probe of nucleic acid probe species may have one or a small number of nucleotide variations relative to other probes within the nucleic acid probe species. Regardless, two probes that differ by one or a small number of nucleotide variants still belong to the same nucleic acid probe species because they align to the same position in the genome. Similarly, it can be envisioned that, in some embodiments, a probe in a particular nucleic acid probe species may be one or a small number of nucleotides longer or shorter than other probes in the particular nucleic acid probe species. Furthermore, it can be envisioned that, in some embodiments, a probe in a particular nucleic acid probe species may be shifted by one or a small number of nucleotides relative to the sequence of other probes in the particular nucleic acid probe species. In addition, probes in a particular nucleic acid probe species may be differently conjugated to a chemical moiety.

In some embodiments, the plurality of nucleic acid probe species comprises at least 100, at least 500, at least 1000, at least 2000, at least 5000, at least 10,000, at least 50,000, at least 100,000, at least 500,000, at least 1,000,000, at least 2,500,000, or at least 5,000,000 nucleic acid probe species. In some embodiments, the plurality of nucleic acid probe species is no more than 10,000,000, no more than 1,000,000, no more than 500,000, no more than 100,000, no more than 50,000, no more than 10,000, no more than 5000, no more than 1000, or no more than 500 nucleic acid probe species. In some embodiments, the plurality of nucleic acid probe species is from 100 to 500, from 250 to 1000, from 1000 to 5000, from 1000 to 10,000,000, from 1,000,000 to 10,000,000, from 100 to 5,000,000, or from 100,000 to 500,000 nucleic acid probe species. In some embodiments, the plurality of nucleic acid probe species falls within another range starting no lower than 100 nucleic acid probe species and ending no higher than 10,000,000 nucleic acid probe species.

Additional embodiments for probes suitable for use in the present disclosure are further described in U.S. Patent Application Serial No. 17/076,704, filed October 21, 2020, and U.S. Provisional Patent Application Serial No. 63/177,811, filed April 21, 2021.

In some embodiments, the panel-targeted sequencing is used for various diagnostic applications, *e.g*., to perform sequencing using an optimized probe set suitable for a specific patient or for a particular assay (*e.g*., to assay for a mutation, specific cancer type, or other disease). For example, in some implementations, panel-targeted sequencing is used to inform methodologies for characterizing an immune repertoire; monitoring immune response, autoimmune disease, cancer progression, minimal residual disease (MRD), and/or immunotherapy treatment; designing novel immunotherapies; and/or predicting susceptibility to various infectious diseases.

In various embodiments, the panel-targeted sequencing utilizes multi-use probes capable of achieving similar sensitivity of targets across various applications (*e.g*., solid tumor versus liquid biopsy, or targeted panel versus whole exome or whole genome).

In various embodiments, the panel-targeted sequencing facilitates the more accurate detection of single nucleotide variants (SNVs), small INDELs, large INDELs, CNVs, pseudogenes, GC/AT-rich regions of the genome, genetic rearrangements, splice variants, gene expression levels, aneuploidy, trisomy, and/or other possible conclusions based on genetic sequencing results. In various embodiments, the panel-targeted sequencing facilitates genetic analysis of genetic regions of interest of varying sizes, including point locations, small regions or elements, individual exon or intron, multiple exons or multiple introns, entire gene, partial chromosome, and/or whole chromosome. In various embodiments, the panel-targeted sequencing is utilized for genetic sequencing in one or more of the fields of oncology/somatic, germline, infectious or parasitic disease, microbiome, and/or other areas of human healthcare.

Referring to Block 510, in some embodiments, the panel-targeted sequencing is whole exome sequencing.

Referring to Block 512, the method further includes obtaining a first mapped dataset (*e.g*., 135-wgs-md) by a process comprising mapping the first plurality of nucleic acid sequences (*e.g*., 135-wgs-seq) to positions within a reference genome for the species of the subject.

In some embodiments, the obtaining the first mapped dataset includes performing an alignment, as disclosed elsewhere herein (*see, e.g*., the sections entitled "Bioinformatics Module," "Example Workflow for Precision Oncology," and "Copy Number Variation," above).

In some embodiments, the first mapped dataset comprises a first plurality of mapped nucleic acid sequences 408. In some embodiments, the first mapped dataset comprises a first plurality of bin values determined from the first plurality of mapped nucleic acid sequences. In some embodiments, the first mapped dataset comprises a first plurality of copy number states determined from the first plurality of mapped nucleic acid sequences. In some embodiments, the first mapped dataset comprises a first plurality of dimension reduction component values, *e.g*., component values generated from a first plurality of bin values and/or from a first plurality of copy number states.

For instance, referring to Block 514, the obtaining the first mapped dataset (*e.g*., 135-wgs-md) further comprises determining a respective first bin value (*e.g*., 135-wgs-bv) for each respective bin in a first plurality of bins, where each respective bin in the first plurality of bins represents a unique segment of the reference genome, and each respective first bin value is a measure of the number of nucleic acid sequences (*e.g*., 135-wgs-seq) in the first plurality of nucleic acid sequences that were mapped to the unique segment of the reference genome corresponding to the respective bin in the first plurality of bins. In some such embodiments, the all or the portion of the first mapped dataset inputted into the model (as described below) comprises the respective bin value for each respective bin in the first plurality of bins.

In some embodiments, the first plurality of bins comprises at least 1000 bins.

In some embodiments, the first plurality of bins comprises at least 100, at least 1000, at least 2000, at least 5000, at least 10,000, at least 20,000, at least 50,000, at least 80,000, at least 100,000, at least 200,000, at least 500,000, at least 1 million, or at least 2 million bins. In some embodiments, the first plurality of bins comprises no more than 5 million, no more than 2 million, no more than 1 million, no more than 500,000, no more than 200,000, no more than 100,000, no more than 50,000, or no more than 10,000 bins. In some embodiments, the first plurality of bins comprises from 1000 to 50,000, from 10,000 to 1 million, from 20,000 to 2 million, from 100,000 to 500,000, or from 200,000 to 1 million bins. In some embodiments, the first plurality of bins falls within another range starting no lower than 100 bins and ending no higher than 5 million bins.

In some embodiments, the first plurality of bins collectively represents at least 10 kb of the reference genome.

In some embodiments, the first plurality of bins collectively represents at least 1, at least 10, at least 50, at least 100, at least 1000, at least 2000, at least 5000, at least 10,000, at least 20,000, at least 50,000, at least 80,000, at least 100,000, at least 200,000, at least 500,000, or at least 1 million kb. In some embodiments, the first plurality of bins collectively represents no more than 3 million, no more than 1 million, no more than 500,000, no more than 200,000, no more than 100,000, no more than 50,000, no more than 10,000, no more than 1000, or no more than 100 kb. In some embodiments, the first plurality of bins collectively represents from 100 to 5000, from 1000 to 1 million, from 20,000 to 2 million, from 100,000 to 500,000, or from 200,000 to 1 million kb. In some embodiments, the first plurality of bins collectively represents another portion of the reference genome that falls within another range starting no lower than 1 kb and ending no higher than 3 million kb.

In some embodiments, each respective bin in the first plurality of bins corresponds to no more than 1 kb of the reference genome.

In some embodiments, each respective bin in the first plurality of bins corresponds to no more than 1000, no more than 500, no more than 300, no more than 200, no more than 100, no more than 50, no more than 10, no more than 5, no more than 1, or no more than 0.5 kb of the reference genome. In some embodiments, each respective bin in the first plurality of bins corresponds to at least 0.1, at least 0.5, at least 1, at least 10, at least 50, at least 100, or at least 500 kb. In some embodiments, each respective bin in the first plurality of bins corresponds to from 0.1 to 1, from 0.5 to 100, from 0.2 to 10, from 0.5 to 50, or from 0.1 to 500 kb. In some embodiments, each respective bin in the first plurality of bins corresponds to another range of the reference genome starting no lower than 0.1 kb and ending no higher than 1000 kb.

In some embodiments, the corresponding first bin value for a respective bin in the first plurality of bins is the number (*e.g*., count) of nucleic acid sequences in the first plurality of nucleic acid sequences that were mapped to the unique segment of the reference genome corresponding to the respective bin.

In some embodiments, the corresponding first bin value for a respective bin in the first plurality of bins is a normalized or standardized number of nucleic acid sequences in the first plurality of nucleic acid sequences that were mapped to the unique segment of the reference genome corresponding to the respective bin.

For instance, in some embodiments, the corresponding first bin value for a respective bin in the first plurality of bins is normalized for GC content of the respective bin, across some or all of the first plurality of bins. In some embodiments, the corresponding first bin value for a respective bin in the first plurality of bins is normalized for the size of the respective bin, across some or all of the first plurality of bins.

In some embodiments, the corresponding first bin value for a respective bin in the first plurality of bins is standardized relative to some or all (*e.g*., the total number) of nucleic acid sequences in the first plurality of nucleic acid sequences. In some such embodiments, the corresponding first bin value for a respective bin in the first plurality of bins is a measure of central tendency (*e.g*., a mean, median, mode, a weighted mean, weighted median, weighted mode, *etc*.) for the number of nucleic acid sequences assigned to some or all of the first plurality of bins. In some embodiments, the corresponding first bin value for a respective bin in the first plurality of bins is a measure of central tendency for the number of nucleic acids mapping to a reference region of the reference genome, where the reference region of the reference genome includes some or all of the first plurality of bins.

In some embodiments, the method further comprises determining a measure of dispersion (*e.g*., variance, standard deviation, standard error, *etc*.) for some or all of nucleic acid sequences in the first plurality of nucleic acid sequences.

Referring to Block 516, in some embodiments, the obtaining the first mapped dataset (*e.g*., 135-wgs-md) further comprises determining a respective first bin value (*e.g.*, 135-wgs-bv) for each respective bin in a first plurality of bins, where each respective bin in the first plurality of bins represents a unique segment of the reference genome, and each respective first bin value is a measure of the number of nucleic acid sequences (*e.g*., 135-wgs-seq) in the first plurality of nucleic acid sequences that were mapped to the unique segment of the reference genome corresponding to the respective bin in the first plurality of bins. In some such embodiments, the obtaining the first mapped dataset further includes determining a respective copy number state (*e.g.,* 135-wgs-cns) for each respective bin in the first plurality of bins using the respective first bin value for the respective bin. In some such embodiments, the all or the portion of the first mapped dataset inputted into the model (as described below) comprises the respective copy number state for each respective bin in the first plurality of bins.

In some such embodiments, the determining the respective first bin value for each respective bin in a first plurality of bins includes any of the embodiments disclosed above. In some embodiments, the determining the respective copy number state for each respective bin in the first plurality of bins using the respective first bin value for the respective bin comprises any of the embodiments disclosed herein (*see, e.g*., the sections entitled "Bioinformatics Module" and "Example Workflow for Precision Oncology: Copy Number Variation," above).

Referring to Block 518, the method further includes obtaining a second mapped dataset (*e.g*., 135-pt-md) by a process comprising mapping the second plurality of nucleic acid sequences (*e.g*., 135-pt-seq) to positions within a reference construct for a plurality of genomic regions targeted by the panel-targeted sequencing.

In some embodiments, the obtaining the second mapped dataset includes performing an alignment, as disclosed elsewhere herein (*see, e.g*., the sections entitled "Bioinformatics Module," "Example Workflow for Precision Oncology," and "Copy Number Variation," above).

In some embodiments, the second mapped dataset comprises a second plurality of mapped nucleic acid sequences 408. In some embodiments, the second mapped dataset comprises a second plurality of bin values determined from the second plurality of mapped nucleic acid sequences. In some embodiments, the second mapped dataset comprises a second plurality of copy number states determined from the second plurality of mapped nucleic acid sequences. In some embodiments, the second mapped dataset comprises a second plurality of dimension reduction component values, *e.g*., component values generated from a second plurality of bin values and/or from a second plurality of copy number states.

For instance, referring to Block 520, in some embodiments, the obtaining the second mapped dataset (*e.g*., 135-pt-md) further comprises determining a respective second bin value (*e.g*., 135-pt-bv) for each respective bin in a second plurality of bins, where each respective bin in the second plurality of bins represents a unique segment of the reference construct, and each respective second bin value is a measure of the number of nucleic acid sequences (*e.g.*, 135-pt-seq) in the first plurality of nucleic acid sequences that were mapped to the unique segment of the reference genome corresponding to the respective bin in the first plurality of bins. In some such embodiments, the all or the portion of the second mapped dataset inputted into the model (as described below) comprises the respective bin value for each respective bin in the second plurality of bins.

In some embodiments, the second plurality of bins comprises at least 1000 bins.

In some embodiments, the second plurality of bins comprises at least 100, at least 1000, at least 2000, at least 5000, at least 10,000, at least 20,000, at least 50,000, at least 80,000, at least 100,000, at least 200,000, at least 500,000, at least 1 million, or at least 2 million bins. In some embodiments, the second plurality of bins comprises no more than 5 million, no more than 2 million, no more than 1 million, no more than 500,000, no more than 200,000, no more than 100,000, no more than 50,000, or no more than 10,000 bins. In some embodiments, the second plurality of bins comprises from 1000 to 50,000, from 10,000 to 1 million, from 20,000 to 2 million, from 100,000 to 500,000, or from 200,000 to 1 million bins. In some embodiments, the second plurality of bins falls within another range starting no lower than 100 bins and ending no higher than 5 million bins.

In some embodiments, the second plurality of bins collectively represents at least 10 kb of the reference construct.

In some embodiments, the second plurality of bins collectively represents at least 1, at least 10, at least 50, at least 100, at least 1000, at least 2000, at least 5000, at least 10,000, at least 20,000, at least 50,000, at least 80,000, at least 100,000, at least 200,000, at least 500,000, or at least 1 million kb of the reference construct. In some embodiments, the second plurality of bins collectively represents no more than 3 million, no more than 1 million, no more than 500,000, no more than 200,000, no more than 100,000, no more than 50,000, no more than 10,000, no more than 1000, or no more than 100 kb of the reference construct. In some embodiments, the second plurality of bins collectively represents from 100 to 5000, from 1000 to 1 million, from 20,000 to 2 million, from 100,000 to 500,000, or from 200,000 to 1 million kb of the reference construct. In some embodiments, the second plurality of bins collectively represents another portion of the reference construct that falls within another range starting no lower than 1 kb and ending no higher than 3 million kb.

In some embodiments, each respective bin in the second plurality of bins corresponds to no more than 1 kb of the reference construct.

In some embodiments, each respective bin in the second plurality of bins corresponds to no more than 1000, no more than 500, no more than 300, no more than 200, no more than 100, no more than 50, no more than 10, no more than 5, no more than 1, or no more than 0.5 kb of the reference construct. In some embodiments, each respective bin in the second plurality of bins corresponds to at least 0.1, at least 0.5, at least 1, at least 10, at least 50, at least 100, or at least 500 kb of the reference construct. In some embodiments, each respective bin in the second plurality of bins corresponds to from 0.1 to 1, from 0.5 to 100, from 0.2 to 10, from 0.5 to 50, or from 0.1 to 500 kb of the reference construct. In some embodiments, each respective bin in the second plurality of bins corresponds to another range of the reference construct starting no lower than 0.1 kb and ending no higher than 1000 kb.

In some implementations, the corresponding second bin value for a respective bin in the second plurality of bins is the number (*e.g*., count) of nucleic acid sequences in the second plurality of nucleic acid sequences that were mapped to the unique segment of the reference construct corresponding to the respective bin. In some implementations, the corresponding second bin value for a respective bin in the second plurality of bins is a normalized or standardized number of nucleic acid sequences in the second plurality of nucleic acid sequences that were mapped to the unique segment of the reference construct corresponding to the respective bin.

For instance, in some embodiments, the corresponding second bin value for a respective bin in the second plurality of bins is normalized for GC content of the respective bin, across some or all of the second plurality of bins. In some embodiments, the corresponding second bin value for a respective bin in the second plurality of bins is normalized for the size of the respective bin, across some or all of the second plurality of bins. In some embodiments, the corresponding second bin value for a respective bin in the second plurality of bins is standardized relative to some or all (*e.g.*, the total number) of nucleic acid sequences in the second plurality of nucleic acid sequences. In some such embodiments, the corresponding second bin value for a respective bin in the second plurality of bins is a measure of central tendency (*e.g.*, a mean, median, mode, a weighted mean, weighted median, weighted mode, *etc*.) for the number of nucleic acid sequences assigned to some or all of the second plurality of bins. In some embodiments, the corresponding second bin value for a respective bin in the second plurality of bins is a measure of central tendency for the number of nucleic acids mapping to a reference region of the reference construct, where the reference region of the reference construct includes some or all of the second plurality of bins. In some embodiments, the method further comprises determining a measure of dispersion (*e.g*., variance, standard deviation, standard error, *etc*.) for some or all of nucleic acid sequences in the second plurality of nucleic acid sequences.

Referring to Block 522, in some embodiments, the obtaining the second mapped dataset (*e.g*., 135-pt-md) further comprises determining a respective second bin value (*e.g*., 135-pt-bv) for each respective bin in a second plurality of bins, where each respective bin in the second plurality of bins represents a unique segment of the reference construct, and each respective second bin value is a measure of the number of nucleic acid sequences (*e.g*., 135-pt-seq) in the first plurality of nucleic acid sequences that were mapped to the unique segment of the reference genome corresponding to the respective bin in the first plurality of bins. In some such embodiments, the obtaining the second mapped dataset further includes determining a respective copy number state (*e.g*., 135-pt-cns) for each respective bin in the second plurality of bins using the respective second bin value for the respective bin. In some such embodiments, the all or the portion of the second mapped dataset inputted into the model (as described below) comprises the respective copy number state for each respective bin in the second plurality of bins.

In some such embodiments, the determining the respective second bin value for each respective bin in a second plurality of bins includes any of the embodiments disclosed above. In some embodiments, the determining the respective copy number state for each respective bin in the second plurality of bins using the respective second bin value for the respective bin comprises any of the embodiments disclosed herein (*see, e.g*., the sections entitled "Bioinformatics Module" and "Example Workflow for Precision Oncology: Copy Number Variation," above).

In some embodiments, the form of the first mapped dataset and the form of the second mapped dataset are selected independently of each other, *e.g*., the first dataset and the second dataset can have different forms (*e.g*., a plurality of bin values, a plurality of copy number states, and/or a plurality of dimensionality reduction components thereof). In some embodiments, the form of the first mapped dataset and the form of the second mapped dataset are of the same form (*e.g*., a plurality of bin values, a plurality of copy number states, and/or a plurality of dimensionality reduction components thereof). Various embodiments for the first mapped dataset and the second mapped dataset are disclosed, *e.g*., in the section entitled "Additional Embodiments," below.

In some embodiments, the portion of the first mapped dataset collectively represents respective sequencing depths, present in the first plurality of nucleic acid sequences, for at least 10 kb of the reference genome. In some embodiments, the portion of the first mapped dataset collectively represents respective sequencing depths, present in the first plurality of nucleic acid sequences, for at least 1, at least 10, at least 50, at least 100, at least 1000, at least 2000, at least 5000, at least 10,000, at least 20,000, at least 50,000, at least 80,000, at least 100,000, at least 200,000, at least 500,000, or at least 1 million kb of the reference genome. In some embodiments, the portion of the first mapped dataset collectively represents respective sequencing depths, present in the first plurality of nucleic acid sequences, for no more than 3 million, no more than 1 million, no more than 500,000, no more than 200,000, no more than 100,000, no more than 50,000, no more than 10,000, no more than 1000, or no more than 100 kb of the reference genome. In some embodiments, the portion of the first mapped dataset collectively represents respective sequencing depths, present in the first plurality of nucleic acid sequences, that span from 100 to 5000, from 1000 to 1 million, from 20,000 to 2 million, from 100,000 to 500,000, or from 200,000 to 1 million kb of the reference genome. In some embodiments, the portion of the first mapped dataset collectively represents respective sequencing depths, present in the first plurality of nucleic acid sequences, that span another range starting no lower than 1 kb and ending no higher than 3 million kb of the reference genome.

In some embodiments, the portion of the second mapped dataset collectively represents respective sequencing depths, present in the second plurality of nucleic acid sequences, for at least 10 kb of the reference construct. In some embodiments, the portion of the second mapped dataset collectively represents respective sequencing depths, present in the second plurality of nucleic acid sequences, for at least 1, at least 10, at least 50, at least 100, at least 1000, at least 2000, at least 5000, at least 10,000, at least 20,000, at least 50,000, at least 80,000, at least 100,000, at least 200,000, at least 500,000, or at least 1 million kb of the reference construct. In some embodiments, the portion of the second mapped dataset collectively represents respective sequencing depths, present in the second plurality of nucleic acid sequences, for no more than 3 million, no more than 1 million, no more than 500,000, no more than 200,000, no more than 100,000, no more than 50,000, no more than 10,000, no more than 1000, or no more than 100 kb of the reference construct. In some embodiments, the portion of the second mapped dataset collectively represents respective sequencing depths, present in the second plurality of nucleic acid sequences, that span from 100 to 5000, from 1000 to 1 million, from 20,000 to 2 million, from 100,000 to 500,000, or from 200,000 to 1 million kb of the reference construct. In some embodiments, the portion of the second mapped dataset collectively represents respective sequencing depths, present in the second plurality of nucleic acid sequences, that span another range starting no lower than 1 kb and ending no higher than 3 million kb of the reference construct.

Accordingly, in some embodiments, the portion of the first mapped dataset collectively represents respective sequencing depths, present in the first plurality of nucleic acid sequences, for at least 10 kb of the reference genome, and the portion of the second mapped dataset collectively represents respective sequencing depths, present in the second plurality of nucleic acid sequences, for at least 10 kb of the reference construct.

Referring to Block 524, the method includes applying a model to (i) all or a portion of the first mapped dataset (*e.g*., 135-wgs-md) and (ii) all or a portion of the second mapped dataset (*e.g*., 135-pt-md), or a plurality of dimensionality reduction components thereof, thereby identifying one or more copy number variations, as output of the model that indicate the copy number variation status of the subject.

For example, referring to Block 526, in some implementations, the method further comprises applying a dimensionality reduction technique to (i) all or a portion of the first mapped dataset or (ii) all or a portion of the second mapped dataset, thereby generating the plurality of dimensionality reduction components, and the applying comprises applying the plurality of dimensionality reduction components to the model.

A variety of dimensionality reduction techniques can be used. Examples include, but are not limited to, principal component analysis (PCA), non-negative matrix factorization (NMF), linear discriminant analysis (LDA), diffusion maps, or network (*e.g.*, neural network) techniques such as an autoencoder.

In some embodiments, the dimension reduction is a principal components algorithm, a random projection algorithm, an independent component analysis algorithm, a feature selection method, a factor analysis algorithm, Sammon mapping, curvilinear components analysis, a stochastic neighbor embedding (SNE) algorithm, an Isomap algorithm, a maximum variance unfolding algorithm, a locally linear embedding algorithm, a t-SNE algorithm, a non-negative matrix factorization algorithm, a kernel principal component analysis algorithm, a graph-based kernel principal component analysis algorithm, a linear discriminant analysis algorithm, a generalized discriminant analysis algorithm, a uniform manifold approximation and projection (UMAP) algorithm, a LargeVis algorithm, a Laplacian Eigenmap algorithm, or a Fisher's linear discriminant analysis algorithm. See, for example, Fodor, 2002, "A survey of dimension reduction techniques," Center for Applied Scientific Computing, Lawrence Livermore National, Technical Report UCRL-ID-148494; Cunningham, 2007, "Dimension Reduction," University College Dublin, Technical Report UCD-CSI-2007-7, Zahorian et al., 2011, "Nonlinear Dimensionality Reduction Methods for Use with Automatic Speech Recognition," Speech Technologies. doi:10.5772/16863. ISBN 978-953-307-996-7; and Lakshmi et al., 2016, "2016 IEEE 6th International Conference on Advanced Computing (IACC)," pp. 31-34. doi:10.1109/IACC.2016.16, ISBN 978-1-4673-8286-1. Accordingly, in some embodiments, the dimension reduction is a principal component analysis (PCA) algorithm, and each respective extracted dimension reduction component comprises a respective principal component derived by the PCA. In such embodiments, the number of principal components in the plurality of principal components can be limited to a threshold number of principal components calculated by the PCA algorithm. The threshold number of principal components can be, for example, at least 5, at least 10, at least 20, at least 50, at least 100, at least 1000, at least 1500, or any other number.

In some embodiments, the method further includes performing manifold learning using the (i) all or a portion of the first mapped dataset and/or the (ii) all or a portion of the second mapped dataset. Generally, manifold learning is used to describe the low-dimensional structure of high-dimensional data by determining maximal variations in a dataset. Examples include, but are not limited to, force-directed layout (*see, e.g*., Fruchterman, T. M., & Reingold, E. M. (1991). Graph drawing by force-directed placement. Software: Practice and experience, 21(11), 1129-1164) (*e.g*., Force Atlas 2), t-distributed stochastic neighbor embedding (t-SNE), locally linear embedding (see, *e.g*., Roweis, S. T., & Saul, L. K. (2000). Nonlinear dimensionality reduction by locally linear embedding. Science, 290(5500), 2323-2326), local linear isometric mapping (ISOMAP; *see, e.g*., Tenenbaum, J. B., De Silva, V., & Langford, J. C. (2000). A global geometric framework for nonlinear dimensionality reduction. Science, 290(5500), 2319-2323), kernel PCA, graph-based kernel PCA, Potential of Heat-Diffusion for Affinity Based Trajectory Embedding (PHATE), generalized discriminant analysis (GDA), Uniform Manifold Approximation and Projection (UMAP), or kernel discriminant analysis. In some embodiments, the method includes performing discriminant analysis. Force-directed layouts are useful in various particular embodiments because of their ability to identify new, lower dimensions that encode non-linear aspects of the underlying data which arise from underlying relationships between data elements. Force directed layouts use physics-based models as mechanisms for determining a reduced dimensionality that best represents the data. As an example, a force directed layout uses a form of physics simulation in which, in this embodiment, each input element in the first and/or second mapped datasets is assigned a "repulsion" force and there exists a global "gravitation force" that, when computed over the plurality of elements, identifies sectors of the data that "diffuse" together under these competing "forces." Force directed layouts make few assumptions about the structure of the data, and do not impose a de-noising approach. Manifold learning is further described, for example, in Wang et al., 2004, "Adaptive Manifold Learning," Advances in Neural Information Processing Systems 17.

In some embodiments, the model comprises a plurality of at least 500 parameters.

In some embodiments, the plurality of parameters comprises at least 10, at least 50, at least 100, at least 500, at least 1000, at least 2000, at least 5000, at least 10,000, at least 20,000, at least 50,000, at least 100,000, at least 200,000, at least 500,000, at least 1 million, at least 2 million, at least 3 million, at least 4 million or at least 5 million parameters. In some embodiments, the plurality of parameters comprises no more than 8 million, no more than 5 million, no more than 4 million, no more than 1 million, no more than 500,000, no more than 100,000, no more than 50,000, no more than 10,000, no more than 5000, no more than 1000, or no more than 500 parameters. In some embodiments, the plurality of parameters comprises from 10 to 5000, from 500 to 10,000, from 10,000 to 500,000, from 20,000 to 1 million, or from 1 million to 5 million parameters. In some embodiments, the plurality of parameters falls within another range starting no lower than 10 parameters and ending no higher than 8 million parameters.

Referring to Block 528, in some embodiments, the model comprises a first component model and a second component model, where the first component model provides a first respective copy number state for a respective genomic region of the one or more respective genomic regions upon input to the first component model of all or a portion of the first mapped dataset, and the second component model provides a second respective copy number state for the respective genomic region of the one or more respective genomic regions upon input to the second component model of all or a portion of the second mapped dataset. When both (i) the first respective copy number state and (ii) the second respective copy number state indicates the presence of a copy number variation at the respective genomic region, the copy number variation at the respective genomic region is accepted. When either (i) the first respective copy number state or (ii) the second respective copy number state does not indicate the presence of a copy number variation at the respective genomic region, the copy number variation at the respective genomic region is rejected.

In some embodiments, the first component model or the second component model is a statistical inference model. In some embodiments, the first component model or the second component model is a machine-learning model. In some embodiments, the first component model or the second component model comprises any suitable model disclosed herein, and/or any combinations, modifications, substitutions, additions, or deletions thereof as will be apparent to one skilled in the art (*see, e.g*., the section entitled, "Definitions: Classifier," above).

In some embodiments, the component first model indicates the presence of a copy number variation with a sensitivity of at least 90% and a specificity of no more than 90% when applied to data from a plurality of subjects comprising a first cohort population that includes subjects without copy number variations at the respective genomic region and a second cohort population that includes subjects with copy number variation at the respective genomic region.

In some embodiments, the component first model indicates the presence of a copy number variation with a sensitivity of at least 40%, at least 50%, at least 60%, at least 70%, at least 80%, at least 90%, or at least 95% and a specificity of no more than 99%, no more than 95%, no more than 90%, no more than 80%, no more than 70%, or no more than 60%, when applied to data from a plurality of subjects comprising a first cohort population that includes subjects without copy number variations at the respective genomic region and a second cohort population that includes subjects with copy number variation at the respective genomic region. In some embodiments, the component first model indicates the presence of a copy number variation with a sensitivity of from 50% to 95% and a specificity of 40% to 80% when applied to data from a plurality of subjects comprising a first cohort population that includes subjects without copy number variations at the respective genomic region and a second cohort population that includes subjects with copy number variation at the respective genomic region. In some embodiments, the component first model indicates the presence of a copy number variation with a sensitivity of from 70% to 99% and a specificity of 60% to 90% when applied to data from a plurality of subjects comprising a first cohort population that includes subjects without copy number variations at the respective genomic region and a second cohort population that includes subjects with copy number variation at the respective genomic region.

In some embodiments, the component second model indicates the presence of a copy number variation with a sensitivity of at least 90% and a specificity of no more than 90% when applied to data from a plurality of subjects comprising a first cohort population that includes subjects without copy number variations at the respective genomic region and a second cohort population that includes subjects with copy number variation at the respective genomic region.

In some embodiments, the component second model indicates the presence of a copy number variation with a sensitivity of at least 40%, at least 50%, at least 60%, at least 70%, at least 80%, at least 90%, or at least 95% and a specificity of no more than 99%, no more than 95%, no more than 90%, no more than 80%, no more than 70%, or no more than 60%, when applied to data from a plurality of subjects comprising a first cohort population that includes subjects without copy number variations at the respective genomic region and a second cohort population that includes subjects with copy number variation at the respective genomic region. In some embodiments, the component second model indicates the presence of a copy number variation with a sensitivity of from 50% to 95% and a specificity of 40% to 80% when applied to data from a plurality of subjects comprising a first cohort population that includes subjects without copy number variations at the respective genomic region and a second cohort population that includes subjects with copy number variation at the respective genomic region. In some embodiments, the component second model indicates the presence of a copy number variation with a sensitivity of from 70% to 99% and a specificity of 60% to 90% when applied to data from a plurality of subjects comprising a first cohort population that includes subjects without copy number variations at the respective genomic region and a second cohort population that includes subjects with copy number variation at the respective genomic region.

In some embodiments, the first cohort comprises at least 10, at least 50, at least 100, at least 500, at least 1000, at least 2000, at least 5000, at least 10,000, at least 20,000, at least 50,000, at least 100,000, at least 200,000, at least 500,000, or at least 1 million subjects. In some embodiments, the first cohort comprises no more than 5 million, no more than 1 million, no more than 500,000, no more than 100,000, no more than 50,000, no more than 10,000, no more than 5000, no more than 1000, or no more than 500 subjects. In some embodiments, the first cohort comprises from 10 to 5000, from 500 to 10,000, from 10,000 to 500,000, from 20,000 to 1 million, or from 1 million to 5 million subjects. In some embodiments, the first cohort falls within another range starting no lower than 10 subjects and ending no higher than 5 million subjects.

In some embodiments, the second cohort comprises at least 10, at least 50, at least 100, at least 500, at least 1000, at least 2000, at least 5000, at least 10,000, at least 20,000, at least 50,000, at least 100,000, at least 200,000, at least 500,000, or at least 1 million subjects. In some embodiments, the second cohort comprises no more than 5 million, no more than 1 million, no more than 500,000, no more than 100,000, no more than 50,000, no more than 10,000, no more than 5000, no more than 1000, or no more than 500 subjects. In some embodiments, the second cohort comprises from 10 to 5000, from 500 to 10,000, from 10,000 to 500,000, from 20,000 to 1 million, or from 1 million to 5 million subjects. In some embodiments, the second cohort falls within another range starting no lower than 10 subjects and ending no higher than 5 million subjects.

Referring to Block 530, in some embodiments, the model comprises a machine-learning model using (i) all or a portion of the first mapped dataset and (ii) all or a portion of the second mapped dataset as inputs. In some embodiments, the machine-learning model is a support vector regression, a random forest model, an XGBoost model, a Gaussian process model, a deep neural network model, a convolutional neural network model, or a recurrent neural network model. In some embodiments, the machine learning model comprises any suitable machine learning model disclosed herein, and/or any combinations, modifications, substitutions, additions, or deletions thereof as will be apparent to one skilled in the art (*see, e.g*., the section entitled, "Definitions: Classifier," above).

Referring to Block 532, in some embodiments, the model determines the copy number variation status of the genome of the tissue of the subject through a statistical inference. In some embodiments, the statistical inference is a Bayesian inference, a likelihood-based inference, frequentist inference, or an AIC-based inference. In some embodiments, the statistical inference comprises any suitable statistical inference model disclosed herein, and/or any combinations, modifications, substitutions, additions, or deletions thereof as will be apparent to one skilled in the art (*see, e.g*., the section entitled, "Definitions: Classifier," above).

In some embodiments, the model comprises a probabilistic network (*e.g*., a Bayesian classifier and/or a joint Bayesian network). In some embodiments, the probabilistic network comprises any suitable probabilistic network model disclosed herein, and/or any combinations, modifications, substitutions, additions, or deletions thereof as will be apparent to one skilled in the art (*see, e.g*., the section entitled, "Definitions: Classifier," above).

In some embodiments, the model is a statistical inference model, the method further comprises applying a dimensionality reduction technique to (i) all or a portion of the first mapped dataset or (ii) all or a portion of the second mapped dataset, thereby generating the plurality of dimensionality reduction components, and the applying comprises applying the plurality of dimensionality reduction components to the model. In some implementations, the dimensionality reduction technique is principal component analysis, and the statistical inference model is a Bayesian model. Dimensionality reduction techniques suitable for use in the present disclosure are further described elsewhere herein (*see, e.g*., the foregoing description and the section entitled "Definitions: Classifier," above).

In some embodiments, the model processes the (i) all or the portion of the first mapped dataset and (ii) all or the portion of the second mapped dataset, or the plurality of dimensionality reduction components thereof, to identify the one or more copy number variations as output of the model in N-dimensional space in the applying, wherein N is a positive integer of 4 or greater. In some embodiments, N is 5, 6, 7, 8, 9, 10, 11, 12, 13, 14, 15, 20, 50, 100, 1000, 10,000, 100,000, 500,000, 1 x 10⁶, 5 x 10⁶, 1 x 10⁷, or greater.

Referring to Block 534, in some embodiments, the method further comprises, when the model identifies a copy number variation at a respective genomic region, validating the copy number variation using an orthogonal validation technique. In some embodiments, the orthogonal validation technique is selected from the group consisting of multiplex ligation-dependent probe amplification, quantitative PCR analysis, and long-read nucleic acid sequencing.

### Digital and Laboratory Health Care Platform

In some embodiments, the methods and systems described herein are utilized in combination with, or as part of, a digital and laboratory health care platform that is generally targeted to medical care and research. It should be understood that many uses of the methods and systems described above, in combination with such a platform, are possible. One example of such a platform is described in U.S. Patent Publication No. 2021/0090694, titled "Data Based Cancer Research and Treatment Systems and Methods", and published March 25, 2021.

For example, an implementation of one or more embodiments of the methods and systems as described above may include microservices constituting a digital and laboratory health care platform supporting analysis of cancer biopsy samples to provide clinical support for personalized cancer therapy. Embodiments may include a single microservice for executing and delivering analysis of cancer biopsy samples to clinical support for personalized cancer therapy or may include a plurality of microservices each having a particular role, which together implement one or more of the embodiments above. In one example, a first microservice may execute sequence analysis in order to deliver genomic features to a second microservice for curating clinical support for personalized cancer therapy based on the identified features. Similarly, the second microservice may execute therapeutic analysis of the curated clinical support to deliver recommended therapeutic modalities, according to various embodiments described herein.

Where embodiments above are executed in one or more micro-services with or as part of a digital and laboratory health care platform, one or more of such micro-services may be part of an order management system that orchestrates the sequence of events as needed at the appropriate time and in the appropriate order necessary to instantiate embodiments above. A microservices-based order management system is disclosed, for example, in U.S. Patent Publication No. 2020/80365232, titled "Adaptive Order Fulfillment and Tracking Methods and Systems", and published November 19, 2020.

For example, continuing with the above first and second microservices, an order management system may notify the first microservice that an order for curating clinical support for personalized cancer therapy has been received and is ready for processing. The first microservice may execute and notify the order management system once the delivery of genomic features for the patient is ready for the second microservice. Furthermore, the order management system may identify that execution parameters (prerequisites) for the second microservice are satisfied, including that the first microservice has completed, and notify the second microservice that it may continue processing the order to curate clinical support for personalized cancer therapy, according to various embodiments described herein.

Where the digital and laboratory health care platform further includes a genetic analyzer system, the genetic analyzer system may include targeted panels and/or sequencing probes. An example of a targeted panel is disclosed, for example, in U.S. Patent Publication No. 2021/0090694, titled "Data Based Cancer Research and Treatment Systems and Methods", and published March 25, 2021. An example of a targeted panel for sequencing cell-free (cf) DNA and determining various characteristics of a specimen based on the sequencing is disclosed, for example, in U.S. Patent Application No. 17/179,086, titled "Methods And Systems For Dynamic Variant Thresholding In A Liquid Biopsy Assay", and filed 2/18/21, U.S. Patent Application No. 17/179,267, titled "Estimation Of Circulating Tumor Fraction Using Off-Target Reads Of Targeted-Panel Sequencing", and filed 2/18/21, and U.S. Patent Application No. 17/179,279, titled "Methods And Systems For Refining Copy Number Variation In A Liquid Biopsy Assay", and filed 2/18/21. In one example, targeted panels may enable the delivery of next generation sequencing results for providing clinical support for personalized cancer therapy according to various embodiments described herein. An example of the design of next-generation sequencing probes is disclosed, for example, in U.S. Patent Publication No. 2021/0115511, titled "Systems and Methods for Next Generation Sequencing Uniform Probe Design", and published June 22, 2021 and U.S. Patent Application No. 17/323,986, titled "Systems and Methods for Next Generation Sequencing Uniform Probe Design", and filed May 18, 2021.

Where the digital and laboratory health care platform further includes an epigenetic analyzer system, the epigenetic analyzer system may analyze specimens to determine their epigenetic characteristics and may further use that information for monitoring a patient over time. An example of an epigenetic analyzer system is disclosed, for example, in U.S. Patent Application No. 17/352,231, titled "Molecular Response And Progression Detection From Circulating Cell Free DNA", and filed 6/18/21.

Where the digital and laboratory health care platform further includes a bioinformatics pipeline, the methods and systems described above may be utilized after completion or substantial completion of the systems and methods utilized in the bioinformatics pipeline. As one example, the bioinformatics pipeline may receive next-generation genetic sequencing results and return a set of binary files, such as one or more BAM files, reflecting nucleic acid (*e.g*., cfDNA, DNA and/or RNA) read counts aligned to a reference genome. The methods and systems described above may be utilized, for example, to ingest the cfDNA, DNA and/or RNA read counts and produce genomic features as a result.

When the digital and laboratory health care platform further includes an RNA data normalizer, any RNA read counts may be normalized before processing embodiments as described above. An example of an RNA data normalizer is disclosed, for example, in Publication No. 2020/0098448, titled "Methods of Normalizing and Correcting RNA Expression Data", and published March 26, 2020.

When the digital and laboratory health care platform further includes a genetic data deconvolver, any system and method for deconvoluting may be utilized for analyzing genetic data associated with a specimen having two or more biological components to determine the contribution of each component to the genetic data and/or determine what genetic data would be associated with any component of the specimen if it were purified. An example of a genetic data deconvolver is disclosed, for example, in U.S. Patent Publication No. 2020/0210852, published July 2, 2020, and PCT/US19/69161, filed December 31, 2019, both titled "Transcriptome Deconvolution of Metastatic Tissue Samples"; and U.S. Patent Application No. 17/074,984, titled "Calculating Cell-type RNA Profiles for Diagnosis and Treatment", and filed October 20, 2020.

When the digital and laboratory health care platform further includes an automated RNA expression caller, RNA expression levels may be adjusted to be expressed as a value relative to a reference expression level, which is often done in order to prepare multiple RNA expression data sets for analysis to avoid artifacts caused when the data sets have differences because they have not been generated by using the same methods, equipment, and/or reagents. An example of an automated RNA expression caller is disclosed, for example, in U.S. Patent No. 11,043,283, titled "Systems and Methods for Automating RNA Expression Calls in a Cancer Prediction Pipeline", and issued June 22, 2021.

RNA expression levels may be adjusted to be expressed as a value relative to a reference expression level. Furthermore, multiple RNA expression data sets may be adjusted, prepared, and/or combined for analysis and may be adjusted to avoid artifacts caused when the data sets have differences because they have not been generated by using the same methods, equipment, and/or reagents. An example of RNA data set adjustment, preparation, and/or combination is disclosed, for example, in U.S. Patent Application No. 17/405,025, titled "Systems and Methods for Homogenization of Disparate Datasets", and filed August 18, 2021.

The digital and laboratory health care platform may further include one or more insight engines to deliver information, characteristics, or determinations related to a disease state that may be based on genetic and/or clinical data associated with a patient and/or specimen. Exemplary insight engines may include a tumor of unknown origin engine, a human leukocyte antigen (HLA) loss of homozygosity (LOH) engine, a tumor mutational burden engine, a PD-L1 status engine, a homologous recombination deficiency engine, a cellular pathway activation report engine, an immune infiltration engine, a microsatellite instability engine, a pathogen infection status engine, a T cell receptor or B cell receptor profiling engine, a line of therapy engine, a metastatic prediction engine, an IO progression risk prediction engine, and so forth. An example tumor of unknown origin engine is disclosed, for example, in U.S. Patent Application No. 15/930,234, titled "Systems and Methods for Multi-Label Cancer Classification", and filed May 12, 2020. An example of an HLA LOH engine is disclosed, for example, in U.S. Patent No. 11,081,210, titled "Detection of Human Leukocyte Antigen Class I Loss of Heterozygosity in Solid Tumor Types by NGS DNA Sequencing", and issued August 3, 2021. An additional example of an HLA LOH engine is disclosed, for example, in U.S. Patent App. No. 17/304,940, titled "Detection of Human Leukocyte Antigen Loss of Heterozygosity", and filed June 28, 2021. An example of a tumor mutational burden (TMB) engine is disclosed, for example, in U.S. Patent Publication No. 2020/0258601, titled "Targeted-Panel Tumor Mutational Burden Calculation Systems and Methods", and published August 13, 2020. An example of a PD-L1 status engine is disclosed, for example, in U.S. Patent Publication No. 2020/0395097, titled "A Pan-Cancer Model to Predict The PD-L1 Status of a Cancer Cell Sample Using RNA Expression Data and Other Patient Data", and published December 17, 2020. An additional example of a PD-L1 status engine is disclosed, for example, in U.S. Patent No. 10,957,041, titled "Determining Biomarkers from Histopathology Slide Images", issued March 23, 2021. An example of a homologous recombination deficiency engine is disclosed, for example, in U.S. Patent No. 10,975,445, titled "An Integrative Machine-Learning Framework to Predict Homologous Recombination Deficiency", and issued April 13, 2021. An additional example of a homologous recombination deficiency engine is disclosed, for example, in U.S. Patent App. No. 17/492,518, titled "Systems and Methods for Predicting Homologous Recombination Deficiency Status of a Specimen", filed October 1, 2021. An example of a cellular pathway activation report engine is disclosed, for example, in U.S. Patent Publication No. 2021/0057042, titled "Systems And Methods For Detecting Cellular Pathway Dysregulation In Cancer Specimens", and published February 25, 2021. An example of an immune infiltration engine is disclosed, for example, in U.S. Patent Publication No. 2020/0075169, titled "A Multi-Modal Approach to Predicting Immune Infiltration Based on Integrated RNA Expression and Imaging Features", and published March 5, 2020. An example of an MSI engine is disclosed, for example, in U.S. Patent Publication No. 2020/0118644, titled "Microsatellite Instability Determination System and Related Methods", and published April 16, 2020. An additional example of an MSI engine is disclosed, for example, in U.S. Patent Publication No. 2021/0098078, titled "Systems and Methods for Detecting Microsatellite Instability of a Cancer Using a Liquid Biopsy", and published April 1, 2021. An example of a pathogen infection status engine is disclosed, for example, in U.S. Patent No. 11,043,304, titled "Systems And Methods For Using Sequencing Data For Pathogen Detection", and issued June 22, 2021. Another example of a pathogen infection status engine is disclosed, for example, in PCT/US21/18619, titled "Systems And Methods For Detecting Viral DNA From Sequencing", and filed February 18, 2021, which is incorporated herein by reference and in its entirety for all purposes. An example of a T cell receptor or B cell receptor profiling engine is disclosed, for example, in U.S. Patent Application No. 17/302,030, titled "TCR/BCR Profiling Using Enrichment with Pools of Capture Probes", and filed April 21, 2021. An example of a line of therapy engine is disclosed, for example, in U.S. Patent Publication No. 2021/0057071, titled "Unsupervised Learning And Prediction Of Lines Of Therapy From High-Dimensional Longitudinal Medications Data", and published February 25, 2021. An example of a metastatic prediction engine is disclosed, for example, in U.S. Patent No. 11,145,416, titled "Predicting likelihood and site of metastasis from patient records", and issued October 12, 2021. An example of an IO progression risk prediction engine is disclosed, for example, in U.S. Patent Application No. 17/455,876, titled "Determination of Cytotoxic Gene Signature and Associated Systems and Methods For Response Prediction and Treatment", and filed November 19, 2021.

Any data generated by the systems and methods and/or the digital and laboratory health care platform may be downloaded by the user. In one example, the data may be downloaded as a CSV file comprising clinical and/or molecular data associated with tests, data structuring, and/or other services ordered by the user. In various embodiments, this may be accomplished by aggregating clinical data in a system backend, and making it available via a portal. This data may include not only variants and RNA expression data, but also data associated with immunotherapy markers such as MSI and TMB, as well as RNA fusions.

When the digital and laboratory health care platform further includes a device comprising a microphone and speaker for receiving audible queries or instructions from a user and delivering answers or other information, the methods and systems described above may be utilized to add data to a database the device can access. An example of such a device is disclosed, for example, in U.S. Patent Publication No. 2020/0335102, titled "Collaborative Artificial Intelligence Method And System", and published October 22, 2020.

When the digital and laboratory health care platform further includes a mobile application for ingesting patient records, including genomic sequencing records and/or results even if they were not generated by the same digital and laboratory health care platform, the methods and systems described above may be utilized to receive ingested patient records. An example of such a mobile application is disclosed, for example, in U.S. Patent No. 10,395,772, titled "Mobile Supplementation, Extraction, And Analysis Of Health Records", and issued August 27, 2019. Another example of such a mobile application is disclosed, for example, in U.S. Patent No. 10,902,952, titled "Mobile Supplementation, Extraction, And Analysis Of Health Records", and issued January 26, 2021. Another example of such a mobile application is disclosed, for example, in U.S. Patent Publication No. 2021/0151192, titled "Mobile Supplementation, Extraction, And Analysis Of Health Records", and filed May 20, 2021.

When the digital and laboratory health care platform further includes a report generation engine, the methods and systems described above may be utilized to create a summary report of a patient's genetic profile and the results of one or more insight engines for presentation to a physician. For instance, the report may provide to the physician information about the extent to which the specimen that was sequenced contained tumor or normal tissue from a first organ, a second organ, a third organ, and so forth. For example, the report may provide a genetic profile for each of the tissue types, tumors, or organs in the specimen. The genetic profile may represent genetic sequences present in the tissue type, tumor, or organ and may include variants, expression levels, information about gene products, or other information that could be derived from genetic analysis of a tissue, tumor, or organ.

The report may include therapies and/or clinical trials matched based on a portion or all of the genetic profile or insight engine findings and summaries. For example, the therapies may be matched according to the systems and methods disclosed in U.S. Patent Application No. 17/546,049, titled "Artificial Intelligence Driven Therapy Curation and Prioritization", filed December 9, 2021. For example, the clinical trials may be matched according to the systems and methods disclosed in U.S. Patent Publication No. 2020/0381087, titled "Systems and Methods of Clinical Trial Evaluation", published December 3, 2020.

The report may include a comparison of the results (for example, molecular and/or clinical patient data) to a database of results from many specimens. An example of methods and systems for comparing results to a database of results are disclosed in U.S. Patent Publication No. 2020/0135303 titled "User Interface, System, And Method For Cohort Analysis" and published April 30, 2020, and U.S. Patent Publication No. 2020/0211716 titled "A Method and Process for Predicting and Analyzing Patient Cohort Response, Progression and Survival", and published July 2, 2020. The information may be used, sometimes in conjunction with similar information from additional specimens and/or clinical response information, to match therapies likely to be successful in treating a patient, discover biomarkers or design a clinical trial.

When the digital and laboratory health care platform further includes organoids developed in connection with the platform (for example, from the patient specimen), the methods and systems may be used to further evaluate genetic sequencing data derived from an organoid and/or the organoid sensitivity, especially to therapies matched based on a portion or all of the information determined by the systems and methods, including predicted cancer type(s), likely tumor origin(s), etc. These therapies may be tested on the organoid, derivatives of that organoid, and/or similar organoids to determine an organoid's sensitivity to those therapies. Any of the results may be included in a report. If the organoid is associated with a patient specimen, any of the results may be included in a report associated with that patient and/or delivered to the patient or patient's physician or clinician. In various examples, organoids may be cultured and tested according to the systems and methods disclosed in U.S. Patent Publication No. 2021/0155989, titled "Tumor Organoid Culture Compositions, Systems, and Methods", published May 27, 2021; PCT/US20/56930, titled "Systems and Methods for Predicting Therapeutic Sensitivity", filed 10/22/2020; U.S. Patent Publication No. 2021/0172931, titled "Large Scale Organoid Analysis", published June 10, 2021; PCT/US2020/063619, titled "Systems and Methods for High Throughput Drug Screening", filed 12/7/2020 and U.S. Patent Application No. 17/301,975, titled "Artificial Fluorescent Image Systems and Methods", filed 4/20/2021. In one example, the drug sensitivity assays may be especially informative if the systems and methods return results that match with a variety of therapies, or multiple results (for example, multiple equally or similarly likely cancer types or tumor origins), each matching with at least one therapy.

When the digital and laboratory health care platform further includes application of one or more of the above in combination with or as part of a medical device or a laboratory developed test that is generally targeted to medical care and research, such laboratory developed test or medical device results may be enhanced and personalized through the use of artificial intelligence. An example of laboratory developed tests, especially those that may be enhanced by artificial intelligence, is disclosed, for example, in U.S. Patent Publication No. 2021/0118559, titled "Artificial Intelligence Assisted Precision Medicine Enhancements to Standardized Laboratory Diagnostic Testing", and published April 22, 2021.

It should be understood that the examples given above are illustrative and do not limit the uses of the systems and methods described herein in combination with a digital and laboratory health care platform.

The results of the bioinformatics pipeline may be provided for report generation 208. Report generation may comprise variant science analysis, including the interpretation of variants (including somatic and germline variants as applicable) for pathogenic and biological significance. The variant science analysis may also estimate microsatellite instability (MSI) or tumor mutational burden. Targeted treatments may be identified based on gene, variant, and cancer type, for further consideration and review by the ordering physician. In some aspects, clinical trials may be identified for which the patient may be eligible, based on mutations, cancer type, and/or clinical history. Subsequent validation may occur, after which the report may be finalized for sign-out and delivery. In some embodiments, a first or second report may include additional data provided through a clinical dataflow 202, such as patient progress notes, pathology reports, imaging reports, and other relevant documents. Such clinical data is ingested, reviewed, and abstracted based on a predefined set of curation rules. The clinical data is then populated into the patient's clinical history timeline for report generation.

Further details on clinical report generation are disclosed in US Patent Application No. 16/789,363 (PCT/US20/180002), filed February 12, 2020.

### EXAMPLES.

### Example 1 - Copy Number Calling using Low-Pass Whole Genome Sequencing (LPWGS)

Calling copy number events spanning one or two exons from targeted sequencing has proven difficult. Typical CNV callers work by constructing bins along the genome and comparing the observed coverage to either a control sample or reference panel. To avoid false positives, callers require two or more contiguous bins to exhibit inflated or depressed coverage before making a call. However, if the bin size is made too small, the volatility in the coverage signal leads to many false positives.

Since individual exons are quite short on average (for example, the mean length of the regions targeted by IDT xGen probes is 173 nucleotides), this makes calling single exon events problematic. Existing approaches to solve such problems include restricting calling to events spanning multiple exons while ignoring all shorter events; making use of the full capture regions of targeted sequencing, thus allowing slightly wider regions; making use of off target mappings (anti-targets), albeit with variable success that is highly dependent on the reason for off target mappings; or filtering the calls with a machine learning model (*e.g*., DECoNT) trained for the purpose of distinguishing good (for example, accurate) calls from bad (for example, inaccurate).

The present example investigates the possibility of using low-pass whole genome sequencing (LPWGS) as a way to augment calling from targeted sequencing. More specifically, the example assesses whether it is possible to extract a copy number signal from 1X, 3X, and 5X WGS samples. As will be discussed below, in some embodiments, it is possible to combine this sample with a targeted sequencing signal.

*Truth Set.* To evaluate performance, samples with known events were obtained. Although truth sets for CNVs are still rather limited in the art, the nine 1000 Genomes samples characterized by Chaisson *et al.* (2019) were used for a starting truth set (hereinafter, the "Chaisson samples"). *See, e.g*., Chaisson et al., 2019, "Multi-platform discovery of haplotype-resolved structural variation in human genomes," Nature Communications 10, 1784; doi: 10.1038/s41467-018-08148-z. These germline calls are best described for the Genome Reference Consortium Human 38 (GRCh38), such that the GRCh38 build was used as the basis of the following experiments (*e.g*., for assembly, *etc*.).

The events of the Chaisson samples (including all types of structural variations) are summarized below in Table 2. Excluding the "Total" column, all the other columns are restricted to duplication (DUP) and/or deletion (DEL) copy number events. Combined totals for duplications and deletions are shown in the DEL/DUP column and further stratified by CNV length in the following three columns (1 kbp or greater, 10 kbp or greater, and 100 kbp or greater).

**Table 2. Structural variations of the Chaisson samples.**

| Sample | Total | DEL/DUP | >=1K | >=10K | >=100K | >=1K DUP | >=1K DEL |
|---|---|---|---|---|---|---|---|
| HG00512 | 13827 | 7465 | 1688 | 222 | 19 | 484 | 1204 |
| HG00513 | 14137 | 7684 | 1687 | 219 | 20 | 510 | 1177 |
| HG00514 | 39861 | 13076 | 2193 | 343 | 48 | 494 | 1699 |
| HG00731 | 13953 | 7687 | 1770 | 226 | 21 | 496 | 1274 |
| HG00732 | 14212 | 7708 | 1630 | 213 | 23 | 480 | 1150 |
| HG00733 | 41185 | 12960 | 2075 | 329 | 44 | 488 | 1587 |
| NA19238 | 16419 | 8951 | 1838 | 237 | 25 | 509 | 1329 |
| NA19239 | 15732 | 8564 | 1844 | 230 | 22 | 497 | 1347 |
| NA19240 | 45591 | 15429 | 2510 | 364 | 46 | 503 | 2007 |

The CNV events listed in Table 2 were considered to be "baseline" events. Table 3 further presents the intersection of these baseline events with xGen target regions obtained from an exome research panel.

**Table 3. Intersection of truth set CNV events with example exome target regions.**

| Sample | >=1K | >=10K | >=100K | >=1K DUP | >=1K DEL |
|---|---|---|---|---|---|
| HG00512 | 257 | 96 | 10 | 105 | 152 |
| HG00513 | 254 | 88 | 13 | 107 | 147 |
| HG00514 | 292 | 127 | 24 | 104 | 188 |
| HG00731 | 264 | 93 | 12 | 106 | 158 |
| HG00732 | 272 | 97 | 16 | 105 | 167 |
| HG00733 | 290 | 119 | 27 | 105 | 185 |
| NA19238 | 277 | 96 | 15 | 110 | 167 |
| NA19239 | 270 | 92 | 14 | 107 | 163 |
| NA19240 | 309 | 124 | 28 | 108 | 201 |

Figure 13A shows the frequency with which baseline CNV events in the Chaisson samples overlap a given number of exons. As expected, the majority of events have no overlap with exons at all, and events overlapping many exons are comparatively rare. Similar profiles were observed for all of the Chaisson samples, and thus amalgamated counts from all nine samples were used to plot various views of CNV event frequency relative to CNV length in Figures 13B-D.

For example, Figure 13B illustrates a comparison between the frequency of all CNV events in the Chaisson samples to those overlapping one or more exons, relative to CNV length. As expected, in the absence of selection pressure, these two curves are similar. In Figure 13B, length is indicated in kilobase pairs (knt), such that lengths of 1000-1999 nt correspond to 1 on the x-axis, lengths of 2000-2999 nt correspond to 2, and so on. Figure 13C illustrates events which overlap exactly 1, 2, or 3 exons. It can be seen that for the 1-exon events that there are a comparatively high number of short events. Without being limited to any one theory of operation, in some instances, such observations can be explained because to overlap 2 or 3 exons, the event must be long enough to at least cover the exons concerned. In Figure 13C, length is indicated in kilobase pairs (kb), such that lengths of 1000-1999 nt correspond to 1 on the x-axis, lengths of 2000-2999 nt correspond to 2, and so on. Figure 13D provides a cumulative view showing the count of all CNV events by CNV length, including those less than 1 kb.

*Simulated LPWGS.* Low-pass WGS sequencing of the Chaisson samples was simulated by subsampling nominally 30X WGS samples downloaded from the EBI data portal as CRAM files. In addition to the Chaisson samples, a further 11 samples (NA06985, NA07000, NA10847, NA12878, NA18501, NA19005, NA19307, HG00190, HG00421, HG04098, HG04216) were obtained to form the basis of reference normal panels. The CRAM files were converted back to BAM using samtools view and the actual coverage determined with RealTimeGenomics (RTG) software for coverage detection (RTG coverage). RTG sammerge was then used to construct subsamples with 1X, 3X, and 5X coverage. The resulting BAMs were verified as having the expected coverage using RTG coverage.

*Copy Number Calling.* CNVkit and the RTG segment were used as CNV callers. Both these callers operate in a similar manner forming a plurality of bins along the genome and using those bins as the basis for detecting duplications and deletions. The details of the "segmentation" differs between the two tools, but in both cases the resolution of events depends on the size of bins chosen. Both tools were run requiring at least two consecutive bins of similar character before an event was called. In some instances, to capture events down to a length of 1 knt, a bin size of at least 500 nt is needed, but such small bins may be problematic especially in a low-coverage environment where the coverage will be erratic. Experiments were thus conducted using bin sizes of 100,000, 10,000, 7500, 5000, 2500, 1000, and 500. Larger bin sizes are likely to have more reliable coverage numbers but may overlook smaller events. Both callers were used in "panel of normals" mode. In each case, appropriate panels were constructed from the previously listed 11 normal samples subject to the same coverage and bin sizes. A panel built from the samples at full 30X coverage were also constructed for a bin size of 500.

With two callers, 7 bin sizes, and 9 samples, a total of 126 calling runs were used to perform the basic parameter sweep. Optimization was also performed by repeating these runs multiple times with slight changes in parameters and inputs. The runs were applied to the entire genome. Runs were completed using the resources of RTG in New Zealand and additional resources.

The runtime for RTG segment ranged from seconds to 11 hours depending on bin size, with the smallest bin sizes taking the longest time. For instance, at small bin sizes there are a large number of bins along the full genome, causing segmentation to run slowly. For CNVkit, most runs completed in under an hour. In both cases, similar segmentation runs were also performed for each sample contributing to the panel of normals.

*Evaluation.* In some implementations, evaluation of copy number calls is more difficult than evaluation of small variants. For example, a first issue (*e.g*., a "locus problem") arises from the limited availability of truth sets for CNV variants. As a result, precise breakpoints for copies are often unavailable. Further, when a binning segmentation strategy is used, the resolution of position is limited by the positions of the bins themselves. Often any kind of overlap between a baseline and called variant is considered to be a match. This approach can be especially problematic if a call spans an entire arm or chromosome, as under this interpretation any baseline variant on that same chromosome would be considered a match. From a clinical perspective, it might be sufficient to ensure that appropriate exons or genes are resolved as copy number variants. For the evaluation of callers, a finer grained matching taking account of the size of the overlap between a match may be more appropriate.

A second issue (*e.g*., a "level problem") arises when a copy number call is made, but the level of the call (*e.g*., a deletion or n-fold duplication) differs. Generally, this is more of a problem for somatic events where the value of n can reach into the hundreds. In some instances, such as for germline events, it is generally sufficient to check the direction (deletion or duplication) of the match.

A third issue (*e.g*., a "multiplicity problem") occurs when a single baseline event matches multiple calls or when a single call matches multiple baseline events.

With reference to Figures 14A-17B, the 197769 target regions targeted by the xGen exome research panel were considered for evaluation. In some instances, evaluation was performed by accepting as correct (up to direction of event) any overlap with an expected event. For evaluation, no minimum depth threshold was applied, such that, for each respective bin in the plurality of bins, a respective caller allowed any count in the bin to pass into the segmentation.

Figure 14A shows CNV calling using RTG segment, using a bin size of 500 bp and simulated coverage of 1X. CNV calls made by RTG segment are presented as true positives and false positives, with the percentage of truth set calls shown on the right-hand y-axis. As illustrated in Figures 14B-C, increasing the coverage to 3X and 5X resulted in a modest reduction in false positives, but sensitivity remained poor.

CNV calling using CNVkit identified a low number of calls, with correspondingly worse sensitivity compared to RTG segment (data not shown). Furthermore, running RTG segment on the samples at full 30X coverage did not substantially improve performance and, in fact, resulted in even lower overall sensitivity compared to the low coverage runs of 1X-5X (data not shown). Stratifying the results by baseline CNV events revealed that a large proportion of baseline events were poorly called or not called at all (*e.g.*, as measured as a percentage of 0% to 100% indicating that the baseline event was called in none of the overlapping target regions to every overlapping target region). For instance, at 1X coverage and a bin size of 500, a large proportion of events were not called at all, whereas increasing coverage to 3X and 5X further decreased performance such that the number of uncalled events encompassed a majority of the events (data not shown). Increasing bin size also reduced performance, likely because larger bins prevent resolution of small events (data not shown).

Figures 15A-B provide plots of CNV calls obtained from RTG segment at positions corresponding to specific baseline events determined using the truth set. Shaded regions of the plot indicate locations of nominal deletions, and CNV calls were determined at varying simulated and full coverages (1X to 30X). While Figure 15A illustrates a deletion event that is discernible in the 3X to 30X coverage range, in the majority of cases, no signal was generated for corresponding baseline events even at 30X coverage. For instance, Figure 15B shows an example of a deletion event that is not discernible in any of the simulated or full coverages.

Similarly, as illustrated in Figure 16A, the majority of duplication events were not detected using RTG segment even at 30X coverage. The shaded region of the plot indicates the location of a nominal duplication, as provided by the truth set. Nevertheless, Figure 16B illustrates that, in some cases, duplication events could be discerned at a coverage range of 3X to 30X.

Another independent set of runs was obtained for five of the nine Chaisson samples using the CNVnator CNV caller (*see, e.g*., Ozden et al., "Polishing Copy Number Variant Calls on Exome Sequencing Data via Deep Learning," bioRxiv 2020.05.09.086082; doi: 10.1101/2020.05.09.086082). A comparison of these calls with the Chiasson truth set, illustrated in Figure 17A, shows that CNV calls made by CNVnator correlate poorly with the truth set. For instance, only 20% of the CNV events from the truth set are accurately called using CNVnator. However, as illustrated in Figure 17B, moderate concordance was observed between the CNVnator calls and the RTG segment calls at 30X coverage.

*Conclusions.* In general, CNV calling using LPWGS data failed to exhibit good concordance with the truth set, even at 5X coverage samples. While the fact that the 30X samples and the CNVnator results also failed to show good concordance strongly suggests that part of the problem is the truth set itself, ultimately, WGS sequencing data at simulated low coverages (*e.g*., 1X-5X) failed to generate sensitive and specific calling of CNV events. These results were consistently observed even across multiple CNV calling tools (RTG segment, CNVkit, and CNVnator). The results highlight the need for improved systems and methods for determining CNV calls in cases where the available data includes low-pass WGS data (*e.g*., for reasons of cost effectiveness, practicability, and feasibility, as discussed in the Introduction section, above). However, plots of specific believable events do still show some signal in at least the 3X and 5X coverage samples, as shown, for instance, in Figures 15A-B and 16A-B. These are strong enough to be discerned by the human eye, and thus should be amenable for use in an algorithm.

It has been found that the vast majority of single exon events are so short that they will not extend far into the surrounding introns. In combination with the above data, it is questionable as to whether low-pass sequencing can, in isolation, be used to detect short CNVs. However, in some embodiments, adjacent bins with similar coverage are combined during the segmentation step of CNV calling methods. In some instances, these bins correspond to exons with large gaps existing between them. In such instances, CNV callers such as CNVkit use larger "anti-target" bins to fill these gaps. The signal in these bins tends to be very weak and sporadic as they arise from regions not specifically targeted during sequencing. Thus, as disclosed herein, low-pass whole genome sequencing can be used to directly augment the targeted sequencing data, thereby obtaining more reliable coverage in the anti-target bins.

### ALTERNATIVE EMBODIMENTS

Another aspect of the present disclosure provides a computer system comprising one or more processors, and a non-transitory computer-readable medium including computer-executable instructions that, when executed by the one or more processors, cause the processors to perform a method according to any one of the embodiments disclosed herein, and/or any combinations, modifications, substitutions, additions, or deletions thereof as will be apparent to one skilled in the art.

Another aspect of the present disclosure provides a non-transitory computer-readable storage medium having stored thereon program code instructions that, when executed by a processor, cause the processor to perform the method according to any one of the embodiments disclosed herein, and/or any combinations, modifications, substitutions, additions, or deletions thereof as will be apparent to one skilled in the art.

The present invention can be implemented as a computer program product that comprises a computer program mechanism embedded in a non-transitory computer readable storage medium. For instance, the computer program product could contain the program modules shown in any combination in Figure 1 and/or as described elsewhere within the application. These program modules can be stored on a CD-ROM, DVD, magnetic disk storage product, USB key, or any other non-transitory computer readable data or program storage product.

Many modifications and variations of this disclosure can be made, as will be apparent to those skilled in the art. The specific embodiments described herein were chosen and described in order to best explain the principles of the invention and its practical applications, to thereby enable others skilled in the art to best utilize the invention and various embodiments with various modifications as are suited to the particular use contemplated. The disclosure is to be limited only by the terms of the appended claims, along with the full scope of equivalents to which such claims are entitled.

## Claims

1. A method for determining a copy number variation status of a subject, comprising:
on a computer system having one or more processors, and memory storing one or more programs for execution by the one or more processors:
A) obtaining, in electronic form, a first plurality of at least 100,000 nucleic acid sequences for a first plurality of DNA molecules from a first biological sample of the subject generated by whole genome sequencing at an average sequencing depth of from 0.5X to 5X across at least 90% of a reference genome for the species of the subject;
B) obtaining, in electronic form, a second plurality of at least 10,000 nucleic acid sequences for a second plurality of DNA molecules from a second biological sample of the subject generated by panel-targeted sequencing;
C) obtaining a first mapped dataset by a process comprising mapping the first plurality of nucleic acid sequences to positions within a reference genome for the species of the subject;
D) obtaining a second mapped dataset by a process comprising mapping the second plurality of nucleic acid sequences to positions within a reference construct for a plurality of genomic regions targeted by the panel-targeted sequencing; and
E) applying a model to (i) all or a portion of the first mapped dataset, or a first plurality of dimensionality reduction components thereof, and (ii) all or a portion of the second mapped dataset, or a second plurality of dimensionality reduction components thereof, wherein:
the model comprises a first component model and a second component model, wherein,
the first component model provides a first respective copy number state for a respective genomic region of the one or more respective genomic regions upon input to the first component model of all or a portion of the first mapped dataset, or the first plurality of dimensionality reduction components thereof, and
the second component model provides a second respective copy number state for the respective genomic region of the one or more respective genomic regions upon input to the second component model of all or a portion of the second mapped dataset, or the second plurality of dimensionality reduction components thereof; and
when both (i) the first respective copy number state and (ii) the second respective copy number state indicates the presence of a copy number variation at the respective genomic region, the copy number variation at the respective genomic region is accepted; and
when either (i) the first respective copy number state or (ii) the second respective copy number state does not indicate the presence of a copy number variation at the respective genomic region, the copy number variation at the respective genomic region is rejected,
thereby identifying one or more copy number variations, as output of the model, that indicate the copy number variation status of the subject.

2. The method of claim 1, wherein the first plurality of at least 100,000 nucleic acid sequences collectively provides an average sequencing depth of from 1X to 5X across at least 90% of a reference genome for the species of the subject or an average sequencing depth of from 2X to 3X across at least 90% of a reference genome for the species of the subject.

3. The method of claim 1 or 2, wherein the second plurality of at least 10,000 nucleic acid sequences collectively provides an average sequencing depth of at least 40X across the genomic regions targeted by the panel-targeted sequencing or an average sequencing depth of from 40X to 100X across the genomic regions targeted by the panel-targeted sequencing.

4. The method according to any one of claims 1-3, wherein panel-targeted sequencing targets at least 25 genes or is whole exome sequencing.

5. The method according to any one of claims 1-4, wherein the first biological sample of the subject and the second biological sample of the subject are:
obtained from a common single solid tumor sample from the subject, non-cancerous tissue samples from the subject, or independently selected from a saliva sample and a blood sample.

6. The method according to any one of claims 1-5, wherein:
the obtaining the first mapped dataset C) further comprises:
A) determining a respective first bin value for each respective bin in a first plurality of bins, wherein:
each respective bin in the first plurality of bins represents a unique segment of the reference genome, and
each respective first bin value is a measure of the number of nucleic acid sequences in the first plurality of nucleic acid sequences that were mapped in C) to the unique segment of the reference genome corresponding to the respective bin in the first plurality of bins; and
the all or the portion of the first mapped dataset inputted into the model in E) comprises the respective bin value for each respective bin in the first plurality of bins, or
B) determining a respective first bin value for each respective bin in a first plurality of bins, wherein:
each respective bin in the first plurality of bins represents a unique segment of the reference genome, and
each respective first bin value is a measure of the number of nucleic acid sequences in the first plurality of nucleic acid sequences that were mapped in C) to the unique segment of the reference genome corresponding to the respective bin in the first plurality of bins; and
determining a respective copy number state for each respective bin in the first plurality of bins using the respective first bin value for the respective bin; and
the all or the portion of the first mapped dataset inputted into the model in E) comprises the respective copy number state for each respective bin in the first plurality of bins.

7. The method of claim 6, wherein:
the corresponding first bin value for a respective bin in the first plurality of bins is the number of nucleic acid sequences in the first plurality of nucleic acid sequences that were mapped in C) to the unique segment of the reference genome corresponding to the respective bin, or
the corresponding first bin value for a respective bin in the first plurality of bins is a normalized or standardized number of nucleic acid sequences in the first plurality of nucleic acid sequences that were mapped in C) to the unique segment of the reference genome corresponding to the respective bin.

8. The method according to any one of claims 1-7, wherein:
the obtaining the second mapped dataset D) further comprises:
A) determining a respective second bin value for each respective bin in a second plurality of bins, wherein:
each respective bin in the second plurality of bins represents a unique segment of the reference construct, and
each respective second bin value is a measure of the number of nucleic acid sequences in the second plurality of nucleic acid sequences that were mapped in D) to the unique segment of the reference genome corresponding to the respective bin in the first plurality of bins; and
the all or the portion of the second mapped dataset inputted into the model in E) comprises the respective bin value for each respective bin in the second plurality of bins, or
B) determining a respective second bin value for each respective bin in a second plurality of bins, wherein:
each respective bin in the second plurality of bins represents a unique segment of the reference construct, and
each respective second bin value is a measure of the number of nucleic acid sequences in the first plurality of nucleic acid sequences that were mapped in D) to the unique segment of the reference genome corresponding to the respective bin in the second plurality of bins; and
determining a respective copy number state for each respective bin in the second plurality of bins using the respective second bin value for the respective bin; and
the all or the portion of the second mapped dataset inputted into the model in E) comprises the respective copy number state for each respective bin in the second plurality of bins.

9. The method of claim 8, wherein:
the corresponding second bin value for a respective bin in the second plurality of bins is the number of nucleic acid sequences in the second plurality of nucleic acid sequences that were mapped in D) to the unique segment of the reference construct corresponding to the respective bin, or
the corresponding second bin value for a respective bin in the second plurality of bins is a normalized or standardized number of nucleic acid sequences in the second plurality of nucleic acid sequences that were mapped in D) to the unique segment of the reference construct corresponding to the respective bin.

10. The method according to any one of claims 1-5, wherein:
the method further comprises applying a dimensionality reduction technique to (i) all or a portion of the first mapped dataset or (ii) all or a portion of the second mapped dataset, thereby generating the plurality of dimensionality reduction components; and
the E) applying comprises applying the plurality of dimensionality reduction components to the model.

11. The method of claim 1, wherein the first component model or the second component model:
is a statistical inference model, optionally wherein the statistical inference is a Bayesian inference, a likelihood-based inference, a frequentist inference, or an AIC-based inference is a machine-learning model, or indicates the presence of a copy number variation with a sensitivity of at least 90% and a specificity of no more than 90% when applied to data from a plurality of subjects comprising a first cohort population that includes subjects without copy number variations at the respective genomic region and a second cohort population that includes subjects with copy number variation at the respective genomic region.

12. The method according to any one of claims 1-9, wherein the model comprises a machine-learning model using (i) all or a portion of the first mapped dataset and (ii) all or a portion of the second mapped dataset as inputs, optionally wherein the machine-learning model is a support vector regression, a random forest model, an XGBoost model, a Gaussian process model, a deep neural network model, a convolutional neural network model, or a recurrent neural network model.

13. The method according to any one of claims 1-10, wherein:
the model is a statistical inference model;
the method further comprises applying a dimensionality reduction technique to (i) all or a portion of the first mapped dataset or (ii) all or a portion of the second mapped dataset, thereby generating the plurality of dimensionality reduction components; and
the E) applying comprises applying the plurality of dimensionality reduction components to the model, optionally wherein the dimensionality reduction technique is principal component analysis and the statistical inference model is a Bayesian model.

14. A computer system for determining a copy number variation status, the computer system comprising:
one or more processors; and
memory addressable by the one or more processors, the memory storing at least one program for execution by the one or more processors, the at least one program comprising instructions for:
A) obtaining, in electronic form, a first plurality of at least 100,000 nucleic acid sequences for a first plurality of DNA molecules from a first biological sample of the subject generated by whole genome sequencing at an average sequencing depth of from 0.5X to 5X across at least 90% of a reference genome for the species of the subject;
B) obtaining, in electronic form, a second plurality of at least 10,000 nucleic acid sequences for a second plurality of DNA molecules from a second biological sample of the subject generated by panel-targeted sequencing;
C) obtaining a first mapped dataset by a process comprising mapping the first plurality of nucleic acid sequences to positions within a reference genome for the species of the subject;
D) obtaining a second mapped dataset by a process comprising mapping the second plurality of nucleic acid sequences to positions within a reference construct for a plurality of genomic regions targeted by the panel-targeted sequencing; and
E) applying a model to (i) all or a portion of the first mapped dataset, or a first plurality of dimensionality reduction components thereof, and (ii) all or a portion of the second mapped dataset, or a second plurality of dimensionality reduction components thereof, wherein:
the model comprises a first component model and a second component model, wherein,
the first component model provides a first respective copy number state for a respective genomic region of the one or more respective genomic regions upon input to the first component model of all or a portion of the first mapped dataset, or the first plurality of dimensionality reduction components thereof, and
the second component model provides a second respective copy number state for the respective genomic region of the one or more respective genomic regions upon input to the second component model of all or a portion of the second mapped dataset, or the second plurality of dimensionality reduction components thereof; and
when both (i) the first respective copy number state and (ii) the second respective copy number state indicates the presence of a copy number variation at the respective genomic region, the copy number variation at the respective genomic region is accepted; and
when either (i) the first respective copy number state or (ii) the second respective copy number state does not indicate the presence of a copy number variation at the respective genomic region, the copy number variation at the respective genomic region is rejected,
thereby identifying one or more copy number variations, as output of the model that indicate the copy number variation status of the subject.

15. A non-transitory computer readable storage medium, wherein the non-transitory computer readable storage medium stores instructions, which when executed by a computer system, cause the computer system to perform a method for determining a copy number variation status, the method comprising:
A) obtaining, in electronic form, a first plurality of at least 100,000 nucleic acid sequences for a first plurality of DNA molecules from a first biological sample of the subject generated by whole genome sequencing at an average sequencing depth of from 0.5X to 5X across at least 90% of a reference genome for the species of the subject;
B) obtaining, in electronic form, a second plurality of at least 10,000 nucleic acid sequences for a second plurality of DNA molecules from a second biological sample of the subject generated by panel-targeted sequencing;
C) obtaining a first mapped dataset by a process comprising mapping the first plurality of nucleic acid sequences to positions within a reference genome for the species of the subject;
D) obtaining a second mapped dataset by a process comprising mapping the second plurality of nucleic acid sequences to positions within a reference construct for a plurality of genomic regions targeted by the panel-targeted sequencing; and
E) applying a model to (i) all or a portion of the first mapped dataset, or a first plurality of dimensionality reduction components thereof, and (ii) all or a portion of the second mapped dataset, or a second plurality of dimensionality reduction components thereof, wherein:
the model comprises a first component model and a second component model, wherein,
the first component model provides a first respective copy number state for a respective genomic region of the one or more respective genomic regions upon input to the first component model of all or a portion of the first mapped dataset, or the first plurality of dimensionality reduction components thereof, and
the second component model provides a second respective copy number state for the respective genomic region of the one or more respective genomic regions upon input to the second component model of all or a portion of the second mapped dataset, or the second plurality of dimensionality reduction components thereof; and
when both (i) the first respective copy number state and (ii) the second respective copy number state indicates the presence of a copy number variation at the respective genomic region, the copy number variation at the respective genomic region is accepted; and
when either (i) the first respective copy number state or (ii) the second respective copy number state does not indicate the presence of a copy number variation at the respective genomic region, the copy number variation at the respective genomic region is rejected,
thereby identifying one or more copy number variations, as output of the model that indicate the copy number variation status of the subject.

## Patentansprüche

1. Verfahren zur Bestimmung des Status einer Kopienzahlvariation eines Subjekts, umfassend:
auf einem Computersystem mit einem oder mehreren Prozessoren und einem Speicher, der ein oder mehrere Programme zur Ausführung durch den einen oder die mehreren Prozessoren speichert:
A) Erhalten einer ersten Vielzahl von mindestens 100.000 Nukleinsäuresequenzen für eine erste Vielzahl von DNA-Molekülen aus einer ersten biologischen Probe des Subjekts in elektronischer Form, die durch gesamte Genomsequenzierung mit einer durchschnittlichen Sequenzierungstiefe von 0,5X bis 5X über mindestens 90 % eines Referenzgenoms für die Spezies des Subjekts erzeugt wurden;
B) Erhalten einer zweiten Vielzahl von mindestens 10.000 Nukleinsäuresequenzen für eine zweite Vielzahl von DNA-Molekülen aus einer zweiten biologischen Probe des Subjekts in elektronischer Form, die durch panel-spezifische Sequenzierung erzeugt wurde;
C) Erhalten eines ersten zugeordneten Datensatzes durch ein Verfahren, umfassend das Zuordnen der ersten Vielzahl von Nukleinsäuresequenzen zu Positionen innerhalb eines Referenzgenoms für die Spezies des Subjekts;
D) Erhalten eines zweiten zugeordneten Datensatzes durch ein Verfahren, umfassend das Zuordnen der zweiten Vielzahl von Nukleinsäuresequenzen zu Positionen innerhalb eines Referenzkonstrukts für eine Vielzahl von genomischen Regionen, auf die die panel-spezifische Sequenzierung abzielt; und
E) Anwenden eines Modells auf (i) den gesamten oder einen Teil des ersten zugeordneten Datensatzes oder eine erste Vielzahl von Dimensionsreduktionskomponenten davon und (ii) den gesamten oder einen Teil des zweiten zugeordneten Datensatzes oder eine zweite Vielzahl von Dimensionsreduktionskomponenten davon, wobei:
das Modell ein erstes Komponentenmodell und ein zweites Komponentenmodell umfasst, wobei
das erste Komponentenmodell einen ersten jeweiligen Status der Kopienzahl für eine jeweilige Genomregion der einen oder mehreren jeweiligen Genomregionen bereitstellt, wenn dem ersten Komponentenmodell der gesamte oder ein Teil des ersten zugeordneten Datensatzes oder die erste Vielzahl von Dimensionsreduktionskomponenten davon eingegeben wird, und
das zweite Komponentenmodell einen zweiten jeweiligen Status der Kopienzahl für die jeweilige Genomregion der einen oder mehreren jeweiligen Genomregionen bereitstellt, wenn dem zweiten Komponentenmodell der gesamte oder ein Teil des zweiten zugeordneten Datensatzes oder die zweite Vielzahl von Dimensionsreduktionskomponenten davon eingegeben wird; und
wenn sowohl (i) der erste jeweilige Status der Kopienzahl als auch (ii) der zweite jeweilige Status der Kopienzahl das Vorhandensein einer Kopienzahlvariation an der jeweiligen Genomregion anzeigen, wird die Kopienzahlvariation in der jeweiligen Genomregion akzeptiert; und
wenn entweder (i) der erste jeweilige Status der Kopienzahl oder (ii) der zweite jeweilige Status der Kopienzahl nicht auf das Vorhandensein einer Kopienzahlvariation in der jeweiligen Genomregion hinweist, wird die Kopienzahlvariation in der jeweiligen Genomregion verworfen,
wodurch eine oder mehrere Kopienzahlvariationen als Ausgabe des Modells identifiziert werden, die den Status der Kopienzahlvariation des Subjekts angeben.

2. Verfahren nach Anspruch 1, wobei die erste Vielzahl von mindestens 100.000 Nukleinsäuresequenzen zusammen eine durchschnittliche Sequenzierungstiefe von 1X bis 5X über mindestens 90 % eines Referenzgenoms für die Spezies des Subjekts oder eine durchschnittliche Sequenzierungstiefe von 2X bis 3X über mindestens 90 % eines Referenzgenoms für die Spezies des Subjekts bereitstellt.

3. Verfahren nach Anspruch 1 oder 2, wobei die zweite Vielzahl von mindestens 10.000 Nukleinsäuresequenzen zusammen eine durchschnittliche Sequenzierungstiefe von mindestens 40X über die Genomregionen, auf die die panel-spezifische Sequenzierung abzielt, oder eine durchschnittliche Sequenzierungstiefe von 40X bis 100X über die Genomregionen, auf die die panel-spezifische Sequenzierung abzielt, bereitstellt.

4. Verfahren nach einem der Ansprüche 1bis 3, wobei die panel-spezifische Sequenzierung auf mindestens 25 Gene abzielt oder eine gesamte Exom-Sequenzierung ist.

5. Verfahren nach einem der Ansprüche 1 bis 4, wobei die erste biologische Probe des Subjekts und die zweite biologische Probe des Subjekts:
aus einer gleichen einzelnen soliden Tumorprobe des Subjekts, aus nicht-krebsartigen Gewebeproben des Subjekts oder unabhängig voneinander aus einer Speichelprobe und einer Blutprobe entnommen werden.

6. Verfahren nach einem der Ansprüche 1 bis 5, wobei:
das Entnehmen des ersten zugeordneten Datensatzes C) ferner umfasst:
A) Bestimmen eines jeweiligen ersten Bin-Werts für jeden jeweiligen Bin in einer ersten Vielzahl von Bins, wobei:
jeder jeweilige Bin in der ersten Vielzahl von Bins ein eindeutiges Segment des Referenzgenoms darstellt, und
jeder jeweilige erste Bin-Wert ein Maß für die Anzahl der Nukleinsäuresequenzen in der ersten Vielzahl von Nukleinsäuresequenzen ist, die in C) dem eindeutigen Segment des Referenzgenoms zugeordnet wurden, das dem jeweiligen Bin in der ersten Vielzahl von Bins entspricht; und
der gesamte oder der Teil des ersten zugeordneten Datensatzes, der in E) in das Modell eingegeben wird, den jeweiligen Bin-Wert für jeden jeweiligen Bin in der ersten Vielzahl von Bins umfasst, oder
B) Bestimmen eines jeweiligen ersten Bin-Werts für jeden jeweiligen Bin in einer ersten Vielzahl von Bins, wobei:
jeder jeweilige Bin in der ersten Vielzahl von Bins ein eindeutiges Segment des Referenzgenoms darstellt, und
jeder jeweilige erste Bin-Wert ein Maß für die Anzahl der Nukleinsäuresequenzen in der ersten Vielzahl von Nukleinsäuresequenzen ist, die in C) dem eindeutigen Segment des Referenzgenoms zugeordnet wurden, das dem jeweiligen Bin in der ersten Vielzahl von Bins entspricht; und
Bestimmen eines jeweiligen Status der Kopienzahl für jeden jeweiligen Bin in der ersten Vielzahl von Bins unter Verwendung des jeweiligen ersten Bin-Werts für den jeweiligen Bin; und
der gesamte oder der Teil des ersten zugeordneten Datensatzes, der in E) in das Modell eingegeben wird, umfasst den jeweiligen Zustand der Kopienzahl für jeden jeweiligen Bin in der ersten Vielzahl von Bins.

7. Verfahren nach Anspruch 6, wobei:
der entsprechende erste Bin-Wert für einen jeweiligen Bin in der ersten Vielzahl von Bins die Anzahl der Nukleinsäuresequenzen in der ersten Vielzahl von Nukleinsäuresequenzen ist, die in C) dem eindeutigen Segment des Referenzgenoms zugeordnet wurden, das dem jeweiligen Bin entspricht, oder
der entsprechende erste Bin-Wert für einen jeweiligen Bin in der ersten Vielzahl von Bins eine normalisierte oder standardisierte Anzahl von Nukleinsäuresequenzen in der ersten Vielzahl von Nukleinsäuresequenzen ist, die in C) dem eindeutigen Segment des Referenzgenoms zugeordnet wurden, das dem jeweiligen Bin entspricht.

8. Verfahren nach einem der Ansprüche 1 bis 7, wobei:
das Erhalten des zweiten zugeordneten Datensatzes D) ferner umfasst:
A) Bestimmen eines jeweiligen zweiten Bin-Werts für jeden jeweiligen Bin in einer zweiten Vielzahl von Bins, wobei:
jeder jeweilige Bin in der zweiten Vielzahl von Bins ein eindeutiges Segment des Referenzkonstrukts darstellt, und
jeder jeweilige zweite Bin-Wert ein Maß für die Anzahl der Nukleinsäuresequenzen in der zweiten Vielzahl von Nukleinsäuresequenzen ist, die in D) dem eindeutigen Segment des Referenzgenoms zugeordnet wurden, das dem jeweiligen Bin in der ersten Vielzahl von Bins entspricht; und
der gesamte oder der Teil des zweiten zugeordneten Datensatzes, der in E) in das Modell eingegeben wird, den jeweiligen Bin-Wert für jeden jeweiligen Bin in der zweiten Vielzahl von Bins umfasst, oder
B) Bestimmen eines jeweiligen zweiten Bin-Werts für jeden jeweiligen Bin in einer zweiten Vielzahl von Bins, wobei:
jeder jeweilige Bin in der zweiten Vielzahl von Bins ein eindeutiges Segment des Referenzkonstrukts darstellt, und
jeder jeweilige zweite Bin-Wert ein Maß für die Anzahl der Nukleinsäuresequenzen in der ersten Vielzahl von Nukleinsäuresequenzen ist, die in D) dem eindeutigen Segment des Referenzgenoms zugeordnet wurden, das dem jeweiligen Bin in der zweiten Vielzahl von Bins entspricht; und
Bestimmen eines jeweiligen Zustands der Kopienzahl für jeden jeweiligen Bin in der zweiten Vielzahl von Bins unter Verwendung des jeweiligen zweiten Bin-Werts für den jeweiligen Bin; und
der gesamte oder der Teil des zweiten zugeordneten Datensatzes, der in E) in das Modell eingegeben wird, umfasst den jeweiligen Zustand der Kopienzahl für jeden jeweiligen Bin in der zweiten Vielzahl von Bins.

9. Verfahren nach Anspruch 8, wobei:
der entsprechende zweite Bin-Wert für einen jeweiligen Bin in der zweiten Vielzahl von Bins die Anzahl der Nukleinsäuresequenzen in der zweiten Vielzahl von Nukleinsäuresequenzen ist, die in D) dem eindeutigen Segment des Referenzkonstrukts zugeordnet wurden, das dem jeweiligen Bin entspricht, oder
der entsprechende zweite Bin-Wert für einen jeweiligen Bin in der zweiten Vielzahl von Bins eine normalisierte oder standardisierte Anzahl von Nukleinsäuresequenzen in der zweiten Vielzahl von Nukleinsäuresequenzen ist, die in D) dem eindeutigen Segment des Referenzkonstrukts zugeordnet wurden, das dem jeweiligen Bin entspricht.

10. Verfahren nach einem der Ansprüche 1 bis 5, wobei:
das Verfahren ferner das Anwenden einer Dimensionsreduktionstechnik auf (i) den gesamten oder einen Teil des ersten zugeordneten Datensatzes oder (ii) den gesamten oder einen Teil des zweiten zugeordneten Datensatzes umfasst, wodurch die Vielzahl von Dimensionsreduktionskomponenten erzeugt wird; und
das Anwenden in E) das Anwenden der Vielzahl von Dimensionsreduktionskomponenten auf das Modell umfasst.

11. Verfahren nach Anspruch 1, wobei das erste Komponentenmodell oder das zweite Komponentenmodell:
ein statistisches Inferenzmodell ist, optional wobei die statistische Inferenz eine Bayes'sche Inferenz, eine likelihood-basierte Inferenz, eine frequentistische Inferenz oder eine AIC-basierte Inferenz ein maschinelles Lernmodell ist oder das Vorhandensein einer Kopienzahlvariation mit einer Sensitivität von mindestens 90 % und einer Spezifität von nicht mehr als 90 % anzeigt, wenn es auf Daten von einer Vielzahl von Subjekten angewendet wird, die eine erste Kohortenpopulation umfassen, die Subjekte ohne Kopienzahlvariationen in der jeweiligen Genomregion enthalten, und eine zweite Kohortenpopulation, die Subjekte mit Kopienzahlvariationen in der jeweiligen Genomregion enthalten.

12. Verfahren nach einem der Ansprüche 1 bis 9, wobei das Modell ein maschinelles Lernmodell umfasst, das (i) den gesamten oder einen Teil des ersten zugeordneten Datensatzes und (ii) den gesamten oder einen Teil des zweiten zugeordneten Datensatzes als Eingaben verwendet, optional wobei das maschinelle Lernmodell eine Support-Vektor-Regression, ein Random-Forest-Modell, ein XGBoost-Modell, ein Gauß-Prozess-Modell, ein tiefes neuronales Netzwerkmodell, ein konvolutionelles neuronales Netzwerkmodell oder ein rekurrentes neuronales Netzwerkmodell ist.

13. Verfahren nach einem der Ansprüche 1 bis 10, wobei:
das Modell ein statistisches Inferenzmodell ist;
das Verfahren ferner das Anwenden einer Dimensionsreduktionstechnik auf (i) den gesamten oder einen Teil des ersten zugeordneten Datensatzes oder (ii) den gesamten oder einen Teil des zweiten zugeordneten Datensatzes umfasst, wodurch die Vielzahl von Dimensionsreduktionskomponenten erzeugt wird; und
das Anwenden in E) das Anwenden der Vielzahl von Dimensionsreduktionskomponenten auf das Modell umfasst, optional wobei die Dimensionsreduktionstechnik eine Hauptkomponentenanalyse ist und das statistische Inferenzmodell ein Bayes'sches Modell ist.

14. Ein Computersystem zur Bestimmung eines Status einer Kopienzahlvariation, das Computersystem umfassend:
einen oder mehrere Prozessoren; und
einen von dem einen oder den mehreren Prozessoren adressierbaren Speicher, wobei der Speicher mindestens ein Programm zur Ausführung durch den einen oder die mehreren Prozessoren speichert, wobei das mindestens eine Programm Anweisungen umfasst für:
A) Erhalten einer ersten Vielzahl von mindestens 100.000 Nukleinsäuresequenzen für eine erste Vielzahl von DNA-Molekülen aus einer ersten biologischen Probe des Subjekts in elektronischer Form, die durch gesamte Genomsequenzierung mit einer durchschnittlichen Sequenzierungstiefe von 0,5X bis 5X über mindestens 90 % eines Referenzgenoms für die Spezies des Subjekts erzeugt wurden;
B) Erhalten einer zweiten Vielzahl von mindestens 10.000 Nukleinsäuresequenzen für eine zweite Vielzahl von DNA-Molekülen aus einer zweiten biologischen Probe des Subjekts in elektronischer Form, die durch panel-spezifische Sequenzierung erzeugt wurde;
C) Erhalten eines ersten zugeordneten Datensatzes durch ein Verfahren, umfassend das Zuordnen der ersten Vielzahl von Nukleinsäuresequenzen zu Positionen innerhalb eines Referenzgenoms für die Spezies des Subjekts;
D) Erhalten eines zweiten zugeordneten Datensatzes durch ein Verfahren, umfassend das Zuordnen der zweiten Vielzahl von Nukleinsäuresequenzen zu Positionen innerhalb eines Referenzkonstrukts für eine Vielzahl von genomischen Regionen, auf die die panel-spezifische Sequenzierung abzielt; und
E) Anwenden eines Modells auf (i) den gesamten oder einen Teil des ersten zugeordneten Datensatzes oder eine erste Vielzahl von Dimensionsreduktionskomponenten davon und (ii) den gesamten oder einen Teil des zweiten zugeordneten Datensatzes oder eine zweite Vielzahl von Dimensionsreduktionskomponenten davon, wobei:
das Modell ein erstes Komponentenmodell und ein zweites Komponentenmodell umfasst, wobei,
das erste Komponentenmodell einen ersten jeweiligen Status der Kopienzahl für eine jeweilige Genomregion der einen oder mehreren jeweiligen Genomregionen bereitstellt, wenn dem ersten Komponentenmodell der gesamte oder ein Teil des ersten zugeordneten Datensatzes oder die erste Vielzahl von Dimensionsreduktionskomponenten davon eingegeben wird, und
das zweite Komponentenmodell einen zweiten jeweiligen Status der Kopienzahl für die jeweilige Genomregion der einen oder mehreren jeweiligen Genomregionen bereitstellt, wenn dem zweiten Komponentenmodell der gesamte oder ein Teil des zweiten zugeordneten Datensatzes oder die zweite Vielzahl von Dimensionsreduktionskomponenten davon eingegeben wird; und
wenn sowohl (i) der erste jeweilige Status der Kopienzahl als auch (ii) der zweite jeweilige Status der Kopienzahl das Vorhandensein einer Kopienzahlvariation an der jeweiligen Genomregion anzeigen, wird die Kopienzahlvariation in der jeweiligen Genomregion akzeptiert; und
wenn entweder (i) der erste jeweilige Status der Kopienzahl oder (ii) der zweite jeweilige Status der Kopienzahl nicht auf das Vorhandensein einer Kopienzahlvariation in der jeweiligen Genomregion hinweist, wird die Kopienzahlvariation in der jeweiligen Genomregion verworfen,
wodurch eine oder mehrere Kopienzahlvariationen als Ausgabe des Modells identifiziert werden, die den Status der Kopienzahlvariation des Subjekts angeben.

15. Nichtflüchtiges, computerlesbares Speichermedium, wobei das nichtflüchtige, computerlesbare Speichermedium Anweisungen speichert, die, wenn sie von einem Computersystem ausgeführt werden, das Computersystem veranlassen, ein Verfahren zur Bestimmung eines Status der Kopienzahl durchzuführen, das Verfahren umfassend:
A) Erhalten einer ersten Vielzahl von mindestens 100.000 Nukleinsäuresequenzen für eine erste Vielzahl von DNA-Molekülen aus einer ersten biologischen Probe des Subjekts in elektronischer Form, die durch gesamte Genomsequenzierung mit einer durchschnittlichen Sequenzierungstiefe von 0,5X bis 5X über mindestens 90 % eines Referenzgenoms für die Spezies des Subjekts erzeugt wurden;
B) Erhalten einer zweiten Vielzahl von mindestens 10.000 Nukleinsäuresequenzen für eine zweite Vielzahl von DNA-Molekülen aus einer zweiten biologischen Probe des Subjekts in elektronischer Form, die durch panel-spezifische Sequenzierung erzeugt wurde;
C) Erhalten eines ersten zugeordneten Datensatzes durch ein Verfahren, umfassend das Zuordnen der ersten Vielzahl von Nukleinsäuresequenzen zu Positionen innerhalb eines Referenzgenoms für die Spezies des Subjekts;
D) Erhalten eines zweiten zugeordneten Datensatzes durch ein Verfahren, umfassend das Zuordnen der zweiten Vielzahl von Nukleinsäuresequenzen zu Positionen innerhalb eines Referenzkonstrukts für eine Vielzahl von genomischen Regionen, auf die die panel-spezifische Sequenzierung abzielt; und
E) Anwenden eines Modells auf (i) den gesamten oder einen Teil des ersten zugeordneten Datensatzes oder eine erste Vielzahl von Dimensionsreduktionskomponenten davon und (ii) den gesamten oder einen Teil des zweiten zugeordneten Datensatzes oder eine zweite Vielzahl von Dimensionsreduktionskomponenten davon, wobei:
das Modell ein erstes Komponentenmodell und ein zweites Komponentenmodell umfasst, wobei,
das erste Komponentenmodell einen ersten jeweiligen Status der Kopienzahl für eine jeweilige Genomregion der einen oder mehreren jeweiligen Genomregionen bereitstellt, wenn dem ersten Komponentenmodell der gesamte oder ein Teil des ersten zugeordneten Datensatzes oder die erste Vielzahl von Dimensionsreduktionskomponenten davon eingegeben wird, und
das zweite Komponentenmodell einen zweiten jeweiligen Status der Kopienzahl für die jeweilige Genomregion der einen oder mehreren jeweiligen Genomregionen bereitstellt, wenn dem zweiten Komponentenmodell der gesamte oder ein Teil des zweiten zugeordneten Datensatzes oder die zweite Vielzahl von Dimensionsreduktionskomponenten davon eingegeben wird; und
wenn sowohl (i) der erste jeweilige Status der Kopienzahl als auch (ii) der zweite jeweilige Status der Kopienzahl das Vorhandensein einer Kopienzahlvariation an der jeweiligen Genomregion anzeigen, wird die Kopienzahlvariation in der jeweiligen Genomregion akzeptiert; und
wenn entweder (i) der erste jeweilige Status der Kopienzahl oder (ii) der zweite jeweilige Status der Kopienzahl nicht auf das Vorhandensein einer Kopienzahlvariation in der jeweiligen Genomregion hinweist, wird die Kopienzahlvariation in der jeweiligen Genomregion verworfen,
wodurch eine oder mehrere Kopienzahlvariationen als Ausgabe des Modells identifiziert werden, die den Status der Kopienzahlvariation des Subjekts angeben.

## Revendications

1. Procédé de détermination d'un état de variation de nombre de copies chez un sujet, comprenant :
sur un système informatique comportant un ou plusieurs processeurs, et une mémoire stockant un ou plusieurs programmes destinés à être exécutés par les un ou plusieurs processeurs :
A) l'obtention, sous forme électronique, d'une première pluralité d'au moins 100 000 séquences d'acide nucléique pour une première pluralité de molécules d'ADN provenant d'un premier échantillon biologique du sujet, générées par séquençage de génome entier à une profondeur moyenne de séquençage de 0,5X à 5X sur au moins 90 % d'un génome de référence de l'espèce du sujet ;
B) l'obtention, sous forme électronique, d'une seconde pluralité d'au moins 10 000 séquences d'acide nucléique pour une seconde pluralité de molécules d'ADN provenant d'un second échantillon biologique du sujet, générées par séquençage ciblé sur panel ;
C) l'obtention d'un premier ensemble de données de mappage par un processus comprenant le mappage de la première pluralité de séquences d'acide nucléique sur des positions au sein d'un génome de référence de l'espèce du sujet ;
D) l'obtention d'un second ensemble de données de mappage par un processus comprenant le mappage de la seconde pluralité de séquences d'acide nucléique sur des positions au sein d'une construction de référence pour une pluralité de régions génomiques ciblées par le séquençage ciblé sur panel ; et
E) l'application d'un modèle à (i) la totalité ou une partie du premier ensemble de données de mappage, ou à une première pluralité de composantes de réduction de dimension de celui-ci, et à (ii) la totalité ou une partie du second ensemble de données de mappage, ou à une seconde pluralité de composantes de réduction de dimension de celui-ci, dans lequel :
le modèle comprend un premier modèle de composante et un second modèle de composante, dans lequel,
le premier modèle de composante fournit un premier état respectif de nombre de copies pour une région génomique respective des une ou plusieurs régions génomiques respectives lors de l'entrée, dans le premier modèle de composante, de la totalité ou d'une partie du premier ensemble de données de mappage, ou de la première pluralité de composantes de réduction de dimension de celui-ci, et
le second modèle de composante fournit un second état respectif de nombre de copies pour la région génomique respective des une ou plusieurs régions génomiques respectives lors de l'entrée, dans le second modèle de composante, de la totalité ou d'une partie du second ensemble de données de mappage, ou de la seconde pluralité de composantes de réduction de dimension de celui-ci ; et
lorsque (i) le premier état respectif de nombre de copies et (ii) le second état respectif de nombre de copies indiquent tous les deux la présence d'une variation de nombre de copies au niveau de la région génomique respective, la variation de nombre de copies au niveau de la région génomique respective est validée ; et
lorsque soit (i) le premier état respectif de nombre de copies, soit (ii) le second état respectif de nombre de copies n'indique pas la présence d'une variation de nombre de copies au niveau de la région génomique respective, la variation de nombre de copies au niveau de la région génomique respective est rejetée,
pour ainsi identifier une ou plusieurs variations de nombre de copies, en tant que sortie du modèle, qui indiquent l'état de variation du nombre de copies du sujet.

2. Procédé selon la revendication 1, dans lequel la première pluralité d'au moins 100 000 séquences d'acide nucléique fournissent collectivement une profondeur moyenne de séquençage de 1X à 5X sur au moins 90 % d'un génome de référence de l'espèce du sujet ou une profondeur moyenne de séquençage de 2X à 3X sur au moins 90 % d'un génome de référence de l'espèce du sujet.

3. Procédé selon la revendication 1 ou 2, dans lequel la seconde pluralité d'au moins 10 000 séquences d'acide nucléique fournissent collectivement une profondeur moyenne de séquençage d'au moins 40X sur les régions génomiques ciblées par le séquençage ciblé sur panel ou une profondeur moyenne de séquençage de 40X à 100X sur les régions génomiques ciblées par le séquençage ciblé sur panel.

4. Procédé selon l'une quelconque des revendications 1 à 3, dans lequel le séquençage ciblé sur panel cible au moins 25 gènes ou est un séquençage de l'exome entier.

5. Procédé selon l'une quelconque des revendications 1 à 4, dans lequel le premier échantillon biologique du sujet et le second échantillon biologique du sujet sont :
obtenus à partir d'un échantillon commun de tumeur solide unique du sujet, d'échantillons de tissus non cancéreux du sujet, ou sélectionnés indépendamment parmi un échantillon de salive et un échantillon de sang.

6. Procédé selon l'une quelconque des revendications 1 à 5, dans lequel :
l'obtention du premier ensemble de données de mappage en C) comprend en outre :
A) la détermination d'une première valeur d'intervalle respective pour chaque intervalle respectif dans une première pluralité d'intervalles, dans lequel :
chaque intervalle respectif dans la première pluralité d'intervalles représente un segment unique du génome de référence, et
chaque première valeur d'intervalle respective est une mesure du nombre de séquences d'acide nucléique dans la première pluralité de séquences d'acide nucléique qui ont été mappées en C) sur le segment unique du génome de référence correspondant à l'intervalle respectif dans la première pluralité d'intervalles ; et
la totalité ou la partie du premier ensemble de données de mappage entrée dans le modèle en E) comprend la valeur d'intervalle respective pour chaque intervalle respectif dans la première pluralité d'intervalles, ou
B) la détermination d'une première valeur d'intervalle respective pour chaque intervalle respectif dans une première pluralité d'intervalles, dans lequel :
chaque intervalle respectif dans la première pluralité d'intervalles représente un segment unique du génome de référence, et
chaque première valeur d'intervalle respective est une mesure du nombre de séquences d'acide nucléique dans la première pluralité de séquences d'acide nucléique qui ont été mappées en C) sur le segment unique du génome de référence correspondant à l'intervalle respectif dans la première pluralité d'intervalles ; et
la détermination d'un état respectif de nombre de copies pour chaque intervalle respectif dans la première pluralité d'intervalles en utilisant la première valeur d'intervalle respective pour l'intervalle respectif ; et
la totalité ou la partie du premier ensemble de données de mappage entrée dans le modèle en E) comprend l'état respectif de nombre de copies pour chaque intervalle respectif dans la première pluralité d'intervalles.

7. Procédé selon la revendication 6, dans lequel :
la première valeur d'intervalle correspondante pour un intervalle respectif dans la première pluralité d'intervalles est le nombre de séquences d'acide nucléique dans la première pluralité de séquences d'acide nucléique qui ont été mappées en C) sur le segment unique du génome de référence correspondant à l'intervalle respectif, ou
la première valeur d'intervalle correspondante pour un intervalle respectif dans la première pluralité d'intervalles est un nombre normalisé ou standardisé de séquences d'acide nucléique dans la première pluralité de séquences d'acide nucléique qui ont été mappées en C) sur le segment unique du génome de référence correspondant à l'intervalle respectif.

8. Procédé selon l'une quelconque des revendications 1 à 7, dans lequel :
l'obtention du second ensemble de données de mappage en D) comprend en outre :
A) la détermination d'une seconde valeur d'intervalle respective pour chaque intervalle respectif dans une seconde pluralité d'intervalles, dans lequel :
chaque intervalle respectif dans la seconde pluralité d'intervalles représente un segment unique de la construction de référence, et
chaque seconde valeur d'intervalle respective est une mesure du nombre de séquences d'acide nucléique dans la seconde pluralité de séquences d'acide nucléique qui ont été mappées en D) sur le segment unique du génome de référence correspondant à l'intervalle respectif dans la première pluralité d'intervalles ; et
la totalité ou la partie du second ensemble de données de mappage entrée dans le modèle en E) comprend la valeur d'intervalle respective pour chaque intervalle respectif dans la seconde pluralité d'intervalles, ou
B) la détermination d'une seconde valeur d'intervalle respective pour chaque intervalle respectif dans une seconde pluralité d'intervalles, dans lequel :
chaque intervalle respectif dans la seconde pluralité d'intervalles représente un segment unique de la construction de référence, et
chaque seconde valeur d'intervalle respective est une mesure du nombre de séquences d'acide nucléique dans la première pluralité de séquences d'acide nucléique qui ont été mappées en D) sur le segment unique du génome de référence correspondant à l'intervalle respectif dans la seconde pluralité d'intervalles ; et
la détermination d'un état respectif de nombre de copies pour chaque intervalle respectif dans la seconde pluralité d'intervalles en utilisant la seconde valeur d'intervalle respective pour l'intervalle respectif ; et
la totalité ou la partie du second ensemble de données de mappage entrée dans le modèle en E) comprend l'état respectif de nombre de copies pour chaque intervalle respectif dans la seconde pluralité d'intervalles.

9. Procédé selon la revendication 8, dans lequel :
la seconde valeur d'intervalle correspondante pour un intervalle respectif dans la seconde pluralité d'intervalles est le nombre de séquences d'acide nucléique dans la seconde pluralité de séquences d'acide nucléique qui ont été mappées en D) sur le segment unique de la construction de référence correspondant à l'intervalle respectif, ou
la seconde valeur d'intervalle correspondante pour un intervalle respectif dans la seconde pluralité d'intervalles est un nombre normalisé ou standardisé de séquences d'acide nucléique dans la seconde pluralité de séquences d'acide nucléique qui ont été mappées en D) sur le segment unique de la construction de référence correspondant à l'intervalle respectif.

10. Procédé selon l'une quelconque des revendications 1 à 5, dans lequel :
le procédé comprend en outre l'application d'une technique de réduction de dimension à (i) la totalité ou une partie du premier ensemble de données de mappage ou à (ii) la totalité ou une partie du second ensemble de données de mappage, pour ainsi générer la pluralité de composantes de réduction de dimension ; et
l'application E) comprend l'application de la pluralité de composantes de réduction de dimension au modèle.

11. Procédé la revendication 1, dans lequel le premier modèle de composante ou le second modèle de composante :
est un modèle d'inférence statistique, facultativement dans lequel l'inférence statistique est une inférence bayésienne, une inférence basée sur la vraisemblance, une inférence fréquentiste, ou une inférence basée sur l'AIC est un modèle d'apprentissage automatique, ou indique la présence d'une variation de nombre de copies avec une sensibilité d'au moins 90 % et une spécificité d'au plus 90 % lorsqu'il est appliqué à des données provenant d'une pluralité de sujets comprenant une première population de cohorte qui inclut des sujets sans variation de nombre de copies au niveau de la région génomique respective et une seconde population de cohorte qui inclut des sujets présentant une variation de nombre de copies au niveau de la région génomique respective.

12. Procédé selon l'une quelconque des revendications 1 à 9, dans lequel le modèle comprend un modèle d'apprentissage automatique utilisant (i) la totalité ou une partie du premier ensemble de données de mappage et (ii) la totalité ou une partie du second ensemble de données de mappage comme entrées, facultativement dans lequel le modèle d'apprentissage automatique est une régression à vecteurs de support, un modèle de forêt aléatoire, un modèle XGBoost, un modèle de processus gaussien, un modèle de réseau de neurones profond, un modèle de réseau de neurones convolutif ou un modèle de réseau de neurones récurrent.

13. Procédé selon l'une quelconque des revendications 1 à 10, dans lequel :
le modèle est un modèle d'inférence statistique ;
le procédé comprend en outre l'application d'une technique de réduction de dimension à (i) la totalité ou une partie du premier ensemble de données de mappage ou à (ii) la totalité ou une partie du second ensemble de données de mappage, pour ainsi générer la pluralité de composantes de réduction de dimension ; et
l'application E) comprend l'application de la pluralité de composantes de réduction de dimension au modèle, facultativement dans lequel la technique de réduction de dimension est une analyse en composantes principales et le modèle d'inférence statistique est un modèle bayésien.

14. Système informatique pour déterminer un état de variation de nombre de copies, le système informatique comprenant :
un ou plusieurs processeurs ; et
une mémoire adressable par les un ou plusieurs processeurs, la mémoire stockant au moins un programme destiné à être exécuté par les un ou plusieurs processeurs, l'au moins un programme comprenant des instructions pour :
A) l'obtention, sous forme électronique, d'une première pluralité d'au moins 100 000 séquences d'acide nucléique pour une première pluralité de molécules d'ADN provenant d'un premier échantillon biologique du sujet, générées par séquençage de génome entier à une profondeur moyenne de séquençage de 0,5X à 5X sur au moins 90 % d'un génome de référence de l'espèce du sujet ;
B) l'obtention, sous forme électronique, d'une seconde pluralité d'au moins 10 000 séquences d'acide nucléique pour une seconde pluralité de molécules d'ADN provenant d'un second échantillon biologique du sujet, générées par séquençage ciblé sur panel ;
C) l'obtention d'un premier ensemble de données de mappage par un processus comprenant le mappage de la première pluralité de séquences d'acide nucléique sur des positions au sein d'un génome de référence de l'espèce du sujet ;
D) l'obtention d'un second ensemble de données de mappage par un processus comprenant le mappage de la seconde pluralité de séquences d'acide nucléique sur des positions au sein d'une construction de référence pour une pluralité de régions génomiques ciblées par le séquençage ciblé sur panel ; et
E) l'application d'un modèle à (i) la totalité ou une partie du premier ensemble de données de mappage, ou à une première pluralité de composantes de réduction de dimension de celui-ci, et à (ii) la totalité ou une partie du second ensemble de données de mappage, ou à une seconde pluralité de composantes de réduction de dimension de celui-ci, dans lequel :
le modèle comprend un premier modèle de composante et un second modèle de composante, dans lequel,
le premier modèle de composante fournit un premier état respectif de nombre de copies pour une région génomique respective des une ou plusieurs régions génomiques respectives lors de l'entrée, dans le premier modèle de composante, de la totalité ou d'une partie du premier ensemble de données de mappage, ou de la première pluralité de composantes de réduction de dimension de celui-ci, et
le second modèle de composante fournit un second état respectif de nombre de copies pour la région génomique respective des une ou plusieurs régions génomiques respectives lors de l'entrée, dans le second modèle de composante, de la totalité ou d'une partie du second ensemble de données de mappage, ou de la seconde pluralité de composantes de réduction de dimension de celui-ci ; et
lorsque (i) le premier état respectif de nombre de copies et (ii) le second état respectif de nombre de copies indiquent tous les deux la présence d'une variation de nombre de copies au niveau de la région génomique respective, la variation de nombre de copies au niveau de la région génomique respective est validée ; et
lorsque soit (i) le premier état respectif de nombre de copies, soit (ii) le second état respectif de nombre de copies n'indique pas la présence d'une variation de nombre de copies au niveau de la région génomique respective, la variation de nombre de copies au niveau de la région génomique respective est rejetée,
pour ainsi identifier une ou plusieurs variations de nombre de copies, en tant que sortie du modèle, qui indiquent l'état de variation du nombre de copies du sujet.

15. Support de stockage non transitoire lisible par ordinateur, dans lequel le support de stockage non transitoire lisible par ordinateur stocke des instructions qui, lorsqu'elles sont exécutées par un système informatique, amènent le système informatique à mettre en œuvre un procédé de détermination d'un état de variation de nombre de copies, le procédé comprenant :
A) l'obtention, sous forme électronique, d'une première pluralité d'au moins 100 000 séquences d'acide nucléique pour une première pluralité de molécules d'ADN provenant d'un premier échantillon biologique du sujet, générées par séquençage de génome entier à une profondeur moyenne de séquençage de 0,5X à 5X sur au moins 90 % d'un génome de référence de l'espèce du sujet ;
B) l'obtention, sous forme électronique, d'une seconde pluralité d'au moins 10 000 séquences d'acide nucléique pour une seconde pluralité de molécules d'ADN provenant d'un second échantillon biologique du sujet, générées par séquençage ciblé sur panel ;
C) l'obtention d'un premier ensemble de données de mappage par un processus comprenant le mappage de la première pluralité de séquences d'acide nucléique sur des positions au sein d'un génome de référence de l'espèce du sujet ;
D) l'obtention d'un second ensemble de données de mappage par un processus comprenant le mappage de la seconde pluralité de séquences d'acide nucléique sur des positions au sein d'une construction de référence pour une pluralité de régions génomiques ciblées par le séquençage ciblé sur panel ; et
E) l'application d'un modèle à (i) la totalité ou une partie du premier ensemble de données de mappage, ou à une première pluralité de composantes de réduction de dimension de celui-ci, et à (ii) la totalité ou une partie du second ensemble de données de mappage, ou à une seconde pluralité de composantes de réduction de dimension de celui-ci, dans lequel :
le modèle comprend un premier modèle de composante et un second modèle de composante, dans lequel,
le premier modèle de composante fournit un premier état respectif de nombre de copies pour une région génomique respective des une ou plusieurs régions génomiques respectives lors de l'entrée, dans le premier modèle de composante, de la totalité ou d'une partie du premier ensemble de données de mappage, ou de la première pluralité de composantes de réduction de dimension de celui-ci, et
le second modèle de composante fournit un second état respectif de nombre de copies pour la région génomique respective des une ou plusieurs régions génomiques respectives lors de l'entrée, dans le second modèle de composante, de la totalité ou d'une partie du second ensemble de données de mappage, ou de la seconde pluralité de composantes de réduction de dimension de celui-ci ; et
lorsque (i) le premier état respectif de nombre de copies et (ii) le second état respectif de nombre de copies indiquent tous les deux la présence d'une variation de nombre de copies au niveau de la région génomique respective, la variation de nombre de copies au niveau de la région génomique respective est validée ; et
lorsque soit (i) le premier état respectif de nombre de copies, soit (ii) le second état respectif de nombre de copies n'indique pas la présence d'une variation de nombre de copies au niveau de la région génomique respective, la variation de nombre de copies au niveau de la région génomique respective est rejetée,
pour ainsi identifier une ou plusieurs variations de nombre de copies, en tant que sortie du modèle, qui indiquent l'état de variation du nombre de copies du sujet.
